(19) Europäisches Patentamt
European Patent Office
Office européen des brevets

(11) Numéro de publication: **0 282 373 B1**

(12) # FASCICULE DE BREVET EUROPEEN

(45) Date de publication de fascicule du brevet: **30.09.92**

(51) Int. Cl.$^5$: **C07D 401/04**, C07D 211/26, A61K 31/445, A61K 31/415

(21) Numéro de dépôt: **88400312.0**

(22) Date de dépôt: **11.02.88**

Le dossier contient des informations techniques présentées postérieurement au dépôt de la demande et ne figurant pas dans le présent fascicule.

(54) **Dérivés de la benzimidazol-2-one, leurs sels, procédé et intermédiaires de préparation, application à titre de médicaments et compositions les renfermant.**

(30) Priorité: **13.02.87 FR 8701836**

(43) Date de publication de la demande:
**14.09.88 Bulletin 88/37**

(45) Mention de la délivrance du brevet:
**30.09.92 Bulletin 92/40**

(84) Etats contractants désignés:
**AT BE CH DE ES FR GB GR IT LI LU NL SE**

(56) Documents cités:
**EP-A- 0 071 520**
**EP-A- 0 071 521**
**EP-A- 0 200 583**
**FR-A- 2 458 549**

(73) Titulaire: **ROUSSEL-UCLAF**
**35, Boulevard des Invalides**
**F-75007 Paris(FR)**

(72) Inventeur: **Agouridas, Constantin**
**67, Ouai d'Orsay**
**F-75007 Paris(FR)**
Inventeur: **Fauveau, Patrick**
**40, Avenue Camille Desmoulins**
**F-93190 Livry-Gargan(FR)**
Inventeur: **Hamon, Gilles**
**40, Rue de Bagneux**
**F-92120 Montrouge(FR)**
Inventeur: **Nedelec, Lucien**
**45, Boulevard de l'Ouest**
**F-93340 Le Raincy(FR)**

(74) Mandataire: **Bourgouin, André et al**
**Département des Brevets ROUSSEL UCLAF**
**111, route de Noisy B.P. no 9**
**F-93230 Romainville(FR)**

EP 0 282 373 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux dérivés de la benzimidazol-2-one ainsi que leurs sels, le procédé et intermédiaires de préparation, l'application à titre de médicaments de ces nouveaux dérivés, les compositions les renfermant.

Les brevets européens EP 0.200.853 et 0.071.521 décrivent des produits possédant des propriétés stimulantes dopaminergiques. Ces produits ont cependant une structure indolique alors que les produits de la présente demande ont une structure benzimidazole.

Le brevet français n° 2 458 549 décrit également des dérivés du 4-(pipéridin-3-yl) 1H-indole doués de propriétés stimulantes dopaminergiques marquées, au niveau central.

La présente invention a pour objet de nouveaux dérivés de la benzimidazol-2-one ainsi que leurs sels d'addition avec les acides minéraux ou organiques, caractérisés en ce qu'ils répondent à la formule générale (I) :

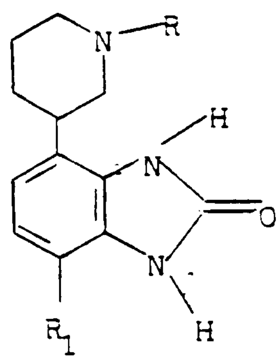

(I)

dans laquelle R représente un atome d'hydrogène, un radical alkyle renfermant de 1 à 5 atomes de carbone, un radical cycloalkylalkyle renfermant de 4 à 7 atomes de carbone, un radical aralkyle renfermant de 7 à 12 atomes de carbone, éventuellement substitué par un ou plusieurs radicaux choisis dans le groupe constitué par les radicaux alkyles renfermant de 1 à 5 atomes de carbone, les radicaux alkoxy renfermant de 1 à 5 atomes de carbone et les atomes d'halogène ou R représente un radical alkényle ou un radical alkynyle renfermant de 3 à 5 atomes de carbone, étant entendu que la liaison multiple ne peut pas se trouver entre les carbones en alpha et béta de l'atome d'azote et $R_1$ représente un atome d'hydrogène, un radical hydroxy, un radical alkoxy renfermant de 1 à 5 atomes de carbone, un radical phénylalkyloxy renfermant de 7 à 9 atomes de carbone ou un radical phénoxy.

Dans la formule générale (I) et dans ce qui suit, le terme radical alkyle renfermant de 1 à 5 atomes de carbone désigne, de préférence, un radical méthyle, éthyle, propyle ou isopropyle ; le terme radical cycloalkylalkyle renfermant de 4 à 7 atomes de carbone désigne, de préférence, un radical cyclopropylméthyle ou cyclobutylméthyle ; le terme radical aralkyle renfermant de 7 à 12 atomes de carbone désigne, de préférence, un radical benzyle, phénéthyle ou naphtylméthyle ; l'aryle peut être plurisubstitué ou de préférence monosubstitué ; le terme radical alkoxy renfermant de 1 à 5 atomes de carbone désigne de préférence un radical méthoxy, éthoxy, propoxy ou isopropoxy ; le terme atome d'halogène désigne, de préférence, un atome de chlore ou de brome ; les termes radical alkényle et alkynyle renfermant de 3 à 5 atomes de carbone désignent de préférence, un radical allyle ou propargyle ; le terme radical phénylalkyloxy renfermant de 7 à 9 atomes de carbone désigne de préférence, un radical benzyloxy ou phénéthyloxy.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléïque, fumarique, succinique, tartrique, citrique, oxalique, glyoxylique, aspartique, alcanesulfoniques tels que les acides méthane et éthanesulfoniques, arylsulfoniques tels que les acides benzène et para-toluènesulfoniques et aryl carboxyliques.

Parmi les produits, objet de l'invention, on peut citer notamment les dérivés répondant à la formule (I) ci-dessus, ainsi que leurs sels d'addition avec les acides minéraux ou organiques, caractérisés en ce que dans ladite formule (I), $R_1$ représente un atome d'hydrogène.

Parmi ceux-ci, on peut citer tout particulièrement les dérivés caractérisés en ce que, dans ladite formule (I), R représente un atome d'hydrogène ou un radical alkyle renfermant de 1 à 5 atomes de carbone et tout particulièrement la 1,3-dihydro 4-(1-propyl-3-pipéridinyl) 2H-benzimidazol-2-one et la 1,3-dihydro 4-hydroxy 7-(1-propyl 3-pipéridinyl) 2H-benzimidazol-2-one ainsi que leurs sels d'addition avec les acides minéraux ou

organiques.

L'invention a également pour objet un procédé de préparation des dérivés, tels que définis par la formule (I) ci-dessus dans laquelle $R_1$ représente un atome d'hydrogène ainsi que de leurs sels, caractérisé en ce que l'on fait réagir un dérivé de formule (II) :

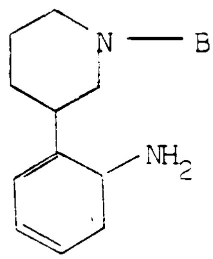

(II)

dans laquelle B représente un agent de blocage réversible d'une amine secondaire avec un halogénoformiate d'alkyle de formule (III) :

Hal-COO-Alk (III)

dans laquelle Hal représente un atome de chlore, de brome ou d'iode et Alk représente un radical alkyle renfermant de 1 à 5 atomes de carbone, pour obtenir un dérivé de formule (IV) :

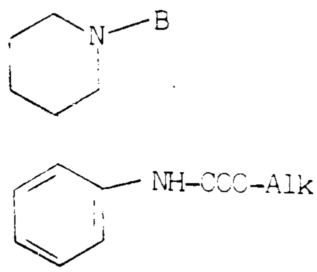

(IV)

dans laquelle B et Alk ont la signification déjà indiquée, que l'on soumet à une nitration, pour obtenir un dérivé de formule (V) :

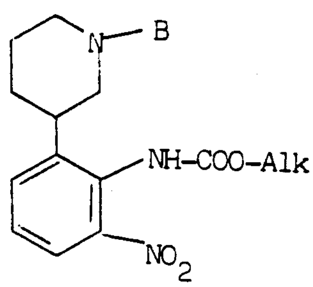

(V)

dans laquelle B et Alk ont la signification déjà indiquée dont soit l'on reduit le groupement nitro, pour obtenir un dérivé de formule (VI) :

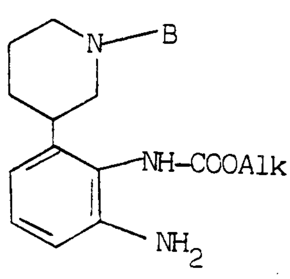

(VI)

dans laquelle B et Alk ont la signification déjà indiquée soit l'on soumet ledit dérivé de formule (V) ou ledit dérivé de formule (VI) à l'action d'un agent de déblocage de l'amine, du cycle pipéridine, pour obtenir respectivement un dérivé de formule (V′) ou (VI′) :

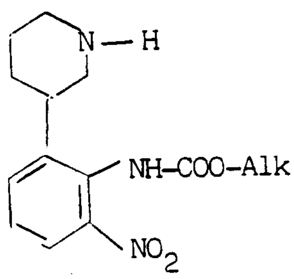

(V')

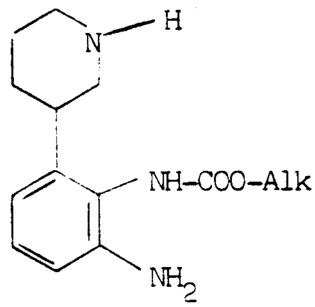

(VI')

dans lesquelles Alk a la signification déjà indiquée, que l'on soumet à l'action d'un agent capable de greffer un radical R′, R′ ayant la signification de R à l'exception de l'atome d'hydrogène, pour obtenir respectivement des dérivés de formules (V″) ou (VI″) :

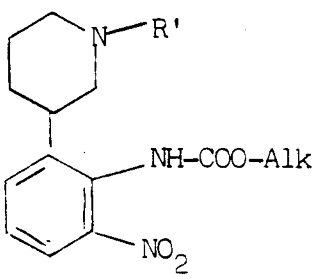

(V")

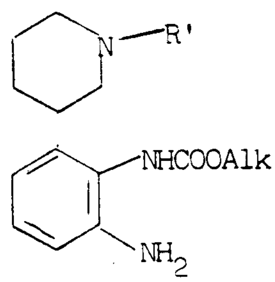

(VI")

dans lesquelles Alk a la signification déjà indiquée, puis réduit le groupement nitro desdits dérivés de formule (V′) et (V″), pour obtenir respectivement les dérivés de formules (VI′) et (VI″) que l'on cyclise par hydrolyse, pour obtenir un dérivé de formule ($I_A$) :

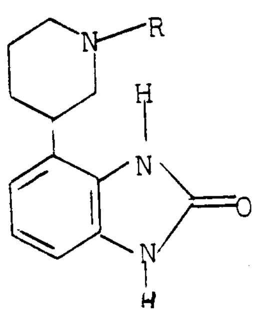

($I_A$)

dans laquelle R a la signification déjà indiquée, que l'on isole et, si désiré, salifie.

Il est également possible de cycliser le dérivé de formule (VI) comme les dérivés de formules (VI′) et (VI″), puis de débloquer l'amine secondaire du cycle pipéridine, pour obtenir un dérivé de formule ($I_A$) dans laquelle R représente un atome d'hydrogène, que l'on peut éventuellement substituer, pour obtenir d'autres valeurs de R.

L'invention a aussi pour objet un procédé de préparation des dérivés tels que définis par la formule (I) ci-dessus dans laquelle $R_1$ est différent d'un atome d'hydrogène ainsi que de leurs sels, caractérisé en ce que l'on fait réagir un dérivé de formule (II′) :

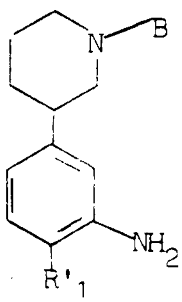

(II')

dans laquelle B a la signification déjà indiquée et $R'_1$ a la signification de $R_1$ déjà indiquée, à l'exception de l'atome d'hydrogène et du radical hydroxyle avec le phosgène ou un précurseur de phosgène, pour obtenir un dérivé de formule (III') :

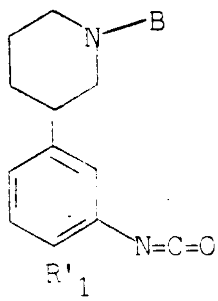

(III')

dans laquelle B et $R'_1$ ont la signification déjà indiquée, que l'on fait réagir avec l'O-benzylhydroxylamine, pour obtenir un dérivé de formule (IV') :

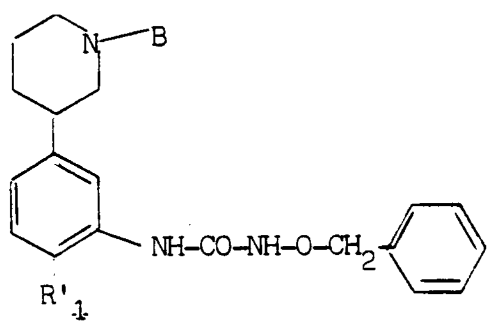

(IV')

dans laquelle B et $R'_1$ ont la signification déjà indiquée, que l'on cyclise, pour obtenir un dérivé de formule (VII) :

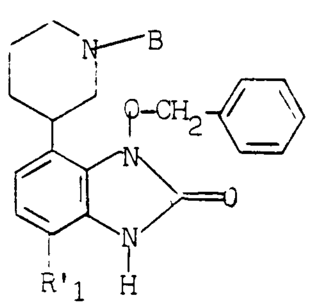

(VII)

dans laquelle B et $R'_1$ ont la signification déjà indiquée, dont <u>soit</u> on débloque les fonctions amines, pour obtenir un dérivé de formule ($I'_A$) :

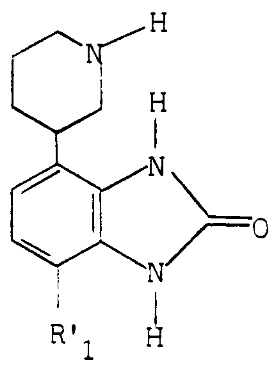

(I'$_A$)

dans laquelle R'$_1$ a la signification déjà indiquée, que soit l'on isole et, si désiré, salifie, soit l'on débloque sélectivement l'amine du cycle pipéridine, pour obtenir un dérivé de formule (VIII) :

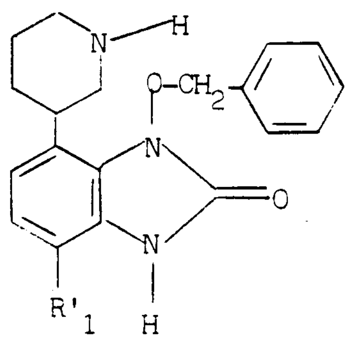

(VIII)

dans laquelle R'$_1$ a la signification déjà indiquée, que l'on soumet à l'action d'un agent capable de greffer un radical R', R' ayant la signification déjà indiquée, pour obtenir un dérivé de formule (IX) :

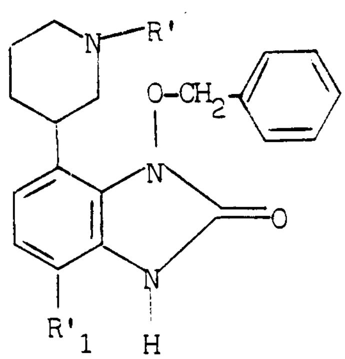

(IX)

dans laquelle R' et R'$_1$ ont la signification déjà indiquée, puis soumet lesdits dérivés de formules (VIII) et (IX) à une hydrogénation catalytique, pour obtenir un dérivé de formule (I'$_B$) :

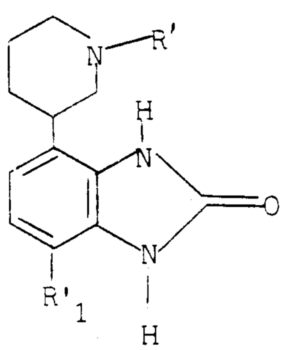

(I'$_B$)

dans laquelle R et $R'_1$ ont la signification déjà indiquée, que soit l'on isole et, si désiré, salifie, soit l'on soumet lesdits dérivés de formule ($I'_A$) et ($I'_B$) à l'action d'un agent de déblocage de l'hydroxyle, pour obtenir un dérivé de formule ($I'_C$) :

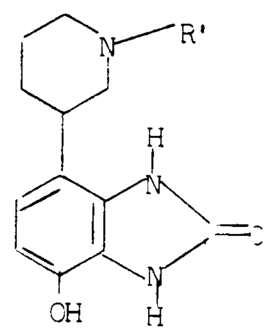

($I'_C$)

dans laquelle R a la signification déjà indiquée, que l'on isole et, si désiré, salifie, soit l'on débloque sélectivement pour le dérivé de formule (VII), l'amine du cycle imidazole, puis l'amine de la pipéridine et enfin l'hydroxyle, si désiré.

L'agent B qui représente un agent de blocage réversible d'une amine secondaire est de préférence un trifluoroacétate.

La réaction du dérivé de formule (II) avec l'halogénoformiate de formule (III) est réalisée de préférence en présence d'un base telle que la pyridine ou la triéthylamine, dans un solvant chloré tel que le chlorure de méthylène.

La nitration du dérivé de formule (IV) est réalisée de préférence à l'aide de tétrafluoroborate de nitronium dans un solvent tel que l'acétonitrile. On peut utiliser également d'autres agents de nitration tels que l'acide nitrique dans l'anhydride acétique ou l'acide sulfurique ou encore le mélange acide nitrique fumant - acide trifluoroacétique.

La réduction du groupement nitro des dérivés de formules (V), (V′) et (V″) est réalisée de préférence par hydrogénation catalytique en utilisant un catalyseur comme le Nickel de Raney ou de préférence le palladium sur charbon ou encore un métal réduit tel que le fer ou l'étain.

L'agent de déblocage de l'amine du cycle pipéridine est de préférence, dans le cas du radical trifluoroacétyle, une base minérale telle que la soude ou la potasse ou un carbonate alcalin tel que le carbonate de sodium ou de potassium.

L'agent capable de greffer un radical R′ est de préférence un halogénure de formule (X) :

Hal-R″ (X)

dans laquelle R″ a la signification de R déjà indiquée, à l'exception de l'hydrogène et du méthyle.

L'halogénure de formule (X) peut être un chlorure ou un bromure, mais de préférence un iodure. Il réagit avantageusement avec les amines secondaires de formule (V′) en présence d'un agent fixateur d'acide, comme par exemple, un carbonate alcalin tel que le carbonate de potassium dans un solvant tel que le diméthylformamide.

Pour fixer un radical R′ sur les dérivés de formules (V′) et (VI′), on peut opérer également par action desdits dérivés de formules (V′) et (VI′) avec un dérivé (selon le cas, un aldéhyde ou une cétone) de formule (XI) :

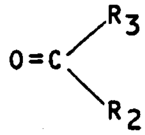

(XI)

dans laquelle $R_2$ et $R_3$ sont tels que

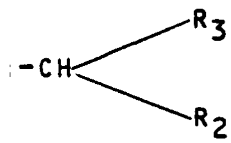

représente un radical alkyle renfermant de 1 à 5 atomes de carbone, en présence d'un agent de réduction (borohydrure ou cyanoborohydrure alcalin, tel que le borohydrure ou cyanoborohydrure de sodium ou hdyrogénation catalytique). C'est ainsi par exemple, que si le dérivé de formule (XI) est le formaldéhyde ou l'acétaldéhyde, on obtient respectivement pour R′, les valeurs méthyle et éthyle.

La cyclisation des dérivés de formules (VI), (VI′) et (VI″) est réalisée de préférence par hydrolyse alcaline, en utilisant comme base un hydroxyde alcalin, tel que le soude ou de préférence la potasse.

Lorsqu'on utilise un précurseur du phosgène pour préparer un dérivé de formule (I) dans laquelle $R_1$ représente un radical hydroxyle ou alkoxy, on utilise le chloroformiate de trichlorométhyle.

La réaction du dérivé de formule (III′) avec l'O-benzylhydroxylaminede préférence salifié sous forme de chlorhydrate est, dans ces conditions préférentielles, réalisée à température ambiante.

Le déblocage de l'amine de l'imidazole est de préférence réalisé par hydrogénation catalytique, à l'aide de platine rhodium ou notamment de nickel de Raney.

Le déblocage de l'amine de la pipéridine et les autres conditions réactionnelles ont été décrits ci-dessus.

Les dérivés de formule (II) peuvent être préparés par réduction d'un dérivé de formule (XII) :

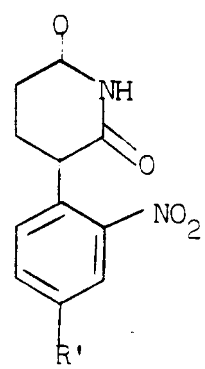

(XII)

dans laquelle R′ a la signification déjà indiquée, pour obtenir un dérivé de formule (XIII) :

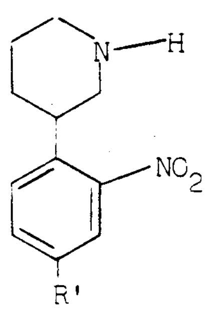

(XIII)

dans laquelle R′ a la signification déjà indiquée, dont on protège l'amine secondaire, pour obtenir un dérivé de formule (XIV) :

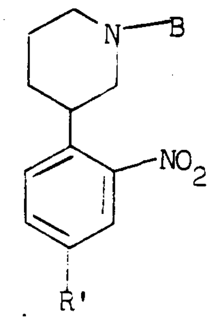

(XIV)

dans laquelle B et R′ ont la signification déjà indiquée, que l'on réduit, pour obtenir le dérivé de formule (II) cherché.

La réduction du dérivé de formule (XII) est réalisée de préférence par action de diboranne préparé in situ, par exemple à l'aide de borohydrure de sodium et d'éthérate de trifluorure de bore.

L'agent susceptible de bloquer de façon réversible l'amine de formule (XIII) est l'anhydride trifluoroacétique de préférence en présence d'une base dans le cas où l'on souhaite que B représente un radical trfluoroacétyle.

La réduction du dérivé de formule (XIV) est réalisée de préférence comme indiqué ci-dessus pour les dérivés de formules (V), (V′) et (V″).

Les dérivés de formule (XII) sont connus, par exemple par Synthesis 1061 (1984) ou peuvent être préparés par exemple par cyclisation d'un dérivé de formule (XV) ou (XVI) :

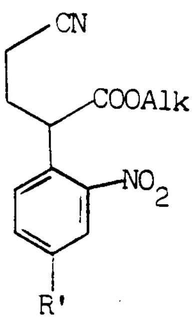

(XV)

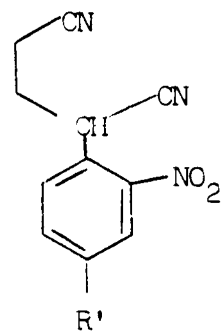

(XVI)

dans lesquelles Alk et R′ ont la signification déjà indiquée, par exemple, à l'aide d'acide sulfurique dans l'acide acétique.

Les produits de formule (XVI) peuvent, par exemple, être préparés par réaction d'un dérivé de formule (XVII) :

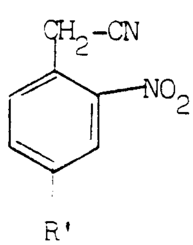

(XVII)

dans laquelle R′ a la signification déjà indiquée, avec l'acrylonitrile en présence d'hydroxyde de N-benzyltriméthyl ammonium.

Les dérivés de formule (I) présentent un caractère basique. On peut avantageusement préparer les sels d'addition des dérivés de formule (I) en faisant réagir, en proportions sensiblement stoechiométriques, un acide minéral ou organique avec ledit dérivé de formule (I). Les sels peuvent être préparés sans isoler les bases correspondantes.

Les dérivés, objet de la présente invention, possèdent de très intéressantes propriétés pharmacologiques.

On peut citer par exemple des propriétés dopaminergiques, des propriétés antihypertensives et vasodilatatrices, anti-anoxiques, anti-ulcéreuses, anti-émétiques.

Ces propriétés sont illustrées plus loin dans la partie expérimentale.

Ces propriétés justifient l'utilisation des dérivés de la benzimidazol-2-one ainsi que de leurs sels, à titre de médicaments.

La présente demande a ainsi également pour objet l'application à titre de médicaments, des dérivés de la benzimidazol-2-one, tels que définis par la formule (I) ainsi que de leurs sels d'addition avec les acides pharmaceutiquement acceptables.

Parmi les médicaments, objet de l'invention, on retient de préférence les médicaments caractérisés en ce qu'ils sont constitués par les nouveaux dérivés de la benzimidazol-2-one répondant à la formule (I) dans laquelle R représente un atome d'hydrogène ainsi que par leurs sels d'addition avec les acides pharmaceutiquement acceptables.

Parmi ceux-ci, on retient notamment ceux répondant à la formule (I) dans laquelle R représente un atome d'hydrogène ou un radical alkyle renfermant de 1 à 5 atomes de carbone ainsi que leurs sels d'addition avec les acides pharmaceutiquement acceptables.

Parmi ces derniers, on retient tout particulièrement la 1,3-dihydro 4-(1-propyl-3-pipéridinyl) 2H-benzimidazol-2-one et la 1,3-dihydro 4-hydroxy 7-(1-propyl 3-pipéridinyl) 2H-benzimidazol-2-one ainsi que leurs sels d'addition avec les acides minéraux ou organiques.

Les médicaments selon l'invention trouvent leur emploi, par exemple, dans le traitement des accidents vasculaires cérébraux, des troubles circulatoires périphériques tels que rétiniens ou néphrétiques, dans le glaucome ou des artériopathies des membres inférieurs. Ils sont également utilisables dans le traitement des insuffisances cardiaques de diverses étiologies, dans le traitement des ulcères gastriques ou duodénaux.

La dose usuelle, variable selon le dérivé utilisé, le sujet et l'affection en cause peut être, par exemple, de 5 mg à 200 mg par jour. Par voie orale, chez l'homme, le dérivé de l'exemple 1 peut être administré à la dose quotidienne de 10 mg à 200 mg, par exemple, pour le traitement de l'insuffisance cardiaque, soit environ de 0,15 mg à 3 mg par kilogramme de poids corporel.

L'invention a enfin pour objet les compositions pharmaceutiques qui renferment au moins un dérivé précité ou l'un de ses sels d'addition avec les acides pharmaceutiquement acceptables à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention s'étend en outre aux produits nouveaux, utiles notamment pour la préparation de dérivés répondant à la formule (I), à savoir les dérivés de formules (VI") et (VIII) :

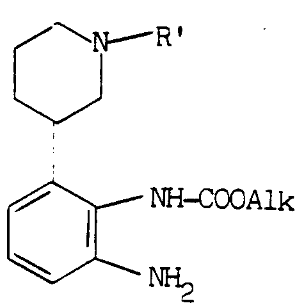

(VI")

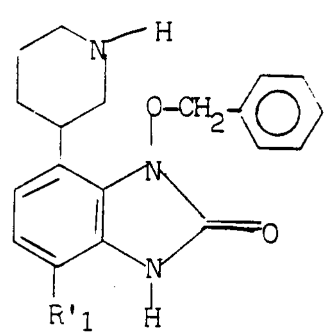

(VIII)

dans lesquelles R', $R'_1$ et Alk ont la signification déjà indiquée ci-dessus.

Les exemples qui suivent illustrent l'invention.

**Exemple 1 : 1,3-dihydro 4-(3-pipéridinyl) 2H-benzimidazol-2-one.**

**Stade A :** /2-/1-(trifluoroacétyl) 3-pipéridinyl/ phényl/carbamate d'éthyle.

On porte sous agitation pendant une heure 2,2 g de 3-(2-amino phényl) 1-(trifluoroacétyl) pipéridine dans 100 cm3 de chlorure de méthylène, 3 cm3 de pyridine et 1 cm3 de chloroformiate d'éthyle, lave avec de l'acide chlorhydrique 2N puis à l'eau, sèche, amène à sec sous pression réduite et obtient 2,8 g d'une huile utilisée telle quelle pour le stade suivant.

**Stade B :** /2-nitro 6-/1-(trifluoroacétyl) 3-pipéridinyl/phényl/ carbamate d'éthyle.

On refroidit à -30° une solution de 18 g de produit du Stade A dans 180 cm3 d'acétonitrile, ajoute sous agitation et atmosphère inerte, par petites fractions, 7,73 g de tétrafluoroborate de nitronium, après une heure d'agitation laisse revenir à température ambiante, verse sur de la glace, extrait au chlorure de méthylène, lave à l'eau, sèche, amène à sec sous pression réduite, purifie par chromatographie sur silice (éluant : cyclohexane - acétate d'éthyle 7-3) et obtient 10,8 g du produit attendu (Rf. = 0,2). F ≃ 124° C après recristallisation dans un mélange éther isopropylique-chlorure de méthylène (1-1).

**Stade C :** 2-amino 6-/1-(trifluoroacétyl) 3-pipéridinyl/phényl/ carbamate d'éthyle.

On hydrogène pendant 3 heurs, 12 g de produit du stade précédent dans 400 cm3 d'éthanol en présence de 3 g de palladium à 10% sur charbon, filtre, lave à l'éthanol, amène à sec sous pression réduite et obtient 10,8 g du produit attendu. F ≃ 96° C après recristallisation dans l'éther isopropylique.

**Stade D :** 1,3-dihydro 4-(3-pipéridinyl)2H-benzimidazol-2-one.

On porte sous agitation et atmosphère inerte pendant 4 heures à 80° C, 4 g de produit du stade précédent dans 80 cm3 de méthanol, 16 cm3 d'eau et 16 cm3 de lessive de potasse, évapore le méthanol sous pression réduite, ajoute 30 cm3 d'eau, ajuste le pH à 6-8 à l'aide d'acide acétique, amène à sec sous pression réduite, purifie par chromatographie sur silice (éluant : chlorure de méthylène - méthanoltriéthylamine 6-3-1) obtient 2,15 g de produit brut que l' on empâte dans l'éther éthylique, essore, sèche et recristallise dans l'éthanol, pour obtenir 1,23 g du produit attendu.
F≃ 260° C.

**Spectre U.V. (éthanol)**

Max. 229 nm $E_1^1$ = 276 $\epsilon$ = 6000
Max. 280 nm $E_1^1$ = 224
Max. 283 nm $E_1^1$ = 278 $\epsilon$ = 4950

PREPARATION DU PRODUIT DE DEPART DE L'EXEMPLE 1.

**Stade 1 :** 3-(2-nitrophényl) 1-(trifluoroacétyl) pipéridine.

REDUCTION.

On ajoute sous agitation et atmosphère inerte, 24 g de borohydrure de sodium à 95% à une solution de 25,9 g de 3-(2-nitrophényl) pipéridine 2,6-dione (décrit dans Synthesis 1061 (1984) dans 600 cm3 de tétrahydrofuranne, refroidit à 0° C, ajoute goutte à goutte en 45 minutes 97 cm3 d'éthérate de trifluorure de bore, laisse 16 heures sous agitation à température ambiante, refroidit à 0° C, élimine l'excès de diboranne par addition lente de 150 cm3 d'éthanol, ajoute 200 cm3 d'acide chlorhydrique 2N, élimine le tétrahydrofuranne sous pression réduite, reprend au chlorure de méthylène, décante, alcalinise à l'aide d'ammoniaque concentrée, extrait au chlorure de méthylène, lave à l'eau, sèche, amène à sec sous pression réduite et obtient 21 g du produit attendu.

BLOCAGE DE L'AMINE SECONDAIRE.

On refroidit à 0° C une solution partielle du produit ci-dessus dans 500 cm3 de chlorure de méthylène,

ajoute goutte à goutte en 25 minutes, 28,9 cm3 d'anhydride trifluoroacétique, laisse une heure 30 minutes sous agitation à température ambiante, refroidit à 0°C, ajoute 100 cm3 d'eau, puis par petites fractions, du bicarbonate de sodium jusqu'à neutralité décante, sèche, amène à sec sous pression réduite cristallise par addition d'éther isopropylique et obtient 23,7 g du produit attendu. F≃ 70°C après recristallisation dans l'éther isopropylique.

**Stade 2 :** 3-(2-amino phényl) 1-(trifluoroacétyl) pipéridine.

On hydrogène pendant 3 heures, 3,86 g de produit du stade 1 dans 150 cm3 d'éthanol en présence de 0,96 g de palladium à 10% sur charbon, filtre, lave à l'éthanol, amène à sec sous pression réduite, empâte dans l'éther éthylique, essore, sèche et obtient 3,2 g du produit attendu. F ≃ 138°C après recristallisation dans l'éther isopropylique.

**Exemple 2 : 1,3-dihydro 4-(1-propyl 3-pipéridinyl) 2H-benzimidazol-2-one et son acétate.**

**Stade A :** /2-amino 6-(1-propyl 3-pipéridinyl) phényl/ carbamate d'éthyle.

1) DEBLOCAGE DE L'AMINE.

On porte une heure sous agitation 4,7 g de produit du stade C de l'exemple 1 dans 80 cm3 de méthanol et 20 cm3 d'eau avec 8 g de carbonate de sodium, extrait au chlorure de méthylène, lave à l'eau, sèche, amène à sec sous pression réduite et obtient 3,2 g du produit attendu.

2) ALKYLATION

On ajoute sous agitation et atmosphère inerte, 0,74 cm3 de propionaldéhyde à une solution de 2,45 g du produit ci-dessus et 635 mg de cyanoborohydure de sodium dans 70 cm3 de méthanol, après 16 heures d'agitation, verse dans l'eau glacée, extrait au chlorure de méthylène, sèche, amène à sec sous pression réduite, purifie par chromatographie sur silice (éluant : chlorure de méthylène - méthanol 98-2) et obtient 1,95 g du produit attendu.

Spectre U.V. (éthanol)

Max. 237 nm $E_1^1$ = 256 $\epsilon$ = 7800
Max. 289 nm $E_1^1$ = 83 $\epsilon$ = 2550

**Stade B :** 1,3-dihydro 4-(1-propyl 3-pipéridinyl) 2H-benzimidazol-2-one et son acétate.

On porte pendant 5 heures sous agitation à 80°C, 1,4 g de produit du stade A dans 30 cm3 de méthanol, 6 cm3 d'eau et 6 cm3 de lessive de potasse, évapore le méthanol, ajoute 6 cm3 d'eau, amène le pH à 6,4 à l'aide d'acide chlorohydrique, amène à sec sous pression réduite, purifie par chromatographie sur silice (éluant : chlorure de méthylène - méthanol 98-2) et obtient 1,02 g du produit attendu sous forme de base.

FORMATION DE L'ACETATE.

On ajoute 2,5 cm3 d'acide acétique à une solution de 0,92 g de base ci-dessus dans 10 cm3 de chlorure de méthylène, amène à sec sous pression réduite, reprend par 15 cm3 d'eau, filtre, lyophilise et obtient 850 mg de l'acétate attendu.

Spectre U.V. (éthanol)

Max. 230 nm $E_1^1$ = 245 $\epsilon$ = 7800
Max. 283 nm $E_1^1$ = 201 $\epsilon$ = 6400
(Ethanol - NaOH 0,1N)
Max. 246 nm $E_1^1$ = 191 $\epsilon$ = 6100
Max. 287 nm $E_1^1$ = 232 $\epsilon$ = 7400

**Exemple 3 : 1,3-dihydro 4-(1-méthyl 3-pipéridinyl) 2H-benzimidazol-2-one et son chlorhydrate.**

On opère comme indiqué à l'exemple 2, mais en opérant l'alkylation à l'aide de formaldéhyde, pour obtenir le produit attendu. F > 250 ° C (chlorhydrate).

Spectre RMN (DMSO) 60MHz (chlorhydrate)

| | |
|---|---|
| H du N-méthyl | 2,2 ppm |
| H aromatiques | 6,9 ppm |
| autres protons du cycle | 1,5 à 2,9 ppm |
| H mobiles | 10,6 à 10,8 ppm |

**Exemple 4 : Bromhydrate de 1,3-dihydro 4-hydroxy 7-(3-pipéridinyl) 2H-benzimidazol-2-one.**

**Stade A :** 3-(3-isocyanate 4-méthoxy phényl) 1-trifluoroacétyl pipéridine.

On mélange 6,04 g de 3-(3-amino 4-méthoxy phényl) 1-trifluoroacétyl pipéridine dans 120 cm3 de dioxanne et 2,9 cm3 de chloroformiate de trichlorométhyle. On agite 4 heures à 80 ° C puis amène à sec sous pression réduite. On chromatographie le résidu sur silice, élue par un mélange cyclohexanne - acétate d'éthyle (7-3) et obtient 6,4 g de produit brut utilisé tel quel pour le stade suivant.

**Stade B :** 3-(3-///(phényl méthoxy) amino/ carbonyl/ amino/ 4-méthoxyphényl) 1-trifluoroacétyl pipéridine.

On met en suspension 1,68 g de chlorhydrate de 0-benzyl hydroxylamine dans 30 cm3 de chlorure de méthylène. On ajoute 1,52 cm3 de triéthylamine puis goutte à goutte une solution de 3,2 g de produit obtenu au stade A dans 10 cm3 de chlorure de méthylène. On agite 2 heures et amène à sec sous pression réduite.

On chromatographie le résidu sur silice (éluant : chlorure de méthylène - acétate d'éthyle 96-4) et obtient 3,75 g de produit attendu.
F = 134 ° C après recristallisation dans un mélange 1-1 (chlorure de méthylène - éther isopropylique.

**Stade C :** 1,3-dihydro 4-méthoxy 1-(phényl méthoxy) 7-/1-(trifluoroacétyl 3-pipéridinyl/ 2H benzimidazol-2-one.

On refroidit à 0 ° C, 3,7 g du produit obtenu ci-dessus dans 90 cm3 de chlorure de méthylène, ajoute goutte à goutte une solution de tétracétate de plomb dans 50 cm3 de chlorure de méthylène anhydre. On laisse une heure sous agitation, filtre le précipité formé et lave le filtrat à l'eau, sèche et amène à sec sous pression réduite. On chromatographie le résidu sur silice, élue par un mélange chlorure de méthylène - méthanol (95-5) et obtient 2,88 g de produit attendu. F = 192 ° C après recristallisation dans un mélange (20-15) éther isopropylique - chlorure de méthylène.

**Stade D :** 1,3-dihydro 4-méthoxy 7-/1-(trifluoroacétyl) 3-pipéridinyl/ 2H-benzimidazol-2-one.

On laisse sous hydrogène pendant 12 heures 2,1 g de produit obtenu ci-dessus dans 70 cm3 d'éthanol et 2 g de Nickel de Raney. On filtre et amène à sec le filtrat sous pression réduite. On recristallise le résidu dans un mélange (15-5) chlorure de méthylène - éther isopropylique et obtient 0,935 g de produit attendu. F≃260 ° C.

Spectre U.V. dans EtOH

| | | |
|---|---|---|
| Max. 214 nm | $E_1^1$ | = 1981 $\epsilon$ = 68000 |
| Inflex.237 nm | $E_1^1$ | = 361 |
| Inflex.246 nm | $E_1^1$ | = 227 |
| Inflex.248 nm | $E_1^1$ | = 189 |
| Max. 270 nm | $E_1^1$ | = 63 $\epsilon$ = 2150 |
| Max. 279 nm | $E_1^1$ | = 46 $\epsilon$ = 1600 |

**Stade E :** Bromhydrate de 1,3-dihydro 4-hydroxy 7-(3-pipéridinyl) 2H-benzimidazol-2-one.

On agite une heure 1,1 g de produit obtenu ci-dessus dans 15 cm3 de méthanol, 5 cm3 d'eau et 680 mg de carbonate de sodium. On ajoute quelques cm3 d'eau, extrait au chlorure de méthylène, sèche et amène à sec sous pression réduite. On reprend le résidu par 50 cm3 de chlorure de méthylène, refroidit à -30 ° C et ajoute goutte à goutte 9,6 cm3 de tribromure de bore en solution M dans le chlorure de méthylène.

On laisse 3 heures sous agitation, refroidit à 0 ° C et ajoute 30 cm3 de méthanol. Après 15 minutes à température ambiante, on amène à sec sous pression réduite. On reprend le résidu par du chlorure de méthylène, essore et obtient 840 mg de produit attendu. F > 250 ° C. Après recristallisation du bromhydrate dans le méthanol, on obtient 600 mg de produit attendu.

Spectre U.V. dans EtOH

| | | |
|---|---|---|
| Max. 212 nm | $E_1^1$ | = 1924 $\epsilon$ = 60500 |
| Inflex.233 nm | $E_1^1$ | = 311 |
| Max. 270 nm | $E_1^1$ | = 51 $\epsilon$ = 1600 |
| Inflex. 280 nm | $E_1^1$ | = 30 |

dans EtOH/NaOH 0,1N

| | | |
|---|---|---|
| Max. 250 nm | $E_1^1$ | = 341 $\epsilon$ = 10700 |
| Inflex.288 nm | $E_1^1$ | = 30 |

Spectre RMN (DMSO)

| | |
|---|---|
| 1,8 ppm | H du $CH_2$ en béta et alpha de N |
| 2,77 à 3,55 ppm - | H du $CH_2$ $N^{\oplus}$ et CH $\Phi$ |
| 6,33 - 6,42 | |
| 6,52 - 6,61 | H aromatiques |
| 8,14 - 8,84 8,98 | |
| 10,4 -10,62 | H mobiles. |

**Exemple 5 : bromhydrate de 1,3-dihydro 4-hydroxy 7-(1-propyl 3-pipéridinyl) 2H-benzimidazol-2-one.**

**Stade A :** 1,3-dihydro 4-méthoxy 1-(phénylméthoxy) 7-(3-pipéridinyl) 2H-benzimidazol-2-one.

On ajoute en 5 minutes 33 cm3 de soude 2N à une solution refroidie de 5 g de produit obtenu comme au stade C de l'exemple 4 dans 500 cm3 d'éthanol. On agite 1 heure à température ambiante, neutralise judqu'à pH 6 avec de l'acide chlorhydrique N, extrait avec un mélange chlorure de méthylène-méthanol (9-1), sèche et élimine les solvants sous pression réduite ; on obtient 4,5 g de produit attendu.

**Stade B :** 1,3-dihydro 4-méthoxy 1-(phénylméthoxy) 7-(1-propyl 3-pipéridinyl) 2H-benzimidazol-2-one.

Dans une solution de 4,39 g de produit obtenu au stade A dans 150 cm3 de méthanol, on ajoute 1,076 cm3 de propionaldéhyde et 0,937 g de cyanoborohydrure de sodium et agite 4 heures à témérature ambiante. On ajoute 50 cm3 d'eau et extrait au chlorure de méthylène, sèche et élimine les solvants sous pression réduite. On obtient 2,2 g de produit attendu, après chromatographie sur silice (éluant : chlorure de méthylène-méthanol 9-1).

Spectre IR ($CHCl_3$)

=C-NH : 3461 $cm^{-1}$
Bandes de Bohlman
$\rangle$=O : 1718 $cm^{-1}$

aromatique : 1640 - 1525 - 1500 $cm^{-1}$

**Stade C :** 1,3-dihydro 4-méthoxy 7-(1-propyl 3-pipéridinyl) 2H-benzimidazol-2-one.

On hydrogène pendant 2 heures et quart 2,2 g du produit obtenu au stade B dans 30 cm3 d'éthanol en présence de 3,5 g de nickel de Raney ; on filtre, amène à sec le filtrat sous pression réduite et obtient 1,5 g de produit brut que l'on chromatographie sur silice (éluant : chlorure de méthylène-méthanol 9-1) et recueille 1,1 g de produit pur.

Spectre IR ($HCl_3$)

-NH : 3470 $cm^{-1}$ (+ associé)

-C- (=O) : 1698 $cm^{-1}$

aromatique : 1642 - 1526 - 1512 $cm^{-1}$

**Stade D :** bromhydrate de 1,3-dihydro 4-hydroxy 7-(1-propyl 3-pipéridinyl) 2H-benzimidazol-2-one.

On refroidit à O¤/+5¤C 1 g de produit obtenu au stade C dans 100 cm3 de chlorure de méthylène, et ajoute goutte à goutte en 5 minutes 24 cm3 d'une solution chlorométhylénique de tribromure de bore (M). On agite 4 heures à température ambiante, refroidit à 0¤C, ajoute lentement 50 cm3 de méthanol et élimine les solvants sous pression réduite ; on reprend le résidu dans le méthanol, filtre et obtient 1,05 g de produit attendu. On purifie 0,75 g de ce produit par chromatographie sur silice (éluant : chlorure de méthylène-méthanol-triéthylamine 8-1-1) et obtient 0,36 g de produit pur que l'on recristallise dans le méthanol .

Spectre UV dans EtOH

max. 213 nm $E^1_1$ = 1649 = 58 800
infl. 233 nm $E^1_1$ = 274
max. 271 nm $E^1_1$ = 44 = 1 550
infl. 279 nm $E^1_1$ = 25

**Exemple 6 : bromhydrate de 1,3-dihydro 4-hydroxy 7-(1-méthyl 3-pipéridinyl) 2H-benzimidazol-3-one.**

**Stade A :** 1,3-dihydro 4-méthoxy 7-(1-méthyl-3-pipéridinyl) 1-(phénylméthoxy) 2H-benzimidazol-2-one.

On opère comme au stade B de l'exemple 5 à partir de 4,4 g de produit obtenu comme au stade A de l'exemple 5 et en utilisant 1,9 cm3 de formaldéhyde. On obtient 3,6 g de produit brut que l'on purifie par double extraction (HCl 2N puis chlorure de méthylène). On recueille 1,7 g de produit attendu.

Spectre IR ($CHCl_3$)

=C-NH : 3461 $cm^{-1}$
Bandes de Bohlman
>C=O : 1719 $cm^{-1}$
aromatique : 1640 - 1525 $cm^{-1}$

**Stade B :** 1,3-dihydro 4-méthoxy 7-(1-méthyl 3-pipéridinyl) 2H-benzimidazol-2-one.

On opère comme à l'exemple 5, stade C,à partir de 1,6 g de produit obtenu comme au stade A précédent en présence de 2 g de nickel de Raney. On obtient 1,1 g de produit que l'on dissout dans un mélange éthanol-chlorure de méthylène, concentre et recristallise par addition d'éther. F > 250¤C.

Spectre IR

| | |
|---|---|
| NH | : 3470 $cm^{-1}$ + absorption OH/NH associé |
| C=O | : 1697 $cm^{-1}$ |
| aromatique | : 1640 - 1525 - 1512 $cm^{-1}$ |

**Stade C :** bromhydrate de 1,3-dihydro 4-hydroxy 7-(1-méthyl 3-pipéridinyl) 2H-benzimidazol-3-one.

On opère comme au stade D de l'exemple 5 à partir de 0,7 g de produit obtenu comme au stade B et 20 cm3 de tribromure de bore. On obtient 0,630 g de produit attendu que l'on purifie par recristallisation dans le méthanol.

Spectre UV dans EtOH

| | | |
|---|---|---|
| max. 212 nm | $E^1_1$ = 1796 | $\epsilon$ = 58 900 |
| infl. 232 nm | $E^1_1$ = 299 | |
| infl. 244 nm | $E^1_1$ = 174 | |
| max. 271 nm | $E^1_1$ = 48 | $\epsilon$ = 1 600 |
| infl. 279 nm | $E^1_1$ = 28 | |

**Exemple 7 :**

On a préparé des comprimés répondant à la formule :

| | |
|---|---|
| - 1,3-dihydro 4-(3-pipéridinyl) 2H-benzimidazol-2-one<br>- Excipient q.s. pour un comprimé terminé à | 10 mg<br>100 mg |
| (Détail de l'excipient : lactose, amidon, talc, stéarate de magnésium). | |

**Exemple 8 :**

On a préparé des comprimés répondant à la formule :

| | |
|---|---|
| - acétate de 1,3-dihydro 4-(1-propyl 3-pipéridinyl 2H-benzimidazol-2-one<br>- Excipient q.s. pour un comprimé terminé à | 10 mg<br>100 mg |
| (Détail de l'excipient : lactose, amidon, talc, stéarate de magnésium). | |

**ETUDE PHARMACOLOGIOUE**

**1) Comportement de rotation après lésion unilatérale du faisceau nigrostriatal par la 6-hydroxydopamine.**

Technique :

Des rats mâles de 220 g environ sont lésés dans le faisceau dopaminergique nigrostrié selon la méthode de Ungerstedt (Acta. physiol. Scand. 1971, 82 suppl. 367-69 93) modifiée, par injection unilatérale de 9,2 g de chlorhydrate de 6-hydroxydopamine en solution dans 4 µl de soluté physiologique contenant 0,4 mg/ml d'acide ascorbique.

Les produits étudiés sont administrés par voie intrapéritonéale. Les animaux traités sont placés individuellement dans un rotamètre qui permet de compter le nombre de rotations effectuées par chaque animal dans le sens opposé au côté lésé.

Dans les conditions de l'expérience, les résultats suivants ont été obtenus :

Le produit de l'exemple 4 est inactif à la dose de 5 mg/kg et n'induit un comportement de rotations qu'à partir de la dose de 10 mg/kg, compte-tenu de la très grande sensibilité de ce modèle, l'activité agoniste dopaminergique centrale de ce composé est donc faible.

**2) Test de mise en évidence d'une activité vasodilatatrice directe (ou activité post-synaptique relaxante vasculaire).**

Les rats de race Sprague-Dawley d'un poids de 320-350 g sont anesthésiés au pentobarbital (50 mg/kg par injection intrapéritonéale). Après mise en place d'un cathéter carotidien destiné à la mesure de la pression artérielle et d'un cathéter jugulaire destiné à l'injection des molécules à étudier ainsi que des antagonistes éventuels, les animaux sont démédullés à l'aide d'une tige d'acier. Cette tige est introduite par le trou orbitaire droit et descendue sur toute la longueur de la moelle épinière. Les animaux sont ensuite placés immédiatement sous respiration assistée. Quelques minutes après stabilisation de la pression artérielle, les animaux reçoivent une perfusion intraveineuse d'angiotensine II de 0,50 ug/kg minute de façon à augmenter leur pression moyenne d'environ 100 mm de mercure. Après obtention d'un plateau, la substance à étudier est injectée par voie intraveineuse chaque 2 minutes et en doses cumulées. Les pourcentages de baisse de la pression artérielle moyenne ainsi obtenus permettent de tracer une courbe dose-réponse reflétant l'activité vasodilatatrice de la molécule étudiée. Le traitement préalable des animaux par différents antagonistes permet de cerner le mécanisme d'action de cette molécule.

Les produits sont testés entre 0,001 et 1 mg/kg. On utilise comme antagonistes la dompéridone et le propanolol.

On peut conclure que le produit de l'exemple 4 a un effet vasodilatateur dose dépendant dès 0,03 mg/kg.

**3) Etude de la toxicité aiguë.**

On a évalué les doses létales $DL_0$ des différents composés testés après administration par voie orale chez la souris.

On appelle $DL_0$ la dose maximale ne provoquant aucune mortalité en 8 jours.

Les résultats obtenus sont les suivants :

| Produit de l'exemple | $DL_0$ en mg/kg |
|---|---|
| 1 | > 400 |
| 4 | > 400 |

**4) Affinités pour les récepteurs dopaminergiques.**

On homogénéise au vingtième (poids/volume) dans le sucrose 0,32M, les corps striés prélevés des cerveaux de 6 rats mâles pesant 150 g en moyenne. Après centrifugation du mélange homogénéisé à 1 000 g pendant 10 minutes à 0¤C, le surnageant est centrifugé à 30 000 g pendant 15 minutes à +4¤C. Le culot est repris dans 25 ml de tampon Tris HCl 50mM pH 7,7 et centrifugé à 30 000 g pendant 15 minutes à +4¤C. Le nouveau culot est repris dans 50 ml de tampon Krebs Tris HCl pH 7,3 et la suspension est préincubée pendant 10 minutes à 37¤C. On fait ensuite incuber pendant 20 minutes au bain-marie à +37¤C en présence de spinopéridol 3H, seul, avec un excès d'halopéridol et avec des concentrations croissantes du produit à tester. Les suspensions incubées sont filtrées sur Whatman GF/C et les filtres sont lavés par trois fois 5 ml de tampon Tris HCl 50 mM. La radioactivité des filtres est mesurée par scintillation liquide.

La fixation non spécifique est déterminée parallèlement par incubation de spiropéridol 3H en présence d'un excès d'halopéridol.

On a obtenu les résultats suivants :

Le résultat est exprimé directement en concentrations inhibitrices 50% ($CI_{50}$ : concentration du produit étudié, exprimée en nM, nécessaire pour déplacer 50% de la radioactivité spécifique fixée sur le récepteur

étudié).

| Produit de l'exemple | $CI_{50}$ (nM) |
|---|---|
| 1 | 172 |
| 4 | 300 |

**5) Etude de l'activité hypotensive sur le rat normotendu anesthésié.**

Des rats mâles Sprague-Dawley (CR) sont anesthésiés par voie intrapéritonéale au pentobarbital sodique (60 mg/kg).

Une veine jugulaire est cathétérisée par l'injection du produit, et une artère carotide est cathétérisée pour l'enregistrement de la pression artérielle.

Le produit à tester est mis en solution dans 10% d'éthanol puis injecté sous un volume de 1 cm3/kg.

La pression est notée au temps 5 minutes et 30 minutes après l'injection du produit.

Le tableau ci-après indique les variations exprimées en pourcentage de la pression artérielle après administration du produit testé par rapport à la pression artérielle témoin initiale.

Résultats :

| Produit de l'exemple | dose mg/kg | 5 mn après administration | 30 mn après administration |
|---|---|---|---|
| 5 | 10 | -23 | -21 |
| | 1 | -19 | -19 |
| 4 | 0,1 | | -17 |

**Revendications**

**Revendications pour les Etats contractants suivants : AT, BE, CH, DE, FR, GB, IT, LI, LU, NL, SE**

1. Les dérivés de la benzimidazol-2-one ainsi que leurs sels d'addition avec les acides minéraux ou organiques, caractérisés en ce qu'ils répondent à la formule générale (I) :

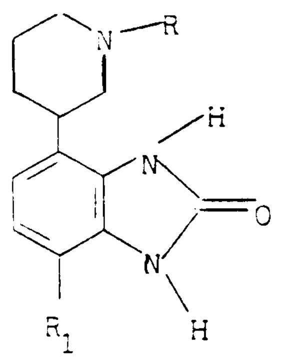

(I)

dans laquelle R représente un atome d'hydrogène, un radical alkyle renfermant de 1 à 5 atomes de carbone, un radical cycloalkylalkyle renfermant de 4 à 7 atomes de carbone, un radical aralkyle renfermant de 7 à 12 atomes de carbone, éventuellement substitué par un ou plusieurs radicaux choisis dans le groupe constitué par les radicaux alkyles renfermant de 1 à 5 atomes de carbone, les radicaux alkoxy renfermant de 1 à 5 atomes de carbone et les atomes d'halogène ou R représente un radical alkényle ou un radical alkynyle renfermant de 3 à 5 atomes de carbone, étant entendu que la liaison multiple ne peut pas se trouver entre les carbones en alpha et béta de l'atome d'azote et $R_1$ représente un atome d'hydrogène, un radical hydroxy, un radical alkoxy renfermant de 1 à 5 atomes de carbone, un radical phénylalkyloxy renfermant de 7 à 9 atomes de carbone ou un radical phénoxy.

**2.** Les dérivés de la benzimidazol-2-one tels que définis par la formule (I) de la revendications 1, ainsi que leurs sels d'addition avec les acides minéraux ou organiques, caractérisés en ce que $R_1$ représente un atom d'hydrogène.

**3.** Les dérivés de la benzimidizol-2-one selon la revendication 2, ainsi que leurs sels d'addition avec les acides minéraux ou organiques, caractérisés en ce que R représente un atome d'hydrogène ou un radical alkyle renfermant de 1 à 5 atomes de carbone.

**4.** Les dérivés de formule (I) selon la revendication 1, dont les noms suivent :
- la 1,3-dihydro 4-(1-propyl-3-pipéridinyl) 2H-benzimidazol-2-one,
- la 1,3-dihdyro 4-hydroxy 7-(1-propyl 3-pipéridinyl) 2H-benzimidazol-2-one,

ainsi que leurs sels d'addition avec les acides minéraux ou organiques.

**5.** Procédé de préparation des dérivés de la benzimidazol-2-one , tels que définis par la formule (I) de la revendication 1, dans laquelle $R_1$ représente un atome d'hydrogène ainsi que de leurs sels, caractérisé en ce que l'on fait réagir un dérivé de formule (II) :

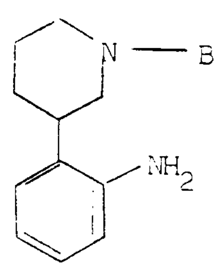

(II)

dans laquelle B représente un agent de blocage réversible d'une amine secondaire avec un halogénoformiate d'alkyle de formule (III) :

Hal-COO-Alk (III)

dans laquelle Hal représente un atome de chlore, de brome ou d'iode et Alk représente un radical alkyle renfermant de 1 à 5 atomes de carbone, pour obtenir un dérivé de formule (IV) :

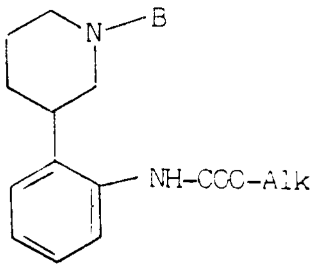

(IV)

dans laquelle B et Alk ont la signification déjà indiquée, que l'on soumet à une nitration, pour obtenir un dérivé de formule (V) :

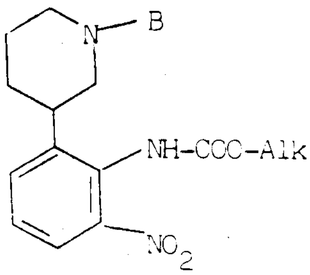

(V)

dans laquelle B et Alk ont la signification déjà indiquée dont soit l'on réduit le groupement nitro, pour obtenir un dérivé de formule (VI) :

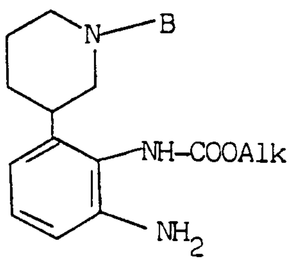

(VI)

dans laquelle B et Alk ont la signification déjà indiquée soit l'on soumet ledit dérivé de formule (V) ou ledit dérivé de formule (VI) à l'action d'un agent de déblocage de l'amine, du cycle pipéridine, pour obtenir respectivement un dérivé de formule (V') ou (VI') :

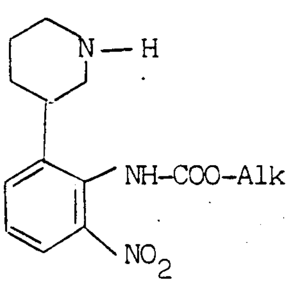

(V')

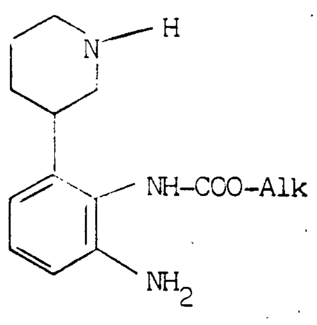

(VI')

dans lesquelles Alk a la signification déjà indiquée, que l'on soumet à l'action d'un agent capable de greffer un radical R', R' ayant la signification de R à l'exception de l'atome d'hydrogène, pour obtenir respectivement des dérivés de formules (V") ou (VI") :

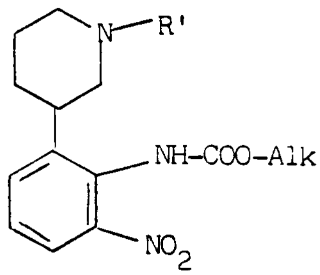

(V")

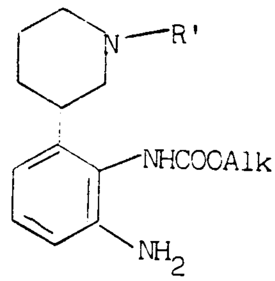

(VI")

dans lesquelles Alk a la signification déjà indiquée, puis réduit le groupement nitro desdits dérivés de formule (V′) et (V″), pour obtenir respectivement les dérivés de formules (VI′) et (VI″) que l'on cyclise par hydrolyse, pour obtenir un dérivé de formule ($I_A$) :

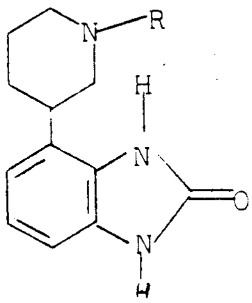

($I_A$)

dans laquelle R a la signification déjà indiquée, que l'on isole et, désiré, salifie, soit l'on cyclise le dérivé de formule (VI) comme les dérivés de formules (VI') et (VI"), puis débloque l'amine secondaire du cycle pipéridine, pour obtenir un dérivé de formule ($I_A$) dans laquelle R représente un atome d'hydrogène, que l'on peut éventuellement substituer, pour obtenir d'autres valeurs de R.

**6.** Procédé selon la revendication 4, caractérisé en ce que :
- B est un radical trifluoroacétyle ;
- la nitration du dérivé de formule (IV) est réalisée à l'aide de tétrafluoroborate de nitronium.

**7.** Procédé de préparation des dérivés tels que définis par la formule (I) de la revendication 1, dans laquelle $R_1$ a la signification indiquée à la revendication 1 à l'exception d'un atome d'hydrogène ainsi que de leurs sels, caractérisé en ce que l'on fait réagir un dérivé de formule (II') :

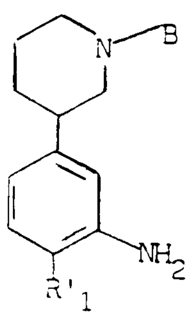

(II')

dans laquelle B a la signification indiquée à la revendication 5 et $R'_1$ a la signification de $R_1$ indiquée à la revendication 1, à l'exception de l'atome d'hydrogène et du radical hydroxyle, avec le phosgène ou un précurseur de phosgène, pour obtenir un dérivé de formule (III') :

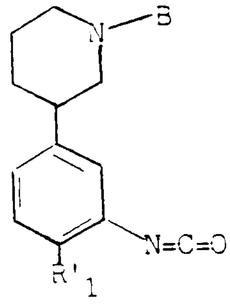

(III')

dans laquelle B et $R'_1$ ont la signification déjà indiquée, que l'on fait réagir avec l'O-benzylhydroxylamine, pour obtenir un dérivé de formule (IV') :

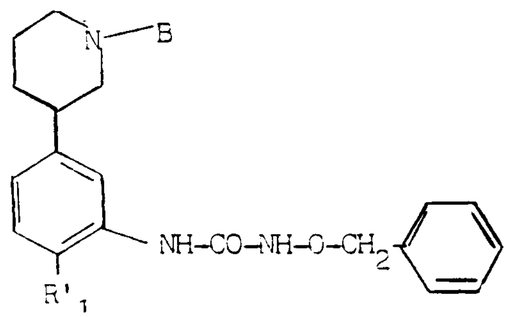

(IV')

dans laquelle B et $R'_1$ ont la signification déjà indiquée, que l'on cyclise, pour obtenir un dérivé de formule (VII) :

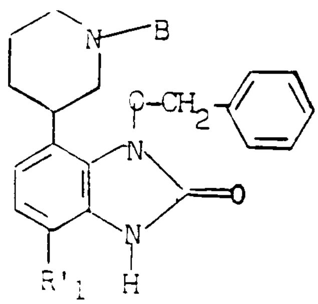

(VII)

dans laquelle B et $R'_1$ ont la signification déjà indiquée, dont soit on débloque les fonctions amines, pour obtenir un dérivé de formule ($I'_A$) :

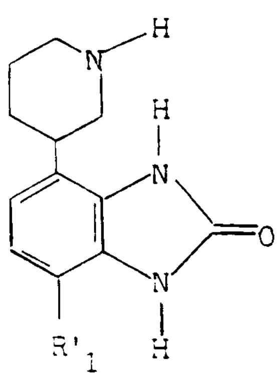

($I'_A$)

dans laquelle $R'_1$ a la signification déjà indiquée, que soit l'on isole et, si désiré, salifie, soit l'on débloque sélectivement l'amine du cycle pipéridine, pour obtenir un dérivé de formule (VIII) :

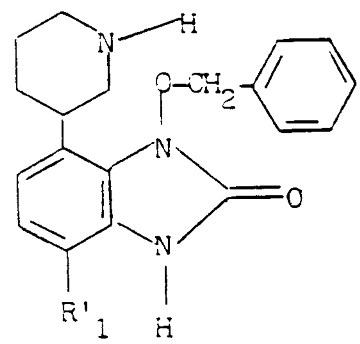

(VIII)

dans laquelle $R'_1$ a la signification déjà indiquée, que l'on soumet à l'action d'un agent capable de greffer un radical R', R' ayant la signification indiquée, à la revendication 5 pour obtenir un dérivé de formule (IX) :

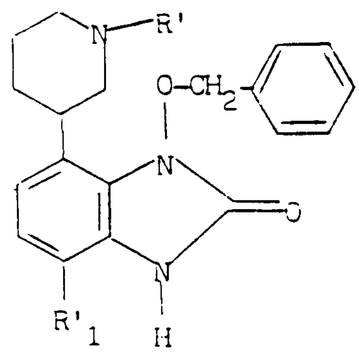

(IX)

dans laquelle R' et $R'_1$ ont la signification déjà indiquée, puis soumet desdits dérivés de formules (VIII) et (IX) à une hydrogénation catalytique, pour obtenir un dérivé de formule ($I'_B$) :

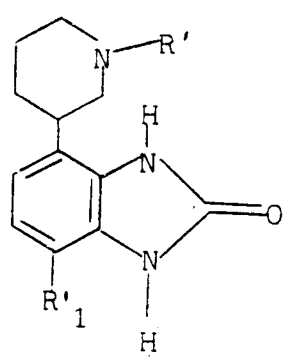

($I'_B$)

dans laquelle R′ et $R'_1$ ont la signification déjà indiquée, que soit l'onisole et, si désiré, salifie, soit l'on soumet lesdits dérivés de formule ($I'_A$) et ($I'_B$) à l'action d'un agent de déblocage de l'hydroxyle, pour obtenir un dérivé de formule ($I'_C$) :

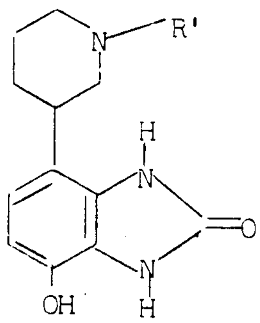

(I'c)

dans laquelle R′ a la signification déjà indiquée, que l'on isole et, si désiré, salifie, soit l'on débloque sélectivement pour le dérivé de formule (VII), l'amine du cycle imidazole, puis l'amine de la pipéridine et enfin l'hydroxyle, si désiré.

**8.** Médicaments, caractérisés en ce qu'ils sont constitués par les nouveaux dérivés de la benzimidazol-2-one, tels que définis par la formule (I) de la revendication 1, ainsi que par leurs sels d'addition avec les acides pharmaceutiquement acceptables.

**9.** Médicaments, caractérisés en ce qu'ils sont constitués par les nouveaux dérivés de la benzimidazol-2-one, tels que définis dans l'une des revendications 2 ou 3, ainsi que par leurs sels d'addition avec les acides pharmaceutiquement acceptables.

**10.** Médicaments, caractérisés en ce qu'ils sont constitués par les dérivés de la benzimidazol-2-one, tels que définis à la revendication 4, ainsi que par leurs sels d'addition avec les acides pharmaceutiquement acceptables.

**11.** Compositions pharmaceutiques, caractérisées en ce qu'elles renferment à titre de principe actif, l'un au moins des médicaments, tels que définis à l'une des revendications 8, 9 ou 10.

**12.** Les dérivés de formules (VI") et (VIII) :

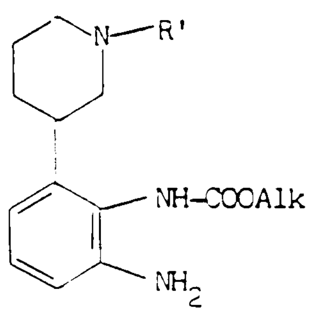

(VI")

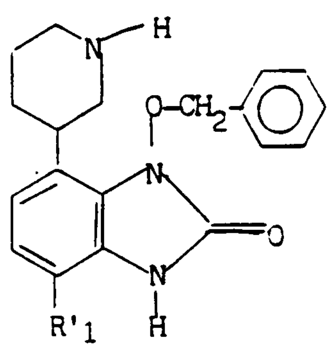

(VIII)

dans lesquelles R' et Alk ont la signification déjà indiquée à la revendication 5 et $R'_1$ a la signification indiquée à la revendication 7.

**Revendications pour l'Etat contractant suivant : ES**

**1.** Procédé de préparation des dérivés de la benzimidazol-2-one ainsi que de leurs sels d'addition avec les acides minéraux ou organiques, répondant à la formule générale (I) :

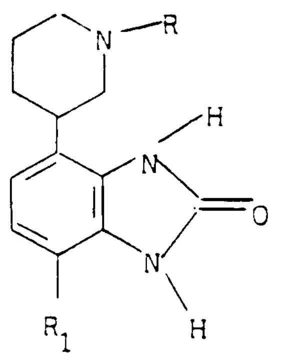

(I)

dans laquelle R représente un atome d'hydrogène, un radical alkyle renfermant de 1 à 5 atomes de carbone, un radical cycloalkylalkyle renfermant de 4 à 7 atomes de carbone, un radical aralkyle renfermant de 7 à 12 atomes de carbone, éventuellement substitué par un ou plusieurs radicaux choisis dans le groupe constitué par les radicaux alkyles renfermant de 1 à 5 atomes de carbone, les radicaux alkoxy renfermant de 1 à 5 atomes de carbone et les atomes d'halogène ou R représente un radical alkényle ou un radical alkynyle renfermant de 3 à 5 atomes de carbone, étant entendu que la liaison multiple ne peut pas se trouver entre les carbones en alpha et béta de l'atome d'azote et $R_1$ représente un atome d'hydrogène, un radical hydroxy, un radical alkoxy renfermant de 1 à 5 atomes de carbone, un radical phénylalkyloxy renfermant de 7 à 9 atomes de carbone ou un radical phénoxy, caractérisé en ce que :

a) pour préparer les dérivés de formule (I), dans laquelle $R_1$ représente un atome d'hydrogène, ainsi que leurs sels, l'on fait réagir un dérivé de formule (II) :

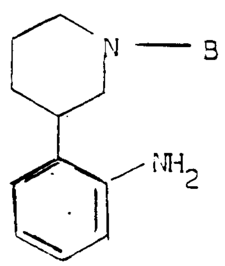

(II)

dans laquelle B représente un agent de blocage réversible d'une amine secondaire avec un halogénoformiate d'alkyle de formule (III) :

Hal-COO-Alk (III)

dans laquelle Hal représente un atome de chlore, de brome ou d'iode et Alk représente un radical alkyle renfermant de 1 à 5 atomes de carbone, pour obtenir un dérivé de formule (IV) :

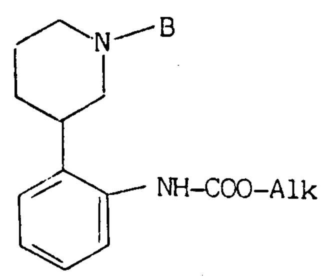

(IV)

dans laquelle B et Alk ont la signification déjà indiquée, que l'on soumet à une nitration, pour obtenir un dérivé de formule (V) :

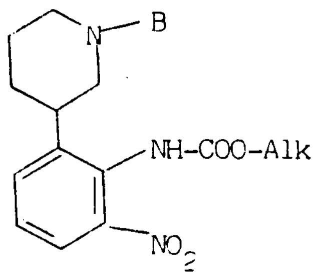

(V)

dans laquelle B et Alk ont la signification déjà indiquée dont soit l'on réduit le groupement nitro, pour obtenir un dérivé de formule (VI) :

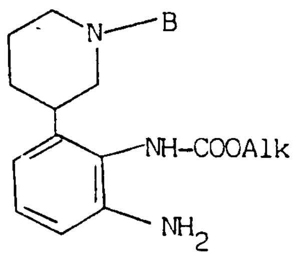

(VI)

dans laquelle B et Alk ont la signification déjà indiquée soit l'on soumet ledit dérivé de formule (V) ou ledit dérivé de formule (VI) à l'action d'un agent de déblocage de l'amine, du cycle pipéridine, pour obtenir respectivement un dérivé de formule (V′) ou (VI′) :

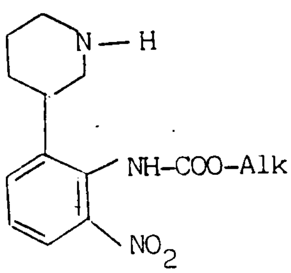

(V')

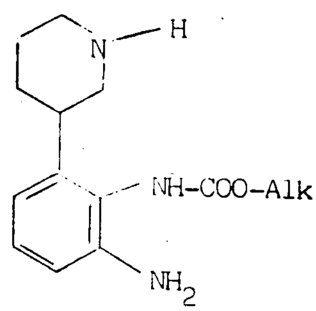

(VI')

dans lesquelles Alk a la signification déjà indiquée, que l'on soumet à l'action d'un agent capable de greffer un radical R′, R′ ayant la signification de R à l'exception de l'atome d'hydrogène, pour obtenir respectivement des dérivés de formules (V″) ou (VI″) :

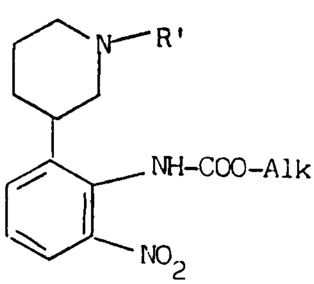

(V")

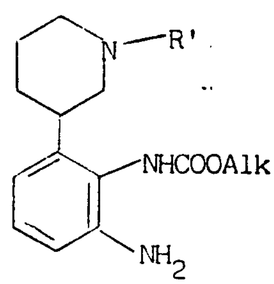

(VI")

dans lesquelles Alk a la signification déjà indiquée, puis réduit le groupement nitro desdits dérivés de formule (V′) et (V″), pour obtenir respectivement les dérivés de formules (VI′) et (VI″) que l'on cyclise par hydrolyse, pour obtenir un dérivé de formule ($I_A$) :

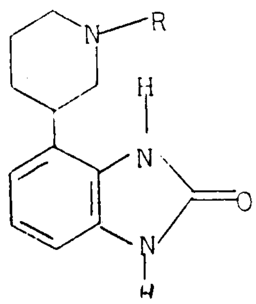

$(I_A)$

dans laquelle R a la signification déjà indiquée, que l'on isole et, si désiré, salifie, soit l'on cyclise le dérivé de formule (VI) comme les dérivés de formules (VI') et (VI''), puis débloque l'amine secondaire du cycle pipéridine, pour obtenir un dérivé de formule $(I_A)$ dans laquelle R représente un atome d'hydrogène, que l'on peut éventuellement substituer, pour obtenir d'autres valeurs de R ;

b) pour préparer les dérivés de formule (I), dans laquelle $R_1$ est différent d'un atome d'hydrogène, ainsi que leurs sels, l'on fait réagir un dérivé de formule (II') :

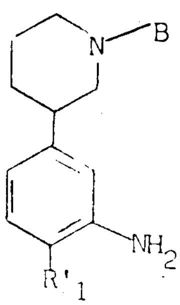

(II')

dans laquelle B a la signification déjà indiquée et $R'_1$ a la signification de $R_1$ déjà indiquée, à l'exception de l'atome d'hydrogène et du radical hydroxyle, avec le phosgène ou un précurseur de phosgène, pour obtenir un dérivé de formule (III') :

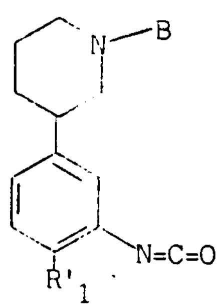

(III')

dans laquelle B et $R'_1$ ont la signification déjà indiquée, que l'on fait réagir avec l'O-benzylhydroxylamine, pour obtenir un dérivé de formule (IV') :

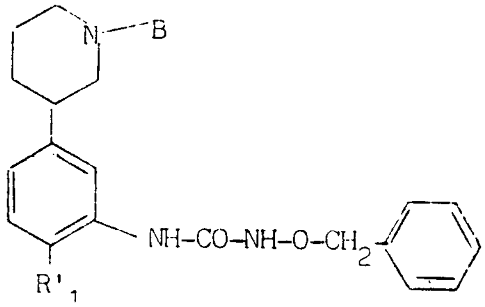

(IV')

dans laquelle B et R'$_1$ ont la signification déjà indiquée, que l'on cyclise, pour obtenir un dérivé de formule (VII) :

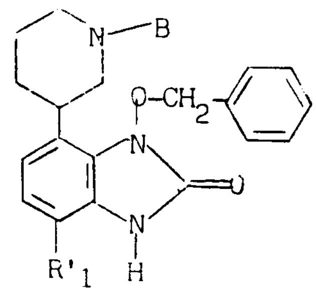

(VII)

dans laquelle B et R'$_1$ ont la signification déjà indiquée, dont <u>soit</u> on débloque les fonctions amines, pour obtenir un dérivé de formule (I'$_A$) :

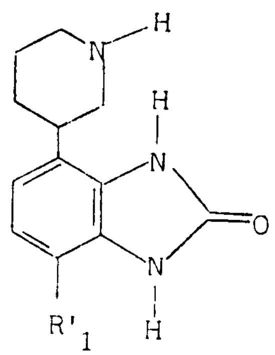

(I'$_A$)

dans laquelle R'$_1$ a la signification déjà indiquée, que soit l'on isole et, si désiré, salifie, <u>soit</u> l'on débloque sélectivement l'amine du cycle pipéridine, pour obtenir un dérivé de formule (VIII) :

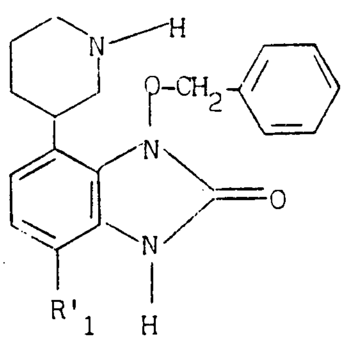

(VIII)

dans laquelle R'$_1$ a la signification déjà indiquée, que l'on soumet à l'action d'un agent capable de greffer un radical R', R' ayant la signification déjà indiquée, pour obtenir un dérivé de formule (IX) :

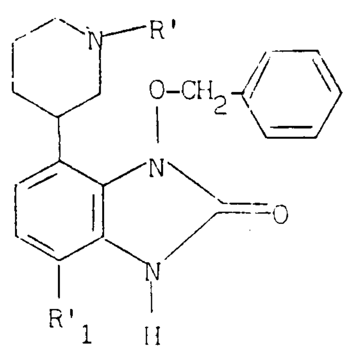

(IX)

dans laquelle R′ et $R'_1$ ont la signification déjà indiquée, puis soumet desdits dérivés de formules (VIII) et (IX) à une hydrogénation catalytique, pour obtenir un dérivé de formule ($I'_B$) :

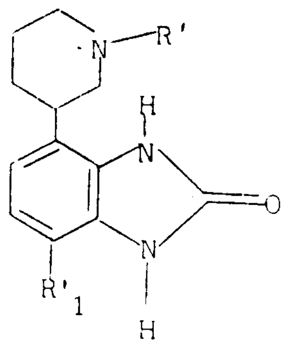

($I'_B$)

dans laquelle R′ et $R'_1$ ont la signification déjà indiquée, que soit l'onisole et, si désiré, salifie, soit l'on soumet lesdits dérivés de formule ($I'_A$) et ($I'_B$) à l'action d'un agent de déblocage de l'hydroxyle, pour obtenir un dérivé de formule ($I'_C$) :

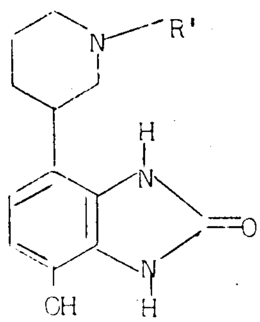

($I'_C$)

dans laquelle R′ a la signification déjà indiquée, que l'on isole et, si désiré, salifie, soit l'on débloque sélectivement pour le dérivé de formule (VII), l'amine du cycle imidazole, puis l'amine de la pipéridine et enfin l'hydroxyle, si désiré.

**2.** Procédé selon la revendication 1, pour la préparation des dérivés de la benzimidazol-2-one , tels que définis par la formule (I) de la revendication 1, dans laquelle $R_1$ représente un atome d'hydrogène ainsi que de leurs sels, caractérisé en ce que l'on fait réagir un dérivé de formule (II) :

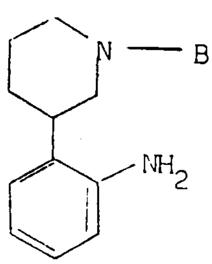

(II)

dans laquelle B représente un agent de blocage réversible d'une amine secondaire avec un halogénoformiate d'alkyle de formule (III) :

Hal-COO-Alk (III)

dans laquelle Hal représente un atome de chlore, de brome ou d'iode et Alk représente un radical alkyle renfermant de 1 à 5 atomes de carbone, pour obtenir un dérivé de formule (IV) :

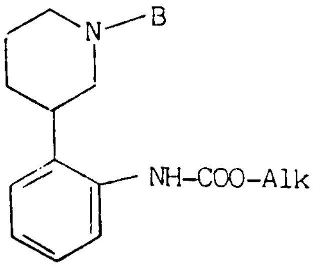

(IV)

dans laquelle B et Alk ont la signification déjà indiquée, que l'on soumet à une nitration, pour obtenir un dérivé de formule (V) :

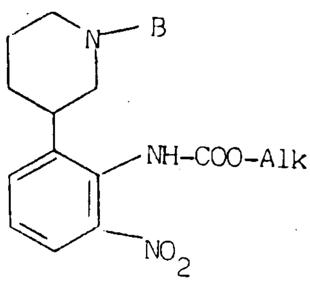

(V)

dans laquelle B et Alk ont la signification déjà indiquée dont soit l'on réduit le groupement nitro, pour obtenir un dérivé de formule (VI) :

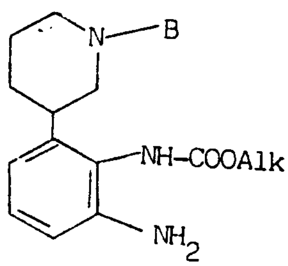

(VI)

dans laquelle B et Alk ont la signification déjà indiquée soit l'on soumet ledit dérivé de formule (V) ou ledit dérivé de formule (VI) à l'action d'un agent de déblocage de l'amine, du cycle pipéridine, pour obtenir respectivement un dérivé de formule (V′) ou (VI′) :

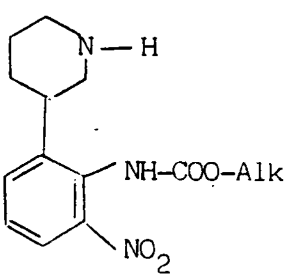

(V')

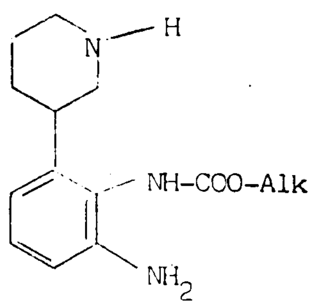

(VI')

dans lesquelles Alk a la signification déjà indiquée, que l'on soumet à l'action d'un agent capable de greffer un radical R′, R′ ayant la signification de R à l'exception de l'atome d'hydrogène, pour obtenir respectivement des dérivés de formules (V″) ou (VI″) :

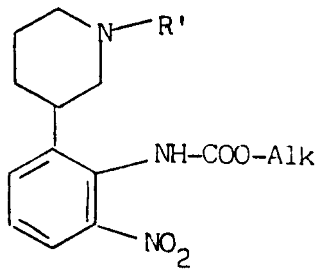

(V")

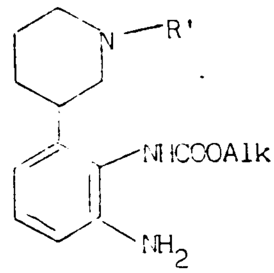

(VI")

dans lesquelles Alk a la signification déjà indiquée, puis réduit le groupement nitro desdits dérivés de formule (V′) et (V″), pour obtenir respectivement les dérivés de formules (VI′) et (VI″) que l'on cyclise par hydrolyse, pour obtenir un dérivé de formule ($I_A$) :

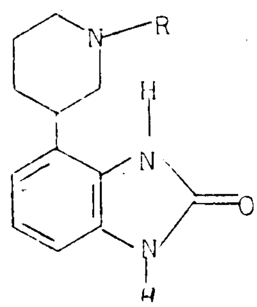

($I_A$)

dans laquelle R a la signification déjà indiquée, que l'on isole et, si désiré, salifie, soit l'on cyclise le dérivé de formule (VI) comme les dérivés de formules (VI′) et (VI″), puis débloque l'amine secondaire du cycle pipéridine, pour obtenir un dérivé de formule ($I_A$) dans laquelle R représente un atome d'hydrogène, que l'on peut éventuellement substituer, pour obtenir d'autres valeurs de R.

**3.** Procédé selon la revendication 2, caractérisé en ce que :
- B est un radical trifluoroacétyle ;
- la nitration du dérivé de formule (IV) est réalisée à l'aide de tétrafluoroborate de nitronium.

**4.** Procédé selon la revendication 1, pour la préparation des dérivés tels que définis par la formule (I) de la revendication 1, dans laquelle $R_1$ est différent d'un atome d'hydrogène ainsi que de leurs sels, caractérisé en ce que l'on fait réagir un dérivé de formule (II′) :

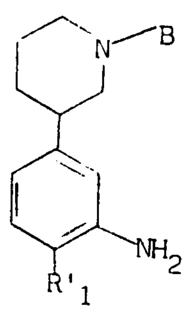

(II')

dans laquelle B a la signification déjà indiquée et $R'_1$ a la signification de $R_1$ déjà indiquée, à l'exception de l'atome d'hydrogène et du radical hydroxyle, avec le phosgène ou un précurseur de phosgène, pour obtenir un dérivé de formule (III′) :

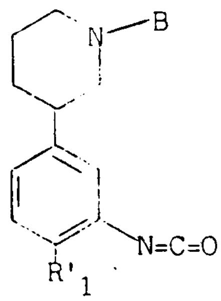

(III')

dans laquelle B et $R'_1$ ont la signification déjà indiquée, que l'on fait réagir avec l'O-benzylhydroxylamine, pour obtenir un dérivé de formule (IV') :

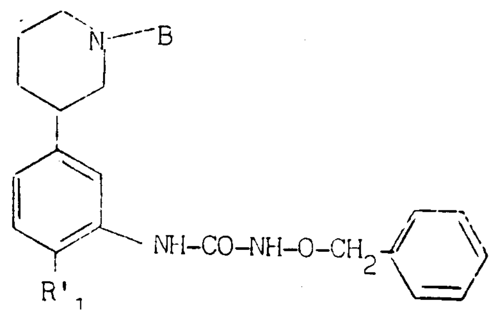

(IV')

dans laquelle B et $R'_1$ ont la signification déjà indiquée, que l'on cyclise, pour obtenir un dérivé de formule (VII) :

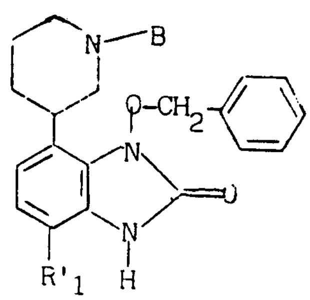

(VII)

dans laquelle B et $R'_1$ ont la signification déjà indiquée, dont soit on débloque les fonctions amines, pour obtenir un dérivé de formule ($I'_A$) :

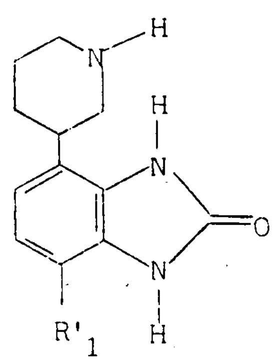

($I'_A$)

dans laquelle $R'_1$ a la signification déjà indiquée, que soit l'on isole et, si désiré, salifie, soit l'on débloque sélectivement l'amine du cycle pipéridine, pour obtenir un dérivé de formule (VIII) :

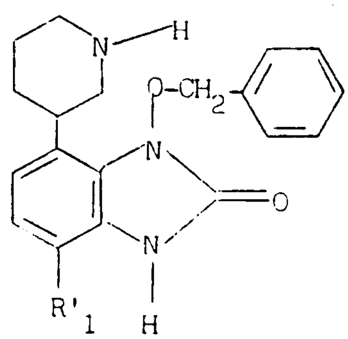

(VIII)

dans laquelle R'$_1$ a la signification déjà indiquée, que l'on soumet à l'action d'un agent capable de greffer un radical R', R' ayant la signification déjà indiquée, pour obtenir un dérivé de formule (IX) :

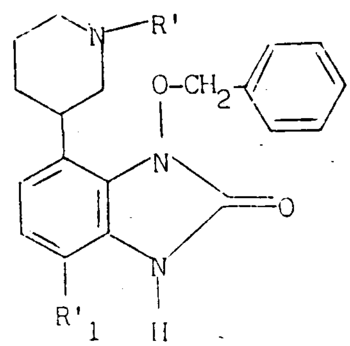

(IX)

dans laquelle R' et R'$_1$ ont la signification déjà indiquée, puis soumet desdits dérivés de formules (VIII) et (IX) à une hydrogénation catalytique, pour obtenir un dérivé de formule (I'$_B$) :

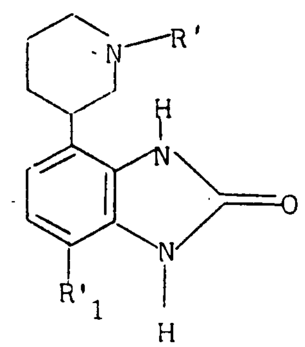

(I'$_B$)

dans laquelle R' et R'$_1$ ont la signification déjà indiquée, que soit l'onisole et, si désiré, salifie, soit l'on soumet lesdits dérivés de formule (I'$_A$) et (I'$_B$) à l'action d'un agent de déblocage de l'hydroxyle, pour obtenir un dérivé de formule (I'$_C$) :

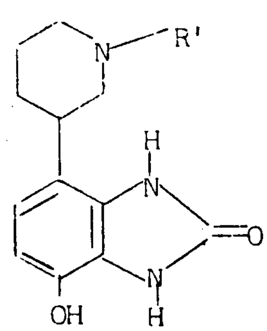

(I'$_C$)

dans laquelle R′ a la signification déjà indiquée, que l'on isole et, si désiré, salifie, soit l'on débloque sélectivement pour le dérivé de formule (VII), l'amine du cycle imidazole, puis l'amine de la pipéridine et enfin l'hydroxyle, si désiré.

**5.** Procédé selon la revendication 1, caractérisé en ce que l'on prépare des dérivés de formule (I), dans laquelle $R_1$ représente un atome d'hydrogène, ainsi que leurs sels.

**6.** Procédé selon la revendication 1 ou 5, caractérisé en ce que l'on prépare des dérivés de formule (I), dans laquelle R représente un atome d'hydrogène ou un radical alkyle renfermant de 1 à 5 atomes de carbone, ainsi que leurs sels.

**7.** procédé selon la revendication 1, caractérisé en ce que l'on prépare :
- la 1,3-dihdyro 4-(1-propyl-3-pipéridinyl) 2H-benzimidazol-2-one,
- la 1,3-dihydro 4-hydroxy 7-(1-propyl 3-pipéridinyl) 2H-benzimidazol-2-one,

ainsi que leurs sels d'addition avec les acides minéraux ou organiques.

**Revendications pour l'Etat contractant suivant : GR**

**1.** Procédé de préparation des dérivés de la benzimidazol-2-one ainsi que de leurs sels d'addition avec les acides minéraux ou organiques, répondant à la formule générale (I) :

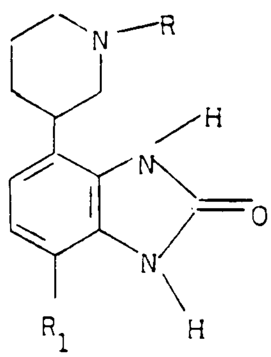

(I)

dans laquelle R représente un atome d'hydrogène, un radical alkyle renfermant de 1 à 5 atomes de carbone, un radical cycloalkylalkyle renfermant de 4 à 7 atomes de carbone, un radical aralkyle renfermant de 7 à 12 atomes de carbone, éventuellement substitué par un ou plusieurs radicaux choisis dans le groupe constitué par les radicaux alkyles renfermant de 1 à 5 atomes de carbone, les radicaux alkoxy renfermant de 1 à 5 atomes de carbone et les atomes d'halogène ou R représente un radical alkényle ou un radical alkynyle renfermant de 3 à 5 atomes de carbone, étant entendu que la liaison multiple ne peut pas se trouver entre les carbones en alpha et béta de l'atome d'azote et $R_1$ représente un atome d'hydrogène, un radical hydroxy, un radical alkoxy renfermant de 1 à 5 atomes de carbone, un radical phénylalkyloxy renfermant de 7 à 9 atomes de carbone ou un radical phénoxy, caractérisé en ce que :

a) pour préparer les dérivés de formule (I), dans laquelle $R_1$ représente un atome d'hydrogène, ainsi que leurs sels, l'on fait réagir un dérivé de formule (II) :

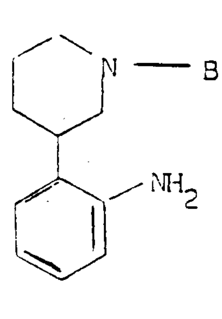

(II)

dans laquelle B représente un agent de blocage réversible d'une amine secondaire avec un

halogénoformiate d'alkyle de formule (III) :

Hal-COO-Alk (III)

dans laquelle Hal représente un atome de chlore, de brome ou d'iode et Alk représente un radical alkyle renfermant de 1 à 5 atomes de carbone, pour obtenir un dérivé de formule (IV) :

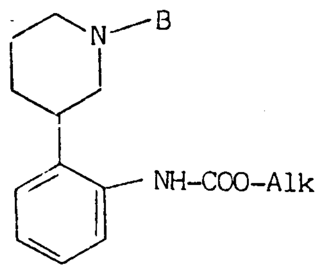

(IV)

dans laquelle B et Alk ont la signification déjà indiquée, que l'on soumet à une nitration, pour obtenir un dérivé de formule (V) :

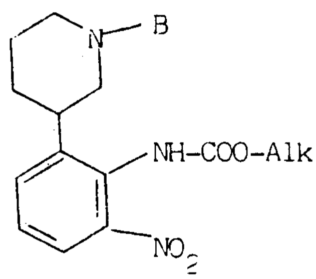

(V)

dans laquelle B et Alk ont la signification déjà indiquée dont soit l'on réduit le groupement nitro, pour obtenir un dérivé de formule (VI) :

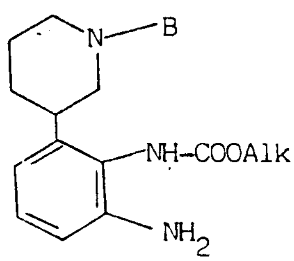

(VI)

dans laquelle B et Alk ont la signification déjà indiquée soit l'on soumet ledit dérivé de formule (V) ou ledit dérivé de formule (VI) à l'action d'un agent de déblocage de l'amine, du cycle pipéridine, pour obtenir respectivement un dérivé de formule (V') ou (VI') :

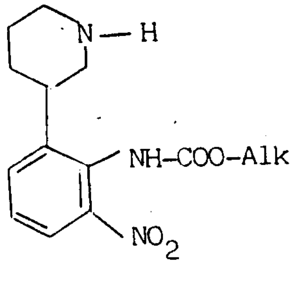

(V')

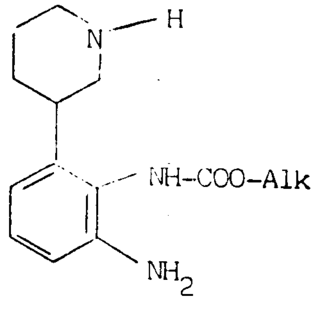

(VI')

dans lesquelles Alk a la signification déjà indiquée, que l'on soumet à l'action d'un agent capable de greffer un radical R′, R′ ayant la signification de R à l'exception de l'atome d'hydrogène, pour obtenir respectivement des dérivés de formules (V″) ou (VI″) :

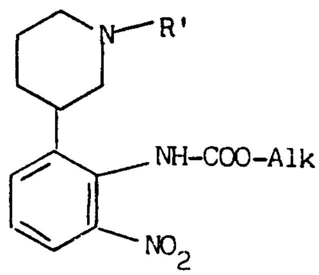

(V")

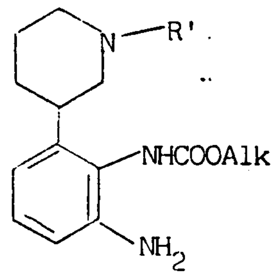

(VI")

dans lesquelles Alk a la signification déjà indiquée, puis réduit le groupement nitro desdits dérivés de formule (V′) et (V″), pour obtenir respectivement les dérivés de formules (VI′) et (VI″) que l'on cyclise par hydrolyse, pour obtenir un dérivé de formule ($I_A$) :

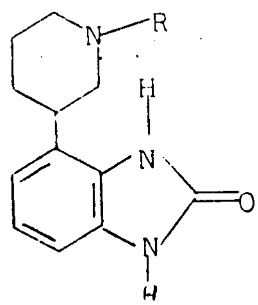

($I_A$)

dans laquelle R a la signification déjà indiquée, que l'on isole et, si désiré, salifie, soit l'on cyclise le dérivé de formule (VI) comme les dérivés de formules (VI′) et (VI″), puis débloque l'amine secondaire du cycle pipéridine, pour obtenir un dérivé de formule ($I_A$) dans laquelle R représente un atome d'hydrogène, que l'on peut éventuellement substituer, pour obtenir d'autres valeurs de R ;

b) pour préparer les dérivés de formule (I), dans laquelle $R_1$ est différent d'un atome d'hydrogène, ainsi que leurs sels, l'on fait réagir un dérivé de formule (II′) :

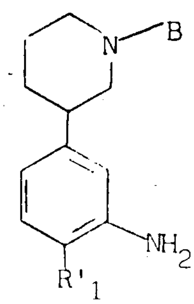

(II')

dans laquelle B a la signification déjà indiquée et $R'_1$ a la signification de $R_1$ déjà indiquée, à l'exception de l'atome d'hydrogène et du radical hydroxyle, avec le phosgène ou un précurseur de phosgène, pour obtenir un dérivé de formule (III′) :

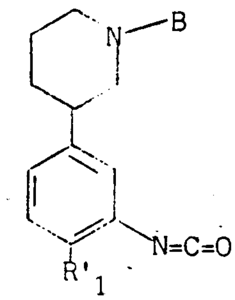

(III')

dans laquelle B et $R'_1$ ont la signification déjà indiquée, que l'on fait réagir avec l'O-benzylhydroxylamine, pour obtenir un dérivé de formule (IV') :

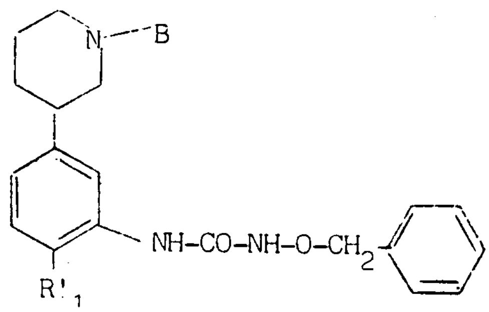

(IV')

dans laquelle B et $R'_1$ ont la signification déjà indiquée, que l'on cyclise, pour obtenir un dérivé de formule (VII) :

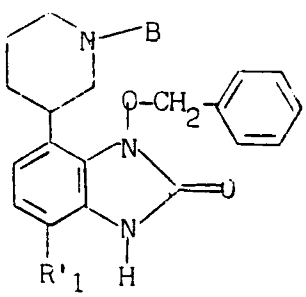

(VII)

dans laquelle B et $R'_1$ ont la signification déjà indiquée, dont soit on débloque les fonctions amines, pour obtenir un dérivé de formule ($I'_A$) :

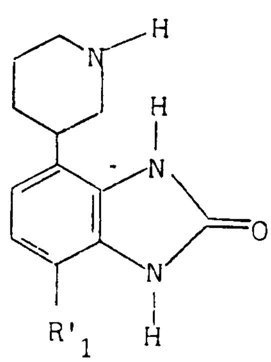

($I'_A$)

dans laquelle $R'_1$ a la signification déjà indiquée, que soit l'on isole et, si désiré, salifie, soit l'on débloque sélectivement l'amine du cycle pipéridine, pour obtenir un dérivé de formule (VIII) :

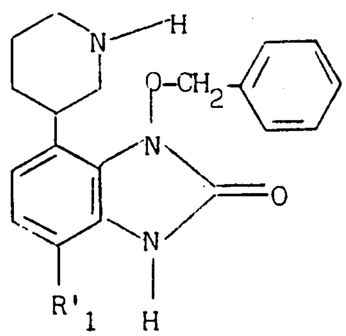

(VIII)

dans laquelle $R'_1$ a la signification déjà indiquée, que l'on soumet à l'action d'un agent capable de greffer un radical R', R' ayant la signification déjà indiquée, pour obtenir un dérivé de formule (IX) :

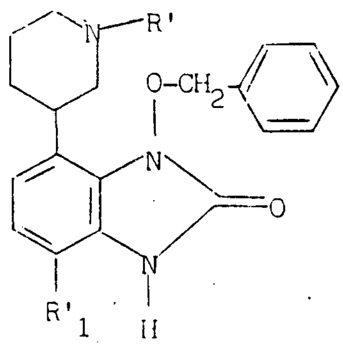

(IX)

dans laquelle R' et $R'_1$ ont la signification déjà indiquée, puis soumet desdits dérivés de formules (VIII) et (IX) à une hydrogénation catalytique, pour obtenir un dérivé de formule ($I'_B$) :

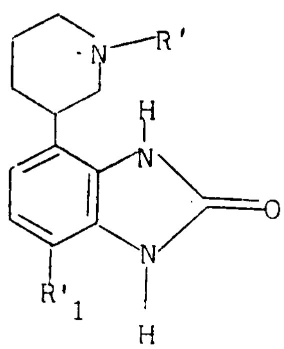

($I'_B$)

dans laquelle R' et $R'_1$ ont la signification déjà indiquée, que soit l'onisole et, si désiré, salifie, soit l'on soumet lesdits dérivés de formule ($I'_A$) et ($I'_B$) à l'action d'un agent de déblocage de l'hydroxyle, pour obtenir un dérivé de formule ($I'_C$) :

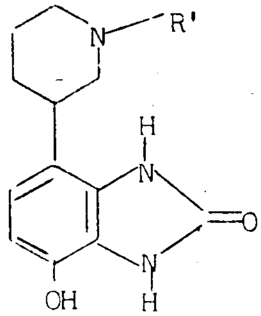

($I'_C$)

dans laquelle R′ a la signification déjà indiquée, que l'on isole et, si désiré, salifie, soit l'on débloque sélectivement pour le dérivé de formule (VII), l'amine du cycle imidazole, puis l'amine de la pipéridine et enfin l'hydroxyle, si désiré.

**2.** Procédé selon la revendication 1, pour la préparation des dérivés de la benzimidazol-2-one , tels que définis par la formule (I) de la revendication 1, dans laquelle $R_1$ représente un atome d'hydrogène ainsi que de leurs sels, caractérisé en ce que l'on fait réagir un dérivé de formule (II) :

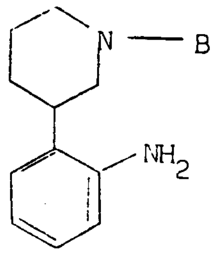

(II)

dans laquelle B représente un agent de blocage réversible d'une amine secondaire avec un halogénoformiate d'alkyle de formule (III) :

Hal-COO-Alk (III)

dans laquelle Hal représente un atome de chlore, de brome ou d'iode et Alk représente un radical alkyle renfermant de 1 à 5 atomes de carbone, pour obtenir un dérivé de formule (IV) :

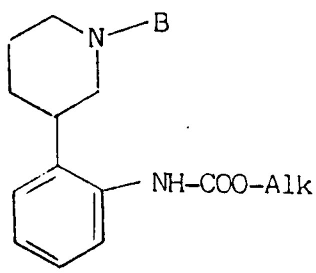

(IV)

dans laquelle B et Alk ont la signification déjà indiquée, que l'on soumet à une nitration, pour obtenir un dérivé de formule (V) :

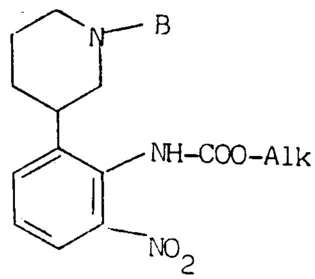

(V)

dans laquelle B et Alk ont la signification déjà indiquée dont soit l'on réduit le groupement nitro, pour obtenir un dérivé de formule (VI) :

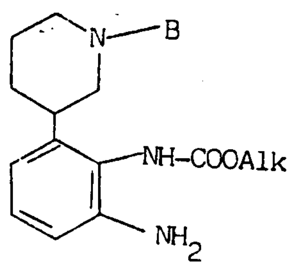

(VI)

dans laquelle B et Alk ont la signification déjà indiquée soit l'on soumet ledit dérivé de formule (V) ou ledit dérivé de formule (VI) à l'action d'un agent de déblocage de l'amine, du cycle pipéridine, pour obtenir respectivement un dérivé de formule (V′) ou (VI′) :

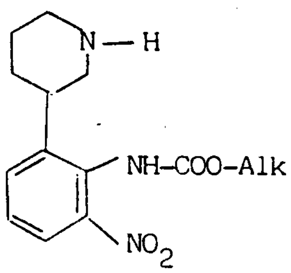

(V')

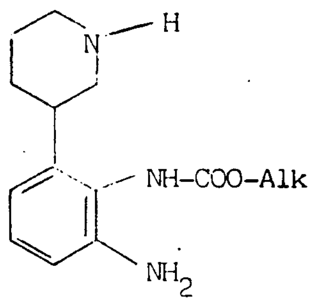

(VI')

dans lesquelles Alk a la signification déjà indiquée, que l'on soumet à l'action d'un agent capable de greffer un radical R′, R′ ayant la signification de R à l'exception de l'atome d'hydrogène, pour obtenir respectivement des dérivés de formules (V″) ou (VI″) :

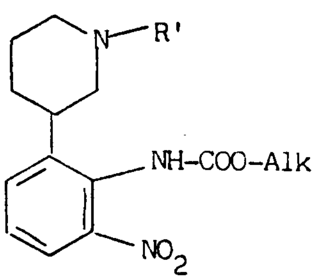

(V")

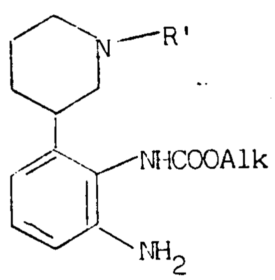

(VI")

dans lesquelles Alk a la signification déjà indiquée, puis réduit le groupement nitro desdits dérivés de formule (V′) et (V″), pour obtenir respectivement les dérivés de formules (VI′) et (VI″) que l'on cyclise par hydrolyse, pour obtenir un dérivé de formule ($I_A$) :

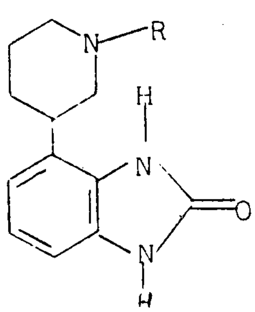

($I_A$)

dans laquelle R a la signification déjà indiquée, que l'on isole et, si désiré, salifie, soit l'on cyclise le dérivé de formule (VI) comme les dérivés de formules (VI′) et (VI″), puis débloque l'amine secondaire du cycle pipéridine, pour obtenir un dérivé de formule ($I_A$) dans laquelle R représente un atome

d'hydrogène, que l'on peut éventuellement substituer, pour obtenir d'autres valeurs de R.

**3.** Procédé selon la revendication 2, caractérisé en ce que :
- B est un radical trifluoroacétyle ;
- la nitration du dérivé de formule (IV) est réalisée à l'aide de tétrafluoroborate de nitronium.

**4.** Procédé selon la revendication 1, pour la préparation des dérivés tels que définis par la formule (I) de la revendication 1, dans laquelle $R_1$ est différent d'un atome d'hydrogène ainsi que de leurs sels, caractérisé en ce que l'on fait réagir un dérivé de formule (II') :

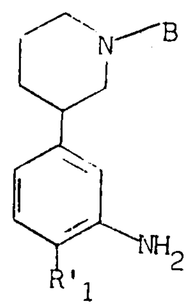

(II')

dans laquelle B a la signification déjà indiquée et $R'_1$ a la signification de $R_1$ déjà indiquée, à l'exception de l'atome d'hydrogène et du radical hydroxyle, avec le phosgène ou un précurseur de phosgène, pour obtenir un dérivé de formule (III') :

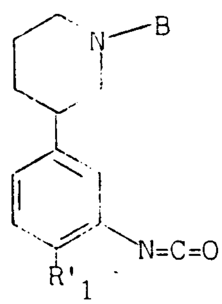

(III')

dans laquelle B et $R'_1$ ont la signification déjà indiquée, que l'on fait réagir avec l'O-benzylhydroxylamine, pour obtenir un dérivé de formule (IV') :

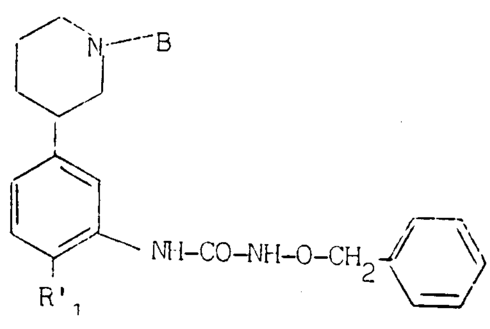

(IV')

dans laquelle B et $R'_1$ ont la signification déjà indiquée, que l'on cyclise, pour obtenir un dérivé de formule (VII) :

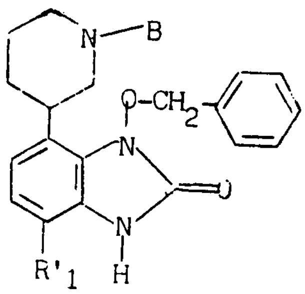

(VII)

dans laquelle B et $R'_1$ ont la signification déjà indiquée, dont soit on débloque les fonctions amines, pour obtenir un dérivé de formule ($I'_A$) :

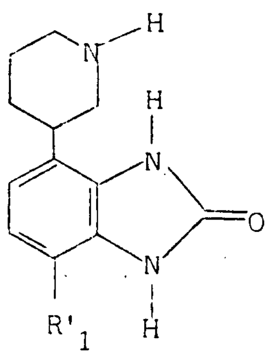

($I'_A$)

dans laquelle $R'_1$ a la signification déjà indiquée, que soit l'on isole et, si désiré, salifie, soit l'on débloque sélectivement l'amine du cycle pipéridine, pour obtenir un dérivé de formule (VIII) :

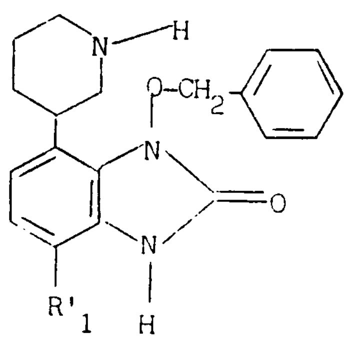

(VIII)

dans laquelle $R'_1$ a la signification déjà indiquée, que l'on soumet à l'action d'un agent capable de greffer un radical R', R' ayant la signification déjà indiquée, pour obtenir un dérivé de formule (IX) :

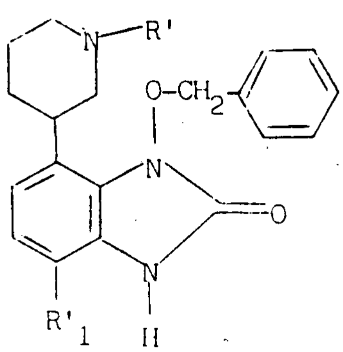

(IX)

dans laquelle R' et $R'_1$ ont la signification déjà indiquée, puis soumet desdits dérivés de formules (VIII)

et (IX) à une hydrogénation catalytique, pour obtenir un dérivé de formule ($I'_B$) :

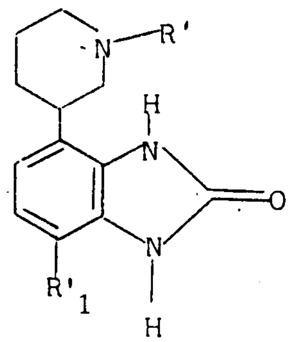

($I'_B$)

dans laquelle R′ et $R'_1$ ont la signification déjà indiquée, que soit l'onisole et, si désiré, salifie, soit l'on soumet lesdits dérivés de formule ($I'_A$) et ($I'_B$) à l'action d'un agent de déblocage de l'hydroxyle, pour obtenir un dérivé de formule ($I'_C$) :

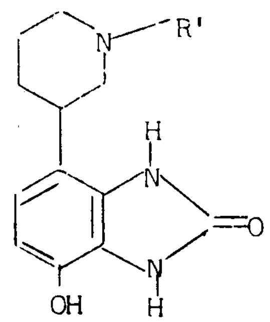

($I'_C$)

dans laquelle R′ a la signification déjà indiquée, que l'on isole et, si désiré, salifie, soit l'on débloque sélectivement pour le dérivé de formule (VII), l'amine du cycle imidazole, puis l'amine de la pipéridine et enfin l'hydroxyle, si désiré.

**5.** Procédé selon la revendication 1, caractérisé en ce que l'on prépare des dérivés de formule (I), dans laquelle $R_1$ représente un atome d'hydrogène, ainsi que leurs sels.

**6.** Procédé selon la revendication 1 ou 5, caractérisé en ce que l'on prépare des dérivés de formule (I), dans laquelle R représente un atome d'hydrogène ou un radical alkyle renfermant de 1 à 5 atomes de carbone, ainsi que leurs sels.

**7.** procédé selon la revendication 1, caractérisé en ce que l'on prépare :
- la 1,3-dihydro 4-(1-propyl-3-pipéridinyl) 2H-benzimidazol-2-one,
- la 1,3-dihdyro 4-hydroxy 7-(1-propyl 3-pipéridinyl) 2H-benzimidazol-2-one,

ainsi que leurs sels d'addition avec les acides minéraux ou organiques.

**8.** Procédé de préparation de compositions pharmaceutiques, caractérisé en ce que l'on met, à titre de principe actif, l'un au moins des dérivés de formule (I), telle que définie à la revendication 1, ou l'un au moins de leurs sels d'addition avec les acides minéraux ou organiques pharmaceutiquement acceptables sous une forme destinée à cet usage.

**9.** Procédé selon la revendication 8, caractérisé en ce que l'on utilise, à titre de principe actif, l'un au moins des dérivés de formule (I), tels que définis à la revendication 7, ou l'un au moins de leurs sels d'addition avec les acides minéraux ou organiques pharmaceutiquement acceptables.

**10.** Les dérivés de formules (VI") et (VIII) :

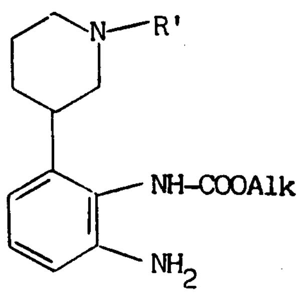

(VI")

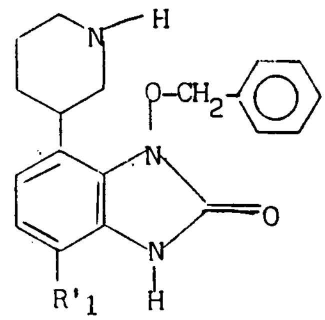

(VIII)

dans lesquelles R', $R'_1$ et Alk ont la signification déjà indiquée à la revendication 1.

## Claims

### Claims for the following Contracting States : AT, BE, CH, DE, FR, GB, IT, LI, LU, NL, SE

1. Benzimidazol-2-one derivatives as well as their addition salts with mineral or organic acids, characterized in that they correspond to the general formula (I):

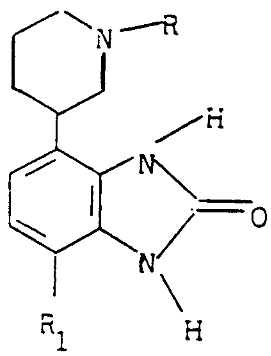

(I)

in which R represents a hydrogen atom, an alkyl radical containing 1 to 5 carbon atoms, a cycloalkylalkyl radical containing 4 to 7 carbon atoms, an aralkyl radical containing 7 to 12 carbon atoms, optionally substituted by one or more radicals chosen from the group constituted by alkyl radicals containing 1 to 5 carbon atoms, alkoxy radicals containing 1 to 5 carbon atoms and halogen atoms or R represents an alkenyl radical or an alkynyl radical containing 3 to 5 carbon atoms, it being understood that the multiple bond cannot be found between the carbons in alpha and beta position of the nitrogen atom, and $R_1$ represents a hydrogen atom, a hydroxy radical, an alkoxy radical containing 1 to 5 carbon atoms, a phenylalkyloxy radical containing 7 to 9 carbon atoms or a phenoxy radical.

2. Benzimidazol-2-one derivatives as defined by formula (I) of claim 1, as well as their addition salts with mineral or organic acids, characterized in that $R_1$ represents a hydrogen atom.

3. Benzimidazol-2-one derivatives according to claim 2, as well as their addition salts with mineral or organic acids, characterized in that R represents a hydrogen atom or an alkyl radical containing 1 to 5 carbon atoms.

4. The derivatives of formula (I) according to claim 1, of which the names follow:
   - 1,3-dihydro-4-(1-propyl-3-piperidinyl)-2H-benzimidazol-2-one,
   - 1,3-dihydro-4-hydroxy-7-(1-propyl-3-piperidinyl)-2H-benzimidazol-2-one,

   as well as their addition salts with mineral or organic acids.

5. Preparation process for benzimidazol-2-one derivatives, as defined by formula (I) of claim 1, in which $R_1$ represents a hydrogen atom, as well as their salts, characterized in that a derivative of formula (II):

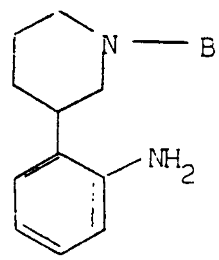

(II)

in which B represents a reversible blocking agent of a secondary amine, is reacted with an alkyl halogenoformate of formula (III):

Hal-COO-Alk (III)

in which Hal represents a chlorine, bromine or iodine atom and Alk represents an alkyl radical containing 1 to 5 carbon atoms, in order to obtain a derivative of formula (IV):

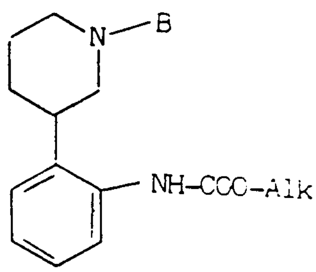

(IV)

in which B and Alk have the meaning already indicated, which is subjected to a nitration, in order to obtain a derivative of formula (V):

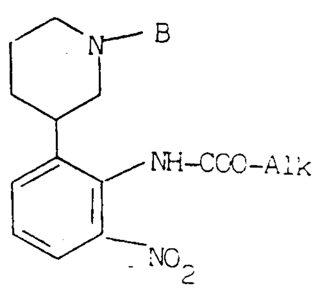

(V)

in which B and Alk have the meaning already indicated, of which either the nitro group is reduced, in order to obtain a derivative of formula (VI):

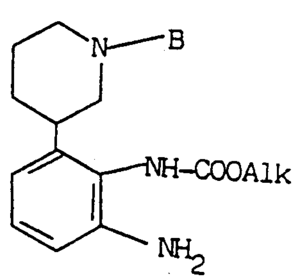

(VI)

in which B and Alk have the meaning already indicated, or the said derivative of formula (V) or the said derivative of formula (VI) are subjected to the action of an unblocking agent of the amine of the piperidine ring, in order to obtain respectively a derivative of formula (V') or (VI'):

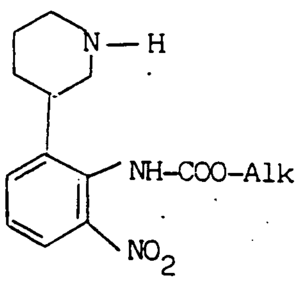 (V')   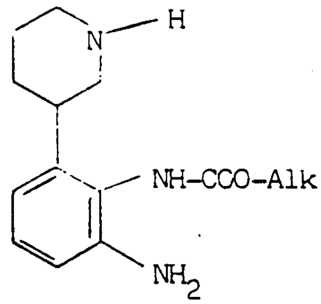 (VI')

in which Alk has the meaning already indicated, which is subjected to the action of an agent capable of grafting an R' radical, R' having the meaning of R with the exception of the hydrogen atom, in order to obtain the derivatives of formula (V") or (VI") respectively:

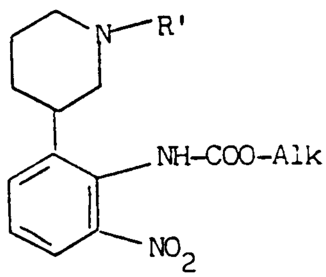 (V")   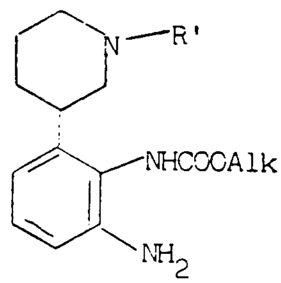 (VI")

in which Alk has the meaning already indicated, then the nitro group of the said derivatives of formulae (V') and (V") is reduced, in order to obtain the derivatives of formulae (VI') and (VI") respectively, which are cyclized by hydrolysis, in order to obtain a derivative of formula ($I_A$):

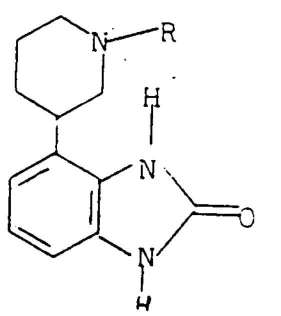

($I_A$)

in which R has the meaning already indicated, which is isolated and, if desired, salified, or the derivative of formula (VI) is cyclized in the same way as the derivatives of formulae (VI') and (VI"), then the secondary amine of the piperidine ring is unblocked, in order to obtain a derivative of formula ($I_A$) in which R represents a hydrogen atom, which can be optionally substituted, in order to obtain other values of R.

**6.** Process according to claim 4, characterized in that:

- B is a trifluoroacetyl radical;
- the nitration of the derivative of formula (IV) is carried out using nitronium tetrafluoroborate.

**7.** Preparation process for derivatives as defined by formula (I) of claim 1, in which $R_1$ has the meaning indicated in claim 1 with the exception of a hydrogen atom, as well as their salts, characterized in that a derivative of formula (II'):

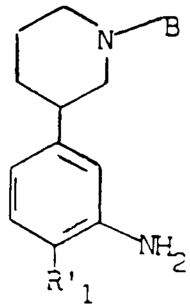

(II')

in which B has the meaning indicated in claim 5 and $R'_1$ has the meaning of $R_1$ indicated in claim 1, with the exception of the hydrogen atom and the hydroxyl radical, is reacted with phosgene or a phosgene precursor, in order to obtain a derivative of formula (III'):

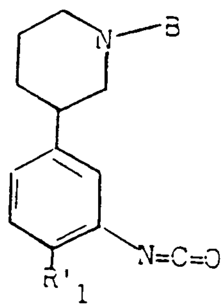

(III')

in which B and $R'_1$ have the meaning already indicated, which is reacted with O-benzylhydroxylamine, in order to obtain a derivative of formula (IV'):

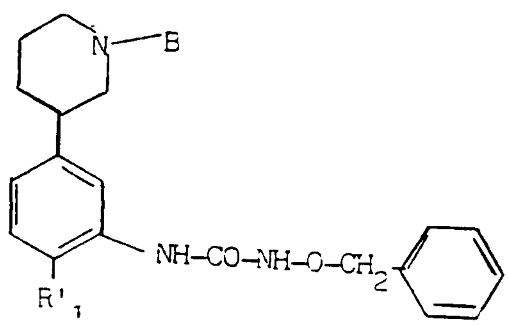

(IV')

in which B and $R'_1$ have the meaning already indicated, which is cyclized, in order to obtain a derivative of formula (VII):

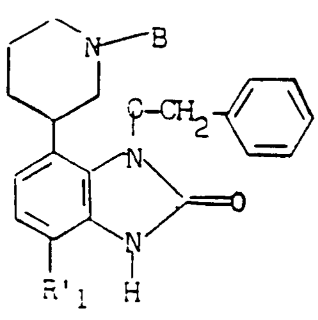

(VII)

in which B and $R'_1$ have the meaning already indicated, of which either the amine functions are

unblocked, in order to obtain a derivative of formula ($I'_A$):

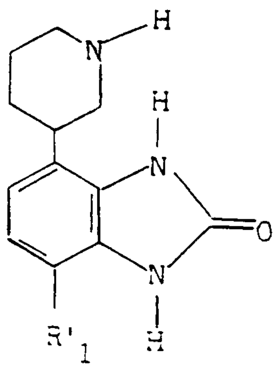

($I'_A$)

in which $R'_1$ has the meaning already indicated, which either is isolated and, if desired, salified, or the amine of the piperidine ring is selectively unblocked, in order to obtain a derivative of formula (VIII):

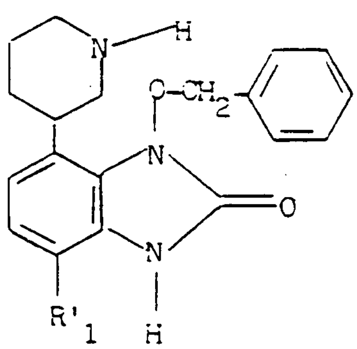

(VIII)

in which $R'_1$ has the meaning already indicated, which is subjected to the action of an agent capable of grafting an R' radical, R' having the meaning indicated in claim 5, in order to obtain a derivative of formula (IX):

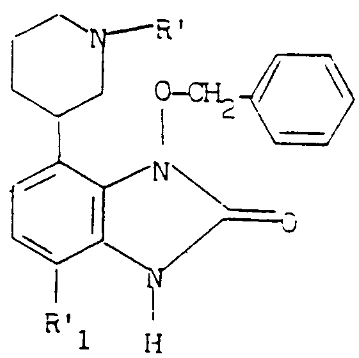

(IX)

in which R' and $R'_1$ have the meaning already indicated, then the said derivatives of formulae (VIII) and (IX) are subjected to a catalytic hydrogenation, in order to obtain a derivative of formula ($I'_B$):

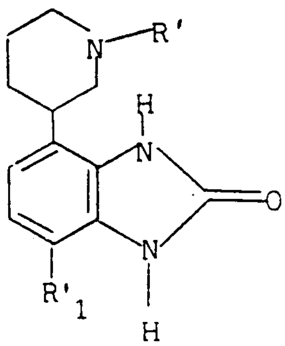

(I'B)

in which R' and $R'_1$ have the meaning already indicated, which either is isolated and, if desired, salified, or the said derivatives of formula ($I'_A$) and ($I'_B$) are subjected to the action of an unblocking agent of the hydroxyl, in order to obtain a derivative of formula ($I'_C$):

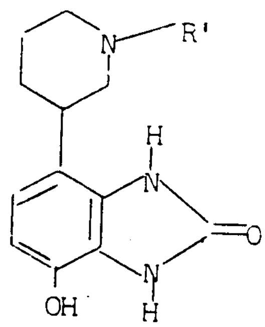

(I'C)

in which R' has the meaning already indicated, which is isolated and, if desired, salified, or if desired, for the derivative of formula (VII), the amine of the imidazole ring, then the amine of the piperidine and finally the hydroxyl are selectively unblocked.

**8.** Medicaments, characterized in that they are constituted by new benzimidazol-2-one derivatives, as defined by formula (I) of claim 1, as well as by their addition salts with pharmaceutically acceptable acids.

**9.** Medicaments, characterized in that they are constituted by new benzimidazol-2-one derivatives, as defined in one of claims 2 or 3, as well as by their addition salts with pharmaceutically acceptable acids.

**10.** Medicaments, characterized in that they are constituted by benzimidazol-2-one derivatives, as defined in claim 4, as well as by their addition salts with pharmaceutically acceptable acids.

**11.** Pharmaceutical compositions, characterized in that they contain as active ingredient, at least one of the medicaments as defined in one of claims 8, 9 or 10.

**12.** The derivatives of formulae (VI") and (VIII):

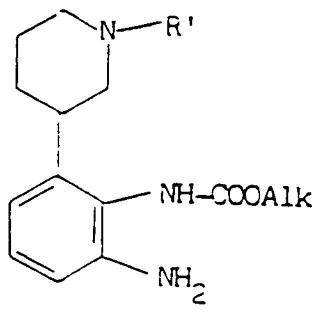

(VI")

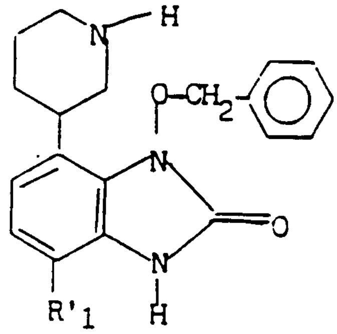

(VIII)

in which R' and Alk have the meaning already indicated in claim 5 and $R'_1$ has the meaning indicated in claim 7.

**Claims for the following Contracting State : ES**

1. Preparation process for benzimidazol-2-one derivatives as well as their addition salts with mineral or organic acids, corresponding to the general formula (I):

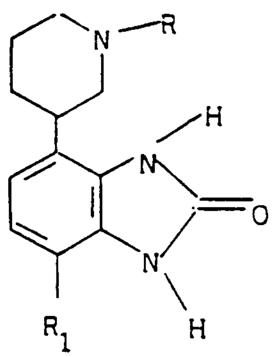

(I)

in which R represents a hydrogen atom, an alkyl radical containing 1 to 5 carbon atoms, a cycloalkylalkyl radical containing 4 to 7 carbon atoms, an aralkyl radical containing 7 to 12 carbon atoms, optionally substituted by one or more radicals chosen from the group constituted by alkyl radicals containing 1 to 5 carbon atoms, alkoxy radicals containing 1 to 5 carbon atoms and halogen atoms or R represents an alkenyl radical or an alkynyl radical containing 3 to 5 carbon atoms, it being understood that the multiple bond cannot be found between the carbons in alpha and beta position of the nitrogen atom, and $R_1$ represents a hydrogen atom, a hydroxy radical, an alkoxy radical containing 1 to 5 carbon atoms, a phenylalkyloxy radical containing 7 to 9 carbon atoms or a phenoxy radical, characterized in that:

a) to prepare the derivatives of formula (I), in which $R_1$ represents a hydrogen atom, as well as their salts, a derivative of formula (II):

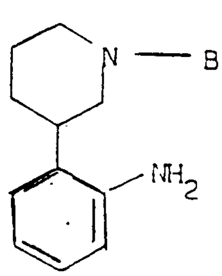

(II)

in which B represents a reversible blocking agent of a secondary amine function, is reacted with an alkyl halogenoformate of formula (III):

Hal-COO-Alk (III)

in which Hal represents a chlorine, bromine or iodine atom and Alk represents an alkyl radical containing 1 to 5 carbon atoms, in order to obtain a derivative of formula (IV):

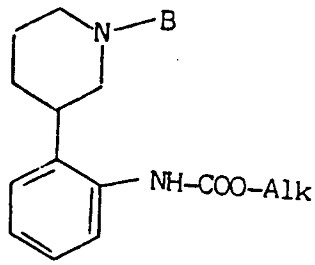

(IV)

in which B and Alk have the meaning already indicated, which is subjected to a nitration, in order to obtain a derivative of formula (V):

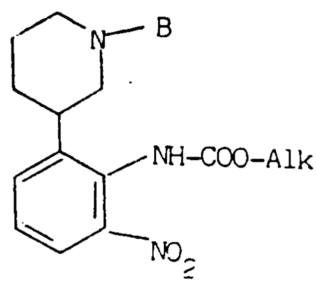

(V)

in which B and Alk have the meaning already indicated, of which either the nitro group is reduced, in order to obtain a derivative of formula (VI):

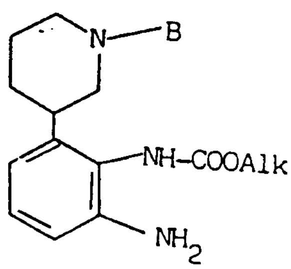

(VI)

in which B and Alk have the meaning already indicated, or the said derivative of formula (V) or the said derivative of formula (VI) are subjected to the action of an unblocking agent of the amine of the piperidine ring, in order to obtain respectively a derivative of formula (V') or (VI'):

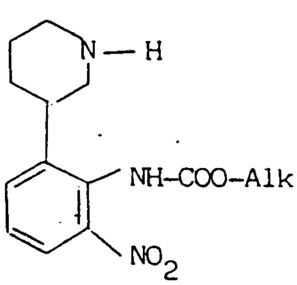

(V')

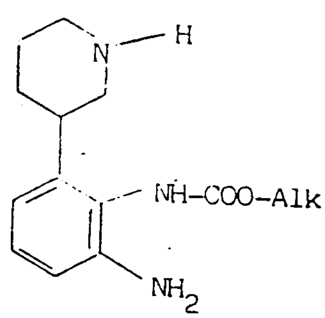

(VI')

in which Alk has the meaning already indicated, which is subjected to the action of an agent capable of grafting an R' radical, R' having the meaning of R with the exception of the hydrogen atom, in order to obtain the derivatives of formula (V") or (VI") respectively:

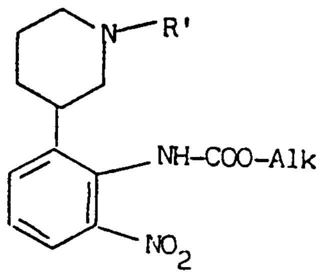

(V")

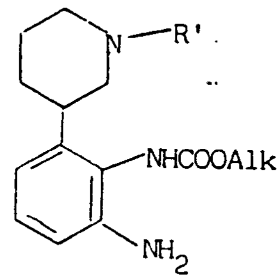

(VI")

in which Alk has the meaning already indicated, then the nitro group of the said derivatives of formulae (V') and (V") is reduced, in order to obtain the derivatives of formulae (VI') and (VI") respectively, which are cyclized by hydrolysis, in order to obtain a derivative of formula ($I_A$):

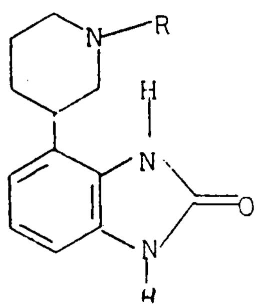

($I_A$)

in which R has the meaning already indicated, which is isolated and, if desired, salified, or the derivative of formula (VI) is cyclized in the same way as the derivatives of formulae (VI') and (VI"), then the secondary amine of the piperidine ring is unblocked, in order to obtain a derivative of formula ($I_A$) in which R represents a hydrogen atom, which can be optionally substituted, in order to obtain other values of R;

b) to prepare the derivatives of formula (I), in which $R_1$ is different from a hydrogen atom, as well as their salts, a derivative of formula (II'):

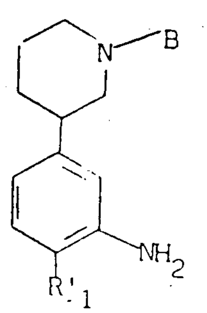

(II')

in which B has the meaning already indicated and $R'_1$ has the meaning of $R_1$ already indicated, with the exception of the hydrogen atom and the hydroxyl radical, is reacted with phosgene or a phosgene precursor, in order to obtain a derivative of formula (III'):

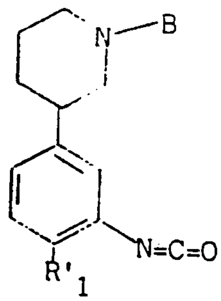

(III')

in which B and $R'_1$ have the meaning already indicated, which is reacted with O-benzylhydroxylamine, in order to obtain a derivative of formula (IV'):

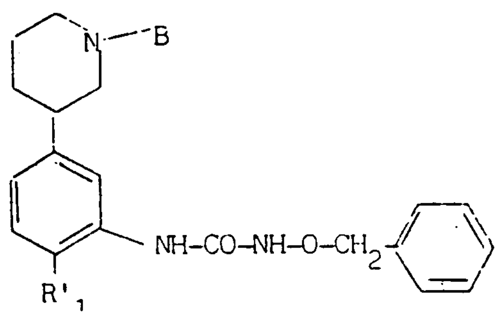

(IV')

in which B and $R'_1$ have the meaning already indicated, which is cyclized, in order to obtain a derivative of formula (VII):

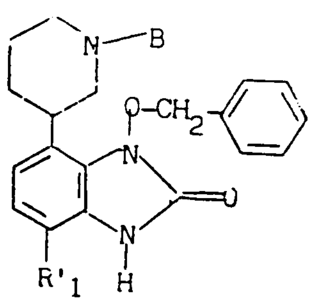

(VII)

in which B and $R'_1$ have the meaning already indicated, of which either the amine functions are unblocked, in order to obtain a derivative of formula ($I'_A$):

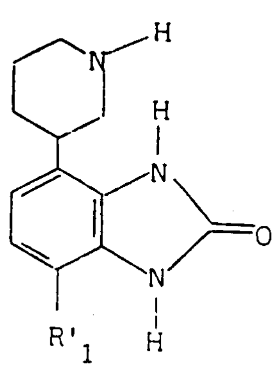

($I'_A$)

in which $R'_1$ has the meaning already indicated, which either is isolated and, if desired, salified, or the amine of the piperidine ring is selectively unblocked, in order to obtain a derivative of formula (VIII):

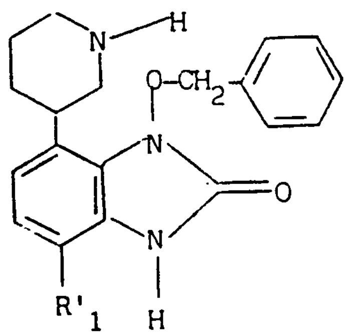

(VIII)

in which $R'_1$ has the meaning already indicated, which is subjected to the action of an agent capable of grafting an R' radical, R' having the meaning already indicated, in order to obtain a derivative of formula (IX):

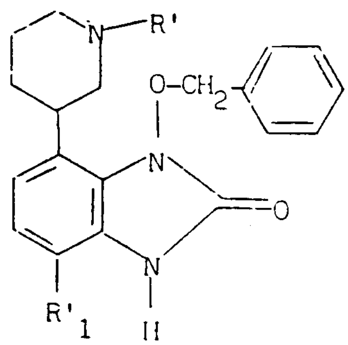

(IX)

in which R' and $R'_1$ have the meaning already indicated, then the said derivatives of formulae (VIII) and (IX) are subjected to a catalytic hydrogenation, in order to obtain a derivative of formula ($I'_B$):

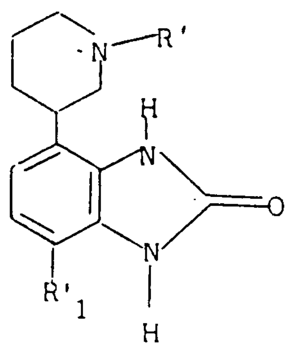

($I'_B$)

in which R' and $R'_1$ have the meaning already indicated, which either is isolated and, if desired, salified, or the said derivatives of formula ($I'_A$) and ($I'_B$) are subjected to the action of an unblocking agent of the hydroxyl, in order to obtain a derivative of formula ($I'_C$):

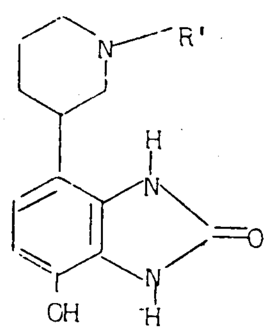

($I'_C$)

in which R' has the meaning already indicated, which is isolated and, if desired, salified, or if desired, for the derivative of formula (VII), the amine of the imidazole ring, then the amine of the piperidine and finally the hydroxyl are selectively unblocked.

2. Process according to claim 1, for the preparation of benzimidazol-2-one derivatives, as defined by formula (I) of claim 1, in which $R_1$ represents a hydrogen atom, as well as their salts, characterized in that a derivative of formula (II):

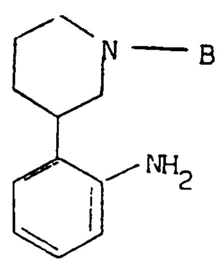

(II)

in which B represents a reversible blocking agent of a secondary amine, is reacted with an alkyl halogenoformate of formula (III):

Hal-COO-Alk (III)

in which Hal represents a chlorine, bromine or iodine atom and Alk represents an alkyl radical containing 1 to 5 carbon atoms, in order to obtain a derivative of formula (IV):

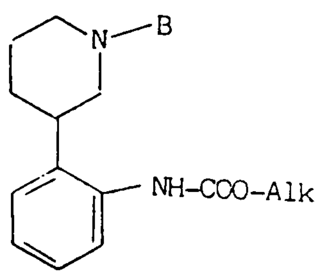

(IV)

in which B and Alk have the meaning already indicated, which is subjected to a nitration, in order to obtain a derivative of formula (V):

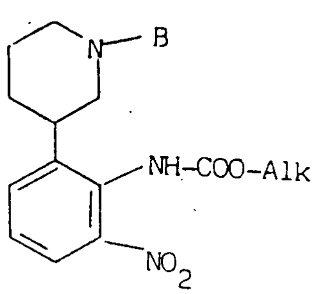

(V)

in which B and Alk have the meaning already indicated, of which either the nitro group is reduced, in order to obtain a derivative of formula (VI):

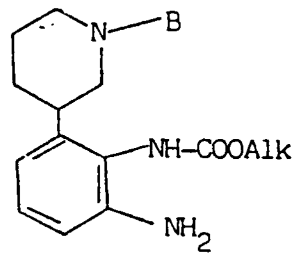

(VI)

in which B and Alk have the meaning already indicated, or the said derivative of formula (V) or the said derivative of formula (VI) are subjected to the action of an unblocking agent of the amine of the piperidine ring, in order to obtain respectively a derivative of formula (V') or (VI'):

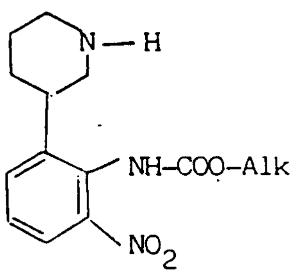

(V')

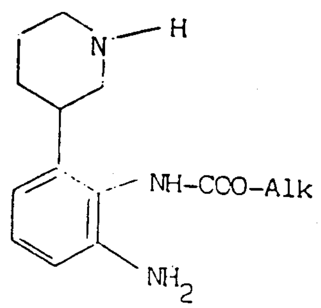

(VI')

in which Alk has the meaning already indicated, which is subjected to the action of an agent capable of grafting an R' radical, R' having the meaning of R with the exception of the hydrogen atom, in order to obtain the derivatives of formula (V") or (VI") respectively:

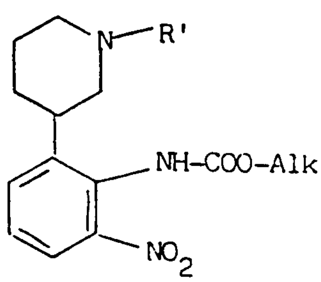

(V")

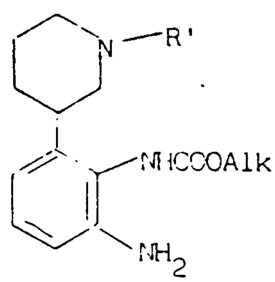

(VI")

in which Alk has the meaning already indicated, then the nitro group of the said derivatives of formulae (V') and (V") is reduced, in order to obtain the derivatives of formulae (VI') and (VI") respectively, which are cyclized by hydrolysis, in order to obtain a derivative of formula ($I_A$):

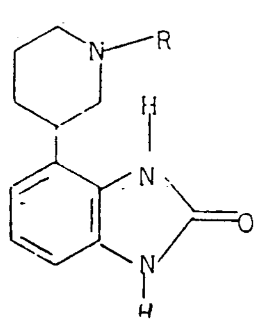

($I_A$)

in which R has the meaning already indicated, which is isolated and, if desired, salified, or the derivative of formula (VI) is cyclized in the same way as the derivatives of formulae (VI') and (VI"), then

the secondary amine of the piperidine ring is unblocked, in order to obtain a derivative of formula ($I_A$) in which R represents a hydrogen atom, which can be optionally substituted, in order to obtain other values of R.

**3.** Process according to claim 2, characterized in that:
- B is a trifluoroacetyl radical;
- the nitration of the derivative of formula (IV) is carried out using nitronium tetrafluoroborate.

**4.** Process according to claim 1, for the preparation of derivatives as defined by formula (I) of claim 1, in which $R_1$ is different from a hydrogen atom, as well as their salts, characterized in that a derivative of formula (II'):

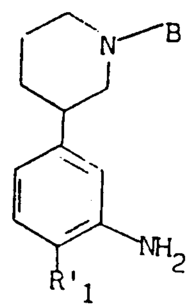

(II')

in which B has the meaning already indicated and $R'_1$ has the meaning of $R_1$ already indicated, with the exception of the hydrogen atom and the hydroxyl radical, is reacted with phosgene or a phosgene precursor, in order to obtain a derivative of formula (III'):

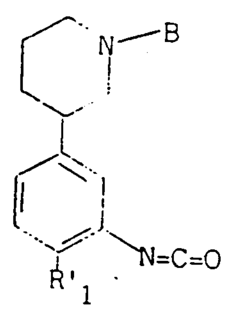

(III')

in which B and $R'_1$ have the meaning already indicated, which is reacted with O-benzylhydroxylamine, in order to obtain a derivative of formula (IV'):

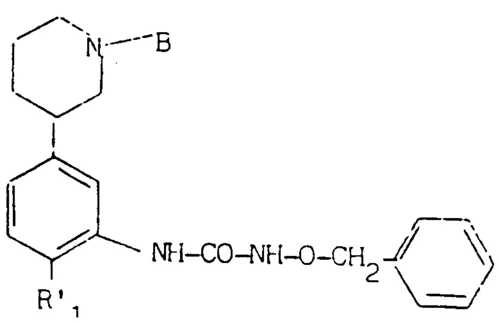

(IV')

in which B and $R'_1$ have the meaning already indicated, which is cyclized, in order to obtain a derivative of formula (VII):

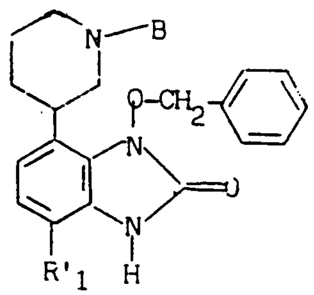

(VII)

in which B and R'$_1$ have the meaning already indicated, of which either the amine functions are unblocked, in order to obtain a derivative of formula (I'$_A$):

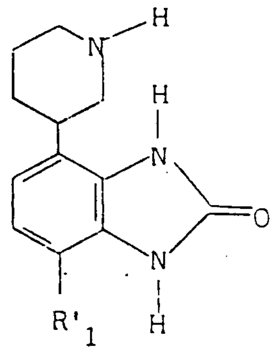

(I'$_A$)

in which R'$_1$ has the meaning already indicated, which either is isolated and, if desired, salified, or the amine of the piperidine ring is selectively unblocked, in order to obtain a derivative of formula (VIII):

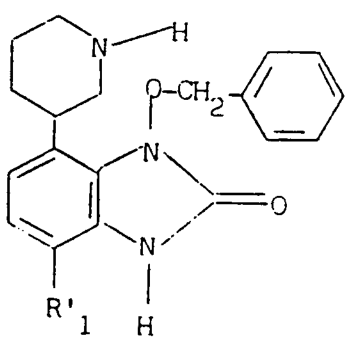

(VIII)

in which R'$_1$ has the meaning already indicated, which is subjected to the action of an agent capable of grafting an R' radical, R' having the meaning already indicated, in order to obtain a derivative of formula (IX):

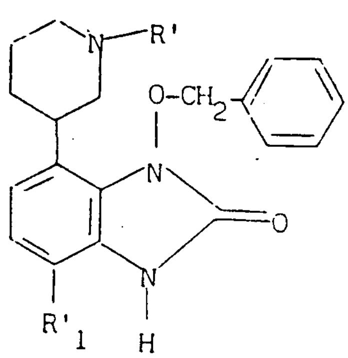

(IX)

in which R' and R'$_1$ have the meaning already indicated, then the said derivatives of formulae (VIII) and

(IX) are subjected to a catalytic hydrogenation, in order to obtain a derivative of formula ($I'_B$):

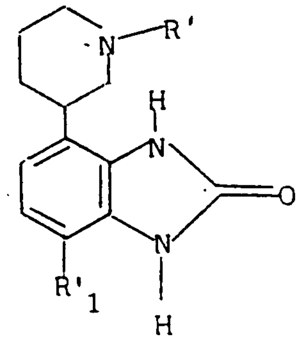

($I'_B$)

in which R' and $R'_1$ have the meaning already indicated, which either is isolated and, if desired, salified, or the said derivatives of formula ($I'_A$) and ($I'_B$) are subjected to the action of an unblocking agent of the hydroxyl, in order to obtain a derivative of formula ($I'_C$):

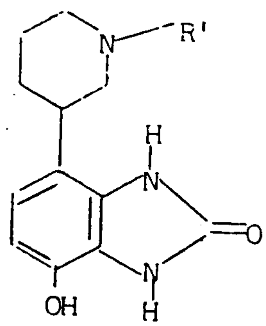

($I'_C$)

in which R' has the meaning already indicated, which is isolated and, if desired, salified, or if desired, for the derivative of formula (VII), the amine of the imidazole ring, then the amine of the piperidine and finally the hydroxyl are selectively unblocked.

**5.** Process according to claim 1, characterized in that derivatives of formula (I) are prepared, in which $R_1$ represents a hydrogen atom, as well as their salts.

**6.** Process according to claim 1 or 5, characterized in that derivatives of formula (I) are prepared, in which R represents a hydrogen atom or an alkyl radical containing 1 to 5 carbon atoms, as well as their salts.

**7.** Process according to claim 1, characterized in that the following are prepared:
- 1,3-dihydro-4-(1-propyl-3-piperidinyl)-2H-benzimidazol-2-one,
- 1,3-dihydro-4-hydroxy-7-(1-propyl-3-piperidinyl)-2H-benzimidazol-2-one,

as well as their addition salts with mineral or organic acids.

**Claims for the following Contracting State : GR**

**1.** Preparation process for benzimidazol-2-one derivatives as well as their addition salts with mineral or organic acids, corresponding to the general formula (I):

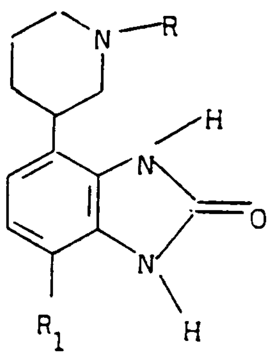

(I)

in which R represents a hydrogen atom, an alkyl radical containing 1 to 5 carbon atoms, a cycloalkylalkyl radical containing 4 to 7 carbon atoms, an aralkyl radical containing 7 to 12 carbon atoms, optionally substituted by one or more radicals chosen from the group constituted by alkyl radicals containing 1 to 5 carbon atoms, alkoxy radicals containing 1 to 5 carbon atoms and halogen atoms or R represents an alkenyl radical or an alkynyl radical containing 3 to 5 carbon atoms, it being understood that the multiple bond cannot be found between the carbons in alpha and beta position of the nitrogen atom, and $R_1$ represents a hydrogen atom, a hydroxy radical, an alkoxy radical containing 1 to 5 carbon atoms, a phenylalkyloxy radical containing 7 to 9 carbon atoms or a phenoxy radical, characterized in that:

a) to prepare the derivatives of formula (I), in which $R_1$ represents a hydrogen atom, as well as their salts, a derivative of formula (II):

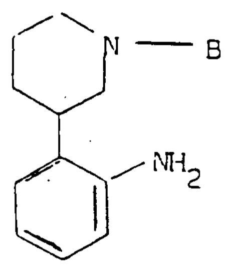

(II)

in which B represents a reversible blocking agent of a secondary amine function, is reacted with an alkyl halogenoformate of formula (III):

Hal-COO-Alk (III)

in which Hal represents a chlorine, bromine or iodine atom and Alk represents an alkyl radical containing 1 to 5 carbon atoms, in order to obtain a derivative of formula (IV):

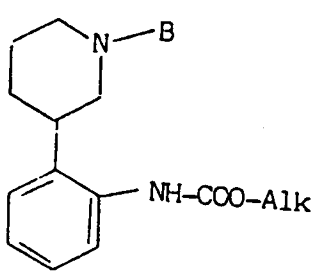

(IV)

in which B and Alk have the meaning already indicated, which is subjected to a nitration, in order to obtain a derivative of formula (V):

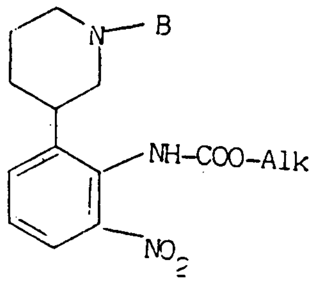

(V)

in which B and Alk have the meaning already indicated, of which either the nitro group is reduced, in order to obtain a derivative of formula (VI):

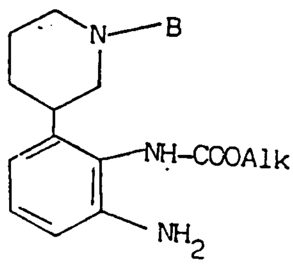

(VI)

in which B and Alk have the meaning already indicated, or the said derivative of formula (V) or the said derivative of formula (VI) are subjected to the action of an unblocking agent of the amine of the piperidine ring, in order to obtain respectively a derivative of formula (V') or (VI'):

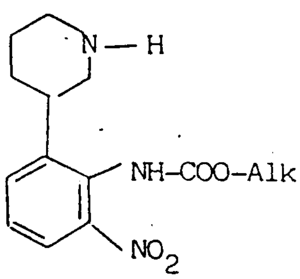

(V')

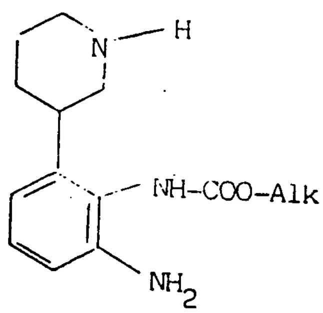

(VI')

in which Alk has the meaning already indicated, which is subjected to the action of an agent capable of grafting an R' radical, R' having the meaning of R with the exception of the hydrogen atom, in order to obtain the derivatives of formula (V") or (VI") respectively:

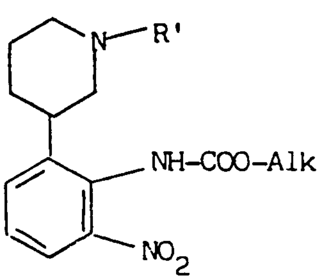

(V")

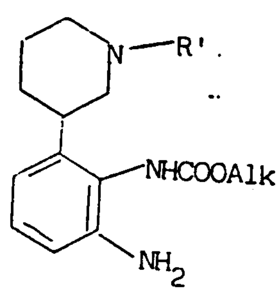

(VI")

in which Alk has the meaning already indicated, then the nitro group of the said derivatives of formulae (V') and (V") is reduced, in order to obtain the derivatives of formulae (VI') and (VI") respectively, which are cyclized by hydrolysis, in order to obtain a derivative of formula ($I_A$):

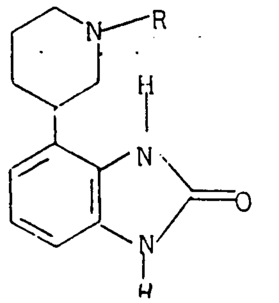

($I_A$)

in which R has the meaning already indicated, which is isolated and, if desired, salified, or the derivative of formula (VI) is cyclized in the same way as the derivatives of formulae (VI') and (VI"), then the secondary amine of the piperidine ring is unblocked, in order to obtain a derivative of formula ($I_A$) in which R represents a hydrogen atom, which can be optionally substituted, in order to obtain other values of R;

b) to prepare the derivatives of formula (I), in which $R_1$ is different from a hydrogen atom, as well as their salts, a derivative of formula (II'):

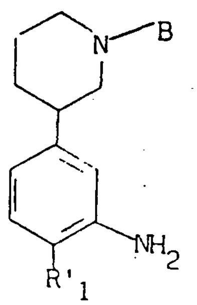

(II')

in which B has the meaning already indicated and $R'_1$ has the meaning of $R_1$ already indicated, with the exception of the hydrogen atom and the hydroxyl radical, is reacted with phosgene or a phosgene precursor, in order to obtain a derivative of formula (III'):

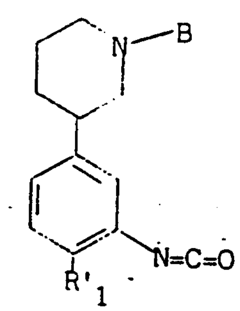

(III')

in which B and $R'_1$ have the meaning already indicated, which is reacted with O-benzylhydroxylamine, in order to obtain a derivative of formula (IV'):

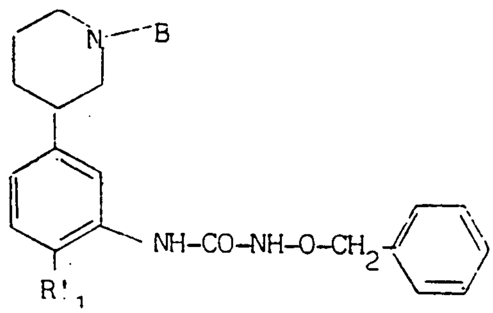

(IV')

in which B and $R'_1$ have the meaning already indicated, which is cyclized, in order to obtain a derivative of formula (VII):

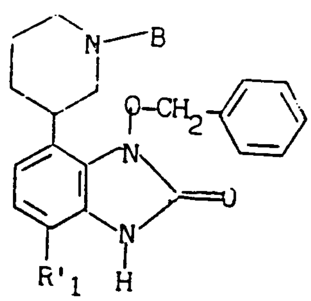

(VII)

in which B and $R'_1$ have the meaning already indicated, of which either the amine functions are unblocked, in order to obtain a derivative of formula ($I'_A$):

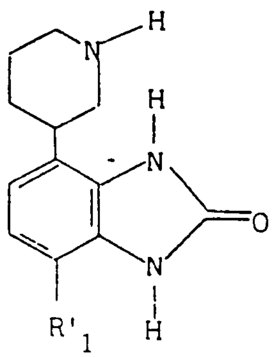

($I'_A$)

in which $R'_1$ has the meaning already indicated, which either is isolated and, if desired, salified, or the amine of the piperidine ring is selectively unblocked, in order to obtain a derivative of formula (VIII):

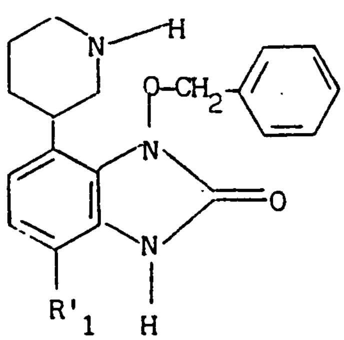

(VIII)

in which $R'_1$ has the meaning already indicated, which is subjected to the action of an agent capable of grafting an R' radical, R' having the meaning already indicated, in order to obtain a derivative of formula (IX):

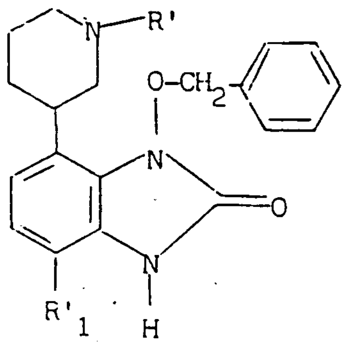

(IX)

in which R' and $R'_1$ have the meaning already indicated, then the said derivatives of formulae (VIII) and (IX) are subjected to a catalytic hydrogenation, in order to obtain a derivative of formula ($I'_B$):

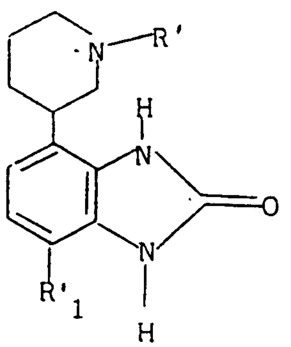

($I'_B$)

in which R' and $R'_1$ have the meaning already indicated, which either is isolated and, if desired, salified, or the said derivatives of formula ($I'_A$) and ($I'_B$) are subjected to the action of an unblocking agent of the hydroxyl, in order to obtain a derivative of formula ($I'_C$):

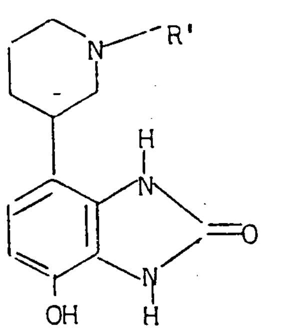

($I'_C$)

in which R' has the meaning already indicated, which is isolated and, if desired, salified, or if desired, for the derivative of formula (VII), the amine of the imidazole ring, then the amine of the piperidine and finally the hydroxyl are selectively unblocked.

**2.** Process according to claim 1, for the preparation of benzimidazol-2-one derivatives, as defined by formula (I) of claim 1, in which $R_1$ represents a hydrogen atom, as well as their salts, characterized in that a derivative of formula (II):

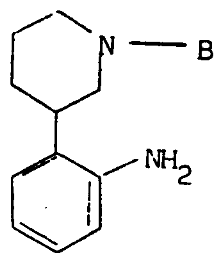

(II)

in which B represents a reversible blocking agent of a secondary amine, is reacted with an alkyl halogenoformate of formula (III):

Hal-COO-Alk (III)

in which Hal represents a chlorine, bromine or iodine atom and Alk represents an alkyl radical containing 1 to 5 carbon atoms, in order to obtain a derivative of formula (IV):

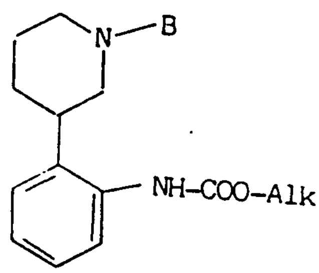

(IV)

in which B and Alk have the meaning already indicated, which is subjected to a nitration, in order to obtain a derivative of formula (V):

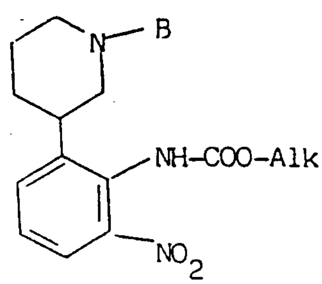

(V)

in which B and Alk have the meaning already indicated, of which either the nitro group is reduced, in order to obtain a derivative of formula (VI):

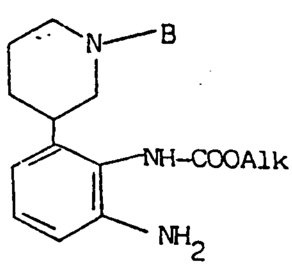

(VI)

in which B and Alk have the meaning already indicated, or the said derivative of formula (V) or the said derivative of formula (VI) are subjected to the action of an unblocking agent of the amine of the piperidine ring, in order to obtain respectively a derivative of formula (V') or (VI'):

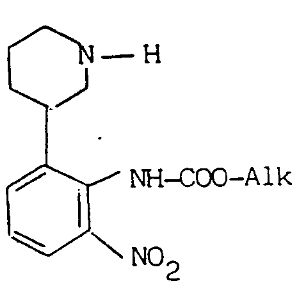 (V')

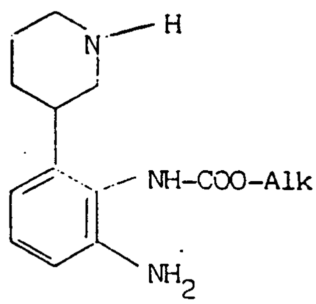 (VI')

in which Alk has the meaning already indicated, which is subjected to the action of an agent capable of grafting an R' radical, R' having the meaning of R with the exception of the hydrogen atom, in order to obtain the derivatives of formula (V") or (VI") respectively:

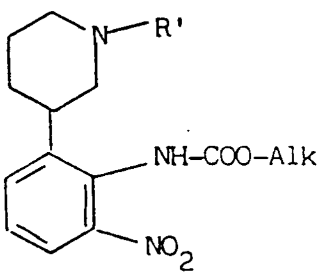 (V")

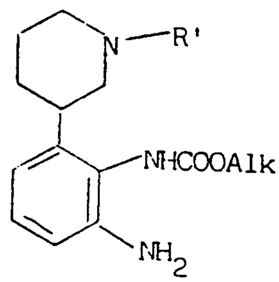 (VI")

in which Alk has the meaning already indicated, then the nitro group of the said derivatives of formulae (V') and (V") is reduced, in order to obtain the derivatives of formulae (VI') and (VI") respectively, which are cyclized by hydrolysis, in order to obtain a derivative of formula ($I_A$):

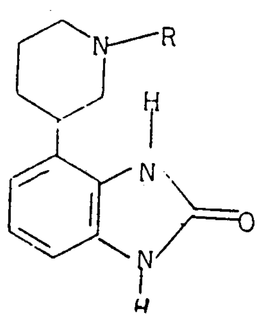 ($I_A$)

in which R has the meaning already indicated, which is isolated and, if desired, salified, or the derivative of formula (VI) is cyclized in the same way as the derivatives of formulae (VI') and (VI"), then the secondary amine of the piperidine ring is unblocked, in order to obtain a derivative of formula ($I_A$) in which R represents a hydrogen atom, which can be optionally substituted, in order to obtain other values of R.

**3.** Process according to claim 2, characterized in that:
- B is a trifluoroacetyl radical;
- the nitration of the derivative of formula (IV) is carried out using nitronium tetrafluoroborate.

**4.** Process according to claim 1, for the preparation of derivatives as defined by formula (I) of claim 1, in which $R_1$ is different from a hydrogen atom, as well as their salts, characterized in that a derivative of formula (II'):

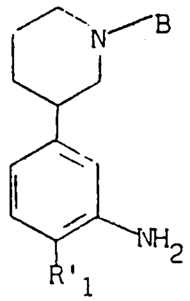

(II')

in which B has the meaning already indicated and $R'_1$ has the meaning of $R_1$ already indicated, with the exception of the hydrogen atom and the hydroxyl radical, is reacted with phosgene or a phosgene precursor, in order to obtain a derivative of formula (III'):

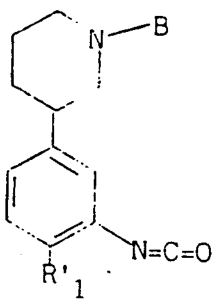

(III')

in which B and $R'_1$ have the meaning already indicated, which is reacted with O-benzylhydroxylamine, in order to obtain a derivative of formula (IV'):

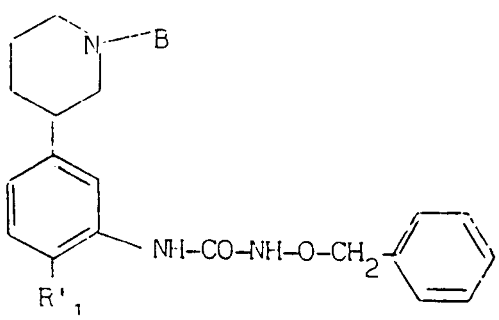

(IV')

in which B and $R'_1$ have the meaning already indicated, which is cyclized, in order to obtain a derivative of formula (VII):

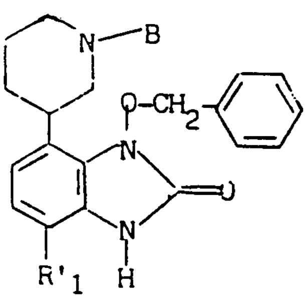

(VII)

in which B and $R'_1$ have the meaning already indicated, of which either the amine functions are unblocked, in order to obtain a derivative of formula ($I'_A$):

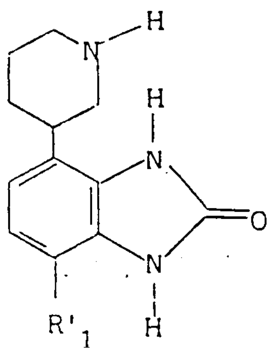

(I'$_A$)

in which R'$_1$ has the meaning already indicated, which either is isolated and, if desired, salified, or the amine of the piperidine ring is selectively unblocked, in order to obtain a derivative of formula (VIII):

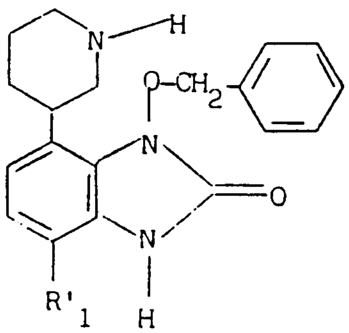

(VIII)

in which R'$_1$ has the meaning already indicated, which is subjected to the action of an agent capable of grafting an R' radical, R' having the meaning indicated in claim 5, in order to obtain a derivative of formula (IX):

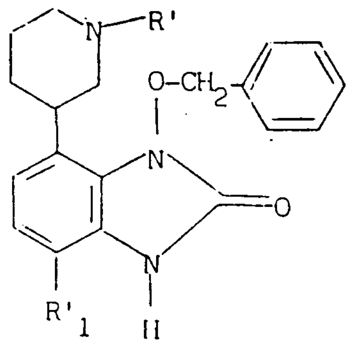

(IX)

in which R' and R'$_1$ have the meaning already indicated, then the said derivatives of formulae (VIII) and (IX) are subjected to a catalytic hydrogenation, in order to obtain a derivative of formula (I'$_B$):

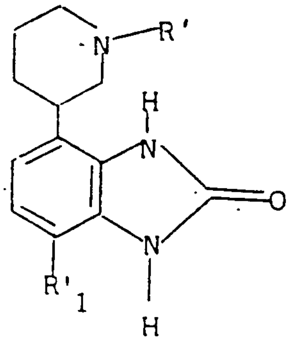

(I'$_B$)

in which R' and $R'_1$ have the meaning already indicated, which either is isolated and, if desired, salified, or the said derivatives of formula ($I'_A$) and ($I'_B$) are subjected to the action of an unblocking agent of the hydroxyl, in order to obtain a derivative of formula ($I'_C$):

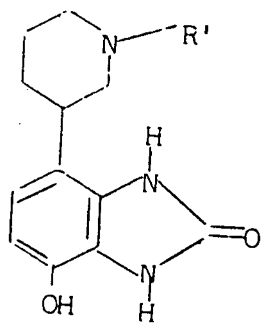

($I'_C$)

in which R' has the meaning already indicated, which is isolated and, if desired, salified, or if desired, for the derivative of formula (VII), the amine of the imidazole ring, then the amine of the piperidine and finally the hydroxyl are selectively unblocked.

5. Process according to claim 1, characterized in that derivatives of formula (I) are prepared, in which $R_1$ represents a hydrogen atom, as well as their salts.

6. Process according to claim 1 or 5, characterized in that derivatives of formula (I) are prepared, in which R represents a hydrogen atom or an alkyl radical containing 1 to 5 carbon atoms, as well as their salts.

7. Process according to claim 1, characterized in that the following are prepared:
   - 1,3-dihydro-4-(1-propyl-3-piperidinyl)-2H-benzimidazol-2-one,
   - 1,3-dihydro-4-hydroxy-7-(1-propyl-3-piperidinyl)-2H-benzimidazol-2-one,

   as well as their addition salts with mineral or organic acids.

8. Preparation process for pharmaceutical compositions, characterized in that at least one of the derivatives of formula (I) as defined in claim 1, or at least one of their addition salts with pharmaceutically acceptable mineral or organic acids, is used as active ingredient in a form intended for this use.

9. Process according to claim 8, characterized in that at least one of the derivatives of formula (I) as defined in claim 7, or at least one of their addition salts with pharmaceutically acceptable mineral or organic acids is used as active ingredient.

10. The derivatives of formulae (VI") and (VIII):

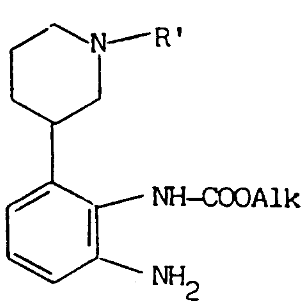

(VI")

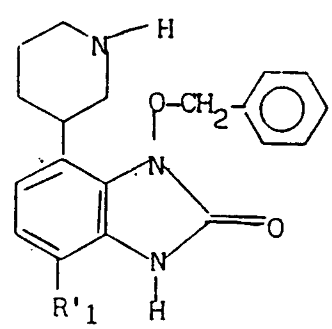

(VIII)

in which R', $R'_1$ and Alk have the meaning already indicated in claim 1.

**Patentansprüche**

**Patentansprüche für folgende Vertragsstaaten : AT, BE, CH, DE, FR, GB, IT, LI, LU, NL, SE**

**1.** Benzimidazol-2-on-Derivate sowie deren Additionssalze mit Mineral- oder organischen Säuren, dadurch gekennzeichnet, daß sie der allgemeinen Formel (I) entsprechen:

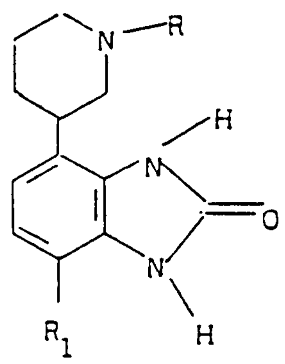

(I)

worin R für ein Wasserstoffatom, einen Alkylrest mit 1 bis 5 Kohlenstoffatomen, einen Cycloalkylalkylrest mit 4 bis 7 Kohlenstoffatomen, einen Aralkylrest mit 7 bis 12 Kohlenstoffatomen, der gegebenenfalls durch einen oder mehrere Reste substituiert ist, ausgewählt unter den Alkylresten mit 1 bis 5 Kohlenstoffatomen, den Alkoxyresten mit 1 bis 5 Kohlenstoffatomen und den Halogenatomen, steht oder R einen Alkenyl- oder einen Alkinylrest mit 3 bis 5 Kohlenstoffatomen bedeutet, wobei sich die Mehrfachbindung nicht zwischen den Kohlenstoffatomen in $\alpha$- und $\beta$-Stellung zum Stickstoffatom befinden kann, und $R_1$ ein Wasserstoffatom, eine Hydroxygruppe, eine Alkoxygruppe mit 1 bis 5 Kohlenstoffatomen, einen Phenylalkoxyrest mit 7 bis 9 Kohlenstoffatomen oder einen Phenoxyrest wiedergibt.

**2.** Benzimidazol-2-on-Derivate der Formel (I) gemäß Anspruch 1 sowie deren Additionssalze mit Mineral- oder organischen Säuren, dadurch gekennzeichnet, daß $R_1$ ein Wasserstoffatom bedeutet.

**3.** Benzimidazol-2-on-Derivate gemäß Anspruch 2 sowie deren Additionssalze mit Mineral- oder organischen Säuren, dadurch gekennzeichnet, daß R ein Wasserstoffatom oder einen Alkylrest mit 1 bis 5 Kohlenstoffatomen bedeutet.

**4.** Derivate der Formel (I) gemäß Anspruch 1 mit den folgenden Bezeichnungen:
1,3-Dihydro-4-(1-propyl-3-piperidinyl)-2H-benzimidazol-2-on,
1,3-Dihydro-4-hydroxy-7-(1-propyl-3-piperidinyl)-2H-benzimidazol-2-on
sowie deren Additionssalze mit Mineral- oder organischen Säuren.

**5.** Verfahren zur Herstellung der Benzimidazol-2-on-Derivate der Formel (I) gemäß Anspruch 1, worin $R_1$ für ein Wasserstoffatom steht, sowie von deren Salzen, dadurch gekennzeichnet, daß man ein Derivat der Formel (II)

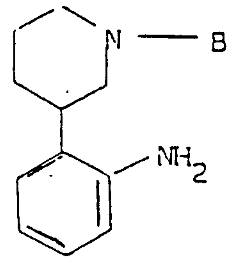

(II)

worin B ein reversibles Blockierungsmittel für ein sekundäres Amin bedeutet, mit einem Alkylhalogenformiat der Formel (III)

Hal-COO-Alk (III)

worin Hal ein Chlor-, Brom- oder Jodatom bedeutet und Alk für einen Alkylrest mit 1 bis 5 Kohlenstoffatomen steht, umsetzt, um zu einem Derivat der Formel (IV)

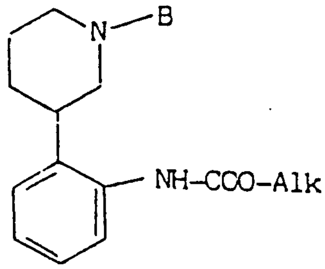

(IV)

zu gelangen, worin B und Alk die vorstehend angegebene Bedeutung besitzen, welches man einer Nitrierung unterzieht, um zu einem Derivat der Formel (V)

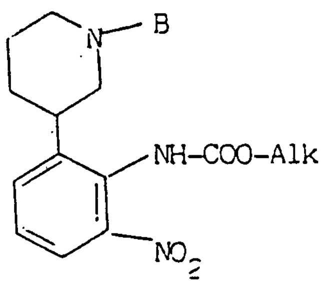

(V)

zu gelangen, worin B und Alk die vorstehend angegebene Bedeutung besitzen, man entweder dessen Nitrogruppe reduziert, um zu einem Derivat der Formel (VI)

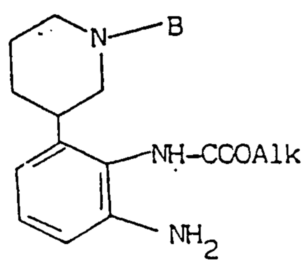

(VI)

zu gelangen, worin B und Alk die vorstehend angegebene Bedeutung besitzen, oder man das Derivat der Formel (V) oder das Derivat der Formel (VI) der Einwirkung eines Mittels für die Deblockierung des Amins des Piperidinrings unterzieht, um zu einem Derivat der Formel (V') bzw. (VI')

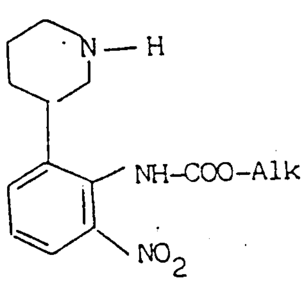

(V')

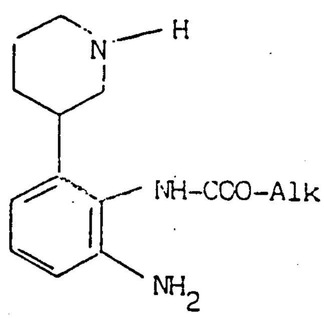

(VI')

zu gelangen, worin Alk die angegebene Bedeutung besitzt, welches man der Einwirkung eines Mittels unterzieht, das in der Lage ist, einen Rest R' einzuführen, wobei R' die Bedeutung von R mit Ausnahme des Wasserstoffatoms besitzt, um zu den Derivaten der Formeln (V") bzw. (VI")

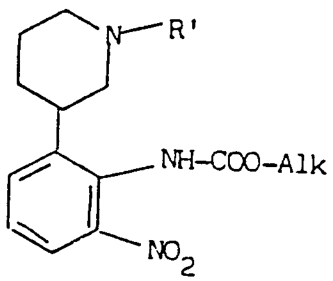 (V")

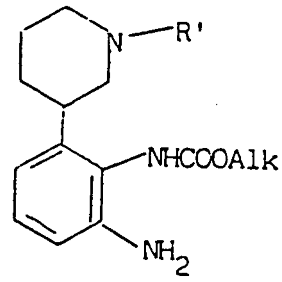 (VI")

zu gelangen, worin Alk die angegebene Bedeutung besitzt, danach die Nitrogruppe der Derivate der Formel (V') und (V") reduziert, um zu den Derivaten der Formeln (VI') bzw. (VI") zu gelangen, welche man durch Hydrolyse cyclisiert, um ein Derivat der Formel ($I_A$)

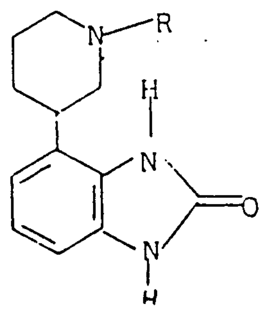

($I_A$)

worin R die angegebene Bedeutung besitzt, zu erhalten, das man isoliert und gewünschtenfalls in ein Salz überführt, oder das Derivat der Formel (VI) wie die Derivate der Formeln (VI') und (VI") cyclisiert, danach das sekundäre Amin des Piperidinrings deblockiert, um ein Derivat der Formel ($I_A$) zu erhalten, worin R für ein Wasserstoffatom steht, welches man gegebenenfalls substituieren kann, um zu den anderen Bedeutungen von R zu gelangen.

**6.** Verfahren gemäß Anspruch 4, dadurch gekennzeichnet, daß B für einen Trifluoracetylrest steht; die Nitrierung des Derivats der Formel (IV) mit Hilfe von Nitroniumtetrafluorborat erfolgt.

**7.** Verfahren zur Herstellung der Derivate der Formel (I) gemäß Anspruch 1, worin $R_1$ die in Anspruch 1 angegebene Bedeutung mit Ausnahme eines Wasserstoffatoms besitzt, sowie von deren Salzen, dadurch gekennzeichnet, daß man ein Derivat der Formel (II')

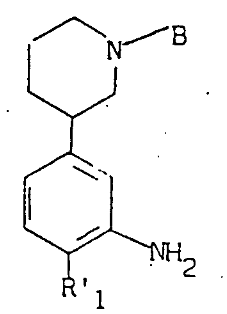

(II')

worin B die in Anspruch 5 angegebene Bedeutung hat und $R'_1$ die in Anspruch 1 für $R_1$ angegebene Bedeutung mit Ausnahme des Wasserstoffatoms oder des Hydroxylrestes besitzt, mit Phosgen oder einem Vorläufer des Phosgens umsetzt, um ein Derivat der Formel (III')

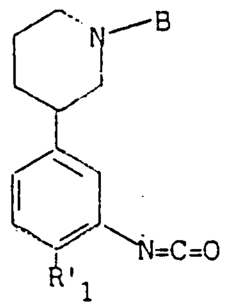

(III')

zu erhalten, worin B und R'$_1$ die angegebene Bedeutung besitzen, welches man mit O-Benzylhydroxylamin umsetzt, um ein Derivat der Formel (IV')

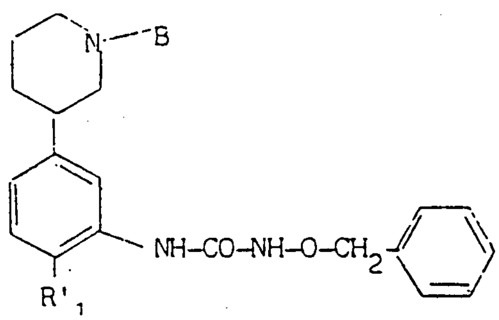

(IV')

zu erhalten, worin B und R'$_1$ die angegebene Bedeutung besitzen, das man cyclisiert, um zu einem Derivat der Formel (VII)

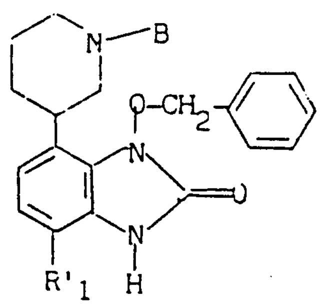

(VII)

zu gelangen, worin B und R'$_1$ die angegebene Bedeutung besitzen, entweder dessen Aminfunktionen deblockiert, um ein Derivat der Formel (I'$_A$)

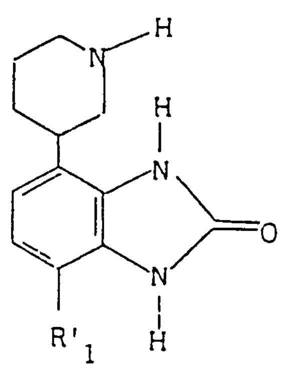

(I'$_A$)

zu erhalten, worin R'$_1$ die angegebene Bedeutung besitzt, welches man entweder isoliert und gewünschtenfalls in ein Salz überführt,oder selektiv das Amin des Piperidinrings deblockiert, um ein

Derivat der Formel (VIII)

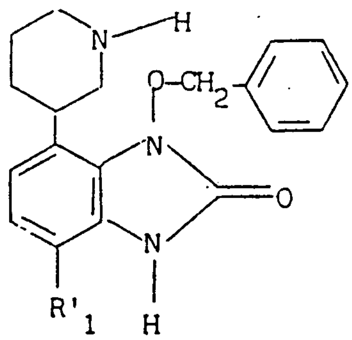

(VIII)

zu erhalten, worin $R'_1$ die angegebene Bedeutung besitzt, welches man der Einwirkung eines Mittels unterzieht, das in der Lage ist, einen Rest R' einzuführen, wobei R' die in Anspruch 5 angegebene Bedeutung besitzt, um ein Derivat der Formel (IX)

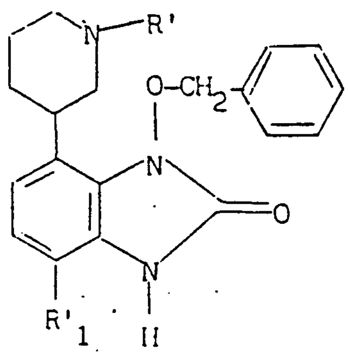

(IX)

zu erhalten, worin R' und $R'_1$ die angegebene Bedeutung besitzen, hiernach die Derivate der Formeln (VIII) und (IX) einer katalytischen Hydrierung unterzieht, um zu einem Derivat der Formel ($I'_B$)

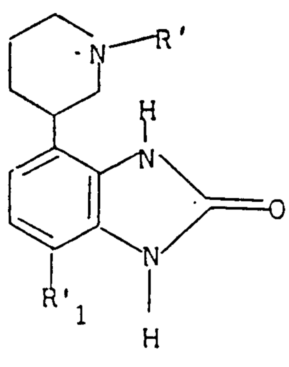

($I'_B$)

zu gelangen, worin R' und $R'_1$ die angegebene Bedeutung besitzen, welches man entweder isoliert und gewünschtenfalls in ein Salz überführt oder die Derivate der Formel ($I'_A$) und ($I'_B$) der Einwirkung eines Mittels zur Deblokkierung der Hydroxylgruppe unterzieht, um ein Derivat der Formel ($I'_C$)

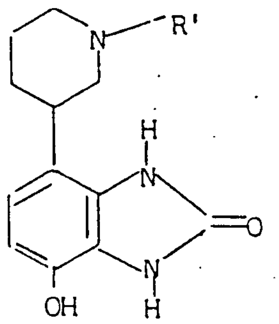

(I'c)

zu erhalten, worin R' die angegebene Bedeutung besitzt, das man isoliert und gewünschtenfalls in ein Salz überführt, oder selektiv bei dem Derivat der Formel (VII) das Amin des Imidazolrings, danach das Amin des Piperidins und schließlich das Hydroxyl, wenn erwünscht, deblockiert.

8. Arzneimittel, dadurch gekennzeichnet, daß sie aus den neuen Benzimidazol-2-on-Derivaten der Formel (I) gemäß Anspruch 1 sowie aus deren Additionssalzen mit pharmazeutisch verträglichen Säuren bestehen.

9. Arzneimittel, dadurch gekennzeichnet, daß sie aus den neuen Benzimidazol-2-on-Derivaten, wie in einem der Ansprüche 2 oder 3 definiert, sowie aus deren Additionssalzen mit pharmazeutisch verträglichen Säuren bestehen.

10. Arzneimittel, dadurch gekennzeichnet, daß sie aus den Benzimidazol-2-on-Derivaten, wie in Anspruch 4 definiert, sowie aus deren Additionssalzen mit pharmazeutisch verträglichen Säuren bestehen.

11. Pharmazeutische Zusammensetzungen, dadurch gekennzeichnet, daß sie als Wirkstoff zumindest eines der Arzneimittel, wie in einem der Ansprüche 8, 9 oder 10 definiert, enthalten.

12. Derivate der Formeln (VI") und (VIII)

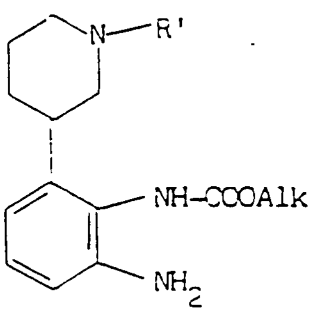

(VI")

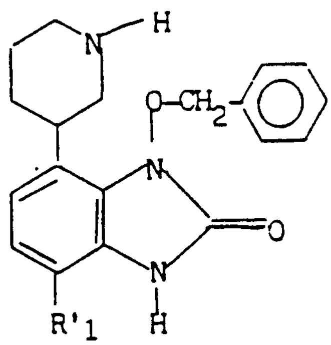

(VIII)

worin R' und Alk die in Anspruch 5 angegebene Bedeutung besitzen und R'$_1$ die in Anspruch 7 angegebene Bedeutung besitzt.

**Patentansprüche für folgenden Vertragsstaat : ES**

1. Verfahren zur Herstellung von Benzimidazol-2-on-Derivaten sowie deren Additionssalzen mit Mineral- oder organischen Säuren der allgemeinen Formel (I)

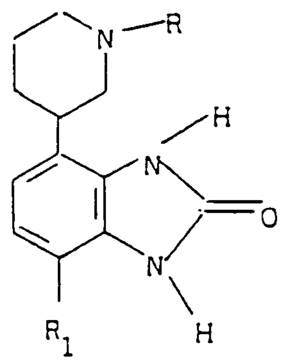

(I)

worin R für ein Wasserstoffatom, einen Alkylrest mit 1 bis 5 Kohlenstoffatomen, einen Cycloalkylalkylrest mit 4 bis 7 Kohlenstoffatomen, einen Aralkylrest mit 7 bis 12 Kohlenstoffatomen, der gegebenenfalls durch einen oder mehrere Reste substituiert ist, ausgewählt unter den Alkylresten mit 1 bis 5 Kohlenstoffatomen, den Alkoxyresten mit 1 bis 5 Kohlenstoffatomen und den Halogenatomen, steht oder R einen Alkenyl- oder einen Alkinylrest mit 3 bis 5 Kohlenstoffatomen bedeutet, wobei sich die Mehrfachbindung nicht zwischen den Kohlenstoffatomen in $\alpha$- und $\beta$-Stellung zum Stickstoffatom befinden kann, und $R_1$ ein Wasserstoffatom, eine Hydroxygruppe, eine Alkoxygruppe mit 1 bis 5 Kohlenstoffatomen, einen Phenylalkoxyrest mit 7 bis 9 Kohlenstoffatomen oder einen Phenoxyrest wiedergibt,

dadurch gekennzeichnet, daß man

(a) zur Herstellung der Derivate der Formel (I), worin $R_1$ für ein Wasserstoffatom steht, sowie von deren Salzen ein Derivat der Formel (II)

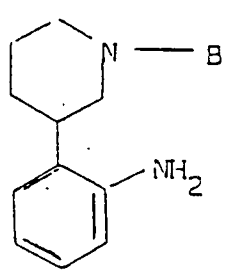

(II)

worin B ein reversibles Blockierungsmittel für ein sekundäres Amin bedeutet, mit einem Alkylhalogenformiat der Formel (III)

Hal-COO-Alk (III)

worin Hal ein Chlor-, Brom- oder Jodatom bedeutet und Alk für einen Alkylrest mit 1 bis 5 Kohlenstoffatomen steht, umsetzt, um zu einem Derivat der Formel (IV)

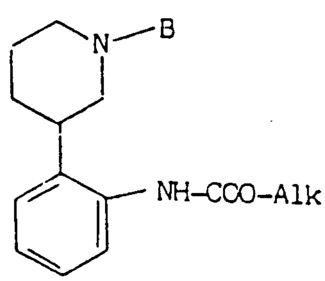

(IV)

zu gelangen, worin B und Alk die vorstehend angegebene Bedeutung besitzen, welches man einer Nitrierung unterzieht, um zu einem Derivat der Formel (V)

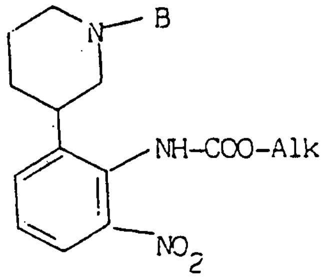

(V)

zu gelangen, worin B und Alk die vorstehend angegebene Bedeutung besitzen, man entweder dessen Nitrogruppe reduziert, um zu einem Derivat der Formel (VI)

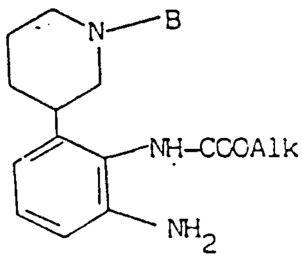

(VI)

zu gelangen, worin B und Alk die vorstehend angegebene Bedeutung besitzen, oder man das Derivat der Formel (V) oder das Derivat der Formel (VI) der Einwirkung eines Mittels für die Deblockierung des Amins des Piperidinrings unterzieht, um zu einem Derivat der Formel (V') bzw. (VI')

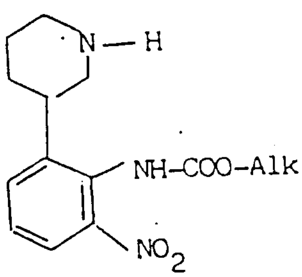

(V')

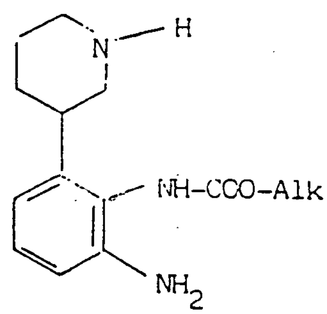

(VI')

zu gelangen, worin Alk die angegebene Bedeutung besitzt, welches man der Einwirkung eines Mittels unterzieht, das in der Lage ist, einen Rest R' einzuführen, wobei R' die Bedeutung von R mit Ausnahme des Wasserstoffatoms besitzt, um zu den Derivaten der Formeln (V") bzw. (VI")

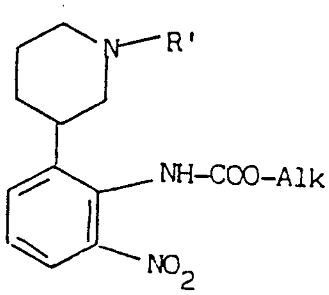

(V")

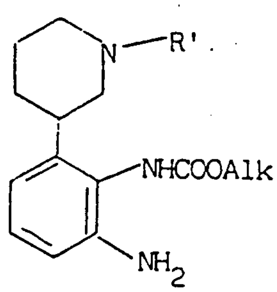

(VI")

zu gelangen, worin Alk die angegebene Bedeutung besitzt, danach die Nitrogruppe der Derivate der Formel (V') und (V") reduziert, um zu den Derivaten der Formeln (VI') bzw. (VI") zu gelangen,

welche man durch Hydrolyse cyclisiert, um ein Derivat der Formel ($I_A$)

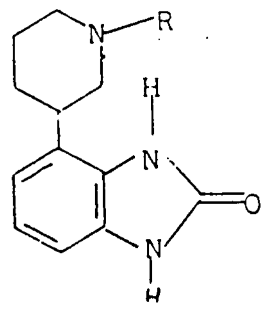

($I_A$)

worin R die angegebene Bedeutung besitzt, zu erhalten, das man isoliert und gewünschtenfalls in ein Salz überführt, oder das Derivat der Formel (VI) wie die Derivate der Formeln (VI') und (VI") cyclisiert, danach das sekundäre Amin des Piperidinrings deblockiert, um ein Derivat der Formel ($I_A$) zu erhalten, worin R für ein Wasserstoffatom steht, welches man gegebenenfalls substituieren kann, um zu den anderen Bedeutungen von R zu gelangen;

(b) zur Herstellung der Derivate der Formel (I), worin $R_1$ von einem Wasserstoffatom verschieden ist, sowie von deren Salzen ein Derivat der Formel (II')

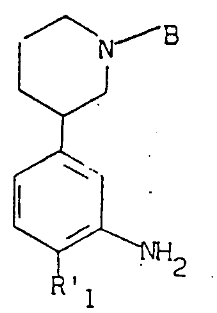

(II')

worin B die angegebene Bedeutung besitzt und $R'_1$ die angegebene Bedeutung von $R_1$ mit Ausnahme des Wasserstoffatoms und des Hydroxylrestes besitzt, mit Phosgen oder einem Vorläufer des Phosgens umsetzt, um ein Derivat der Formel (III')

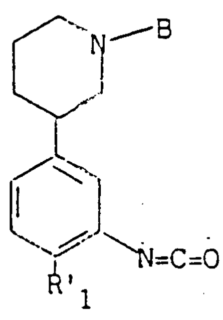

(III')

zu erhalten, worin B und $R'_1$ die angegebene Bedeutung besitzen, welches man mit O-Benzylhydroxylamin umsetzt, um ein Derivat der Formel (IV')

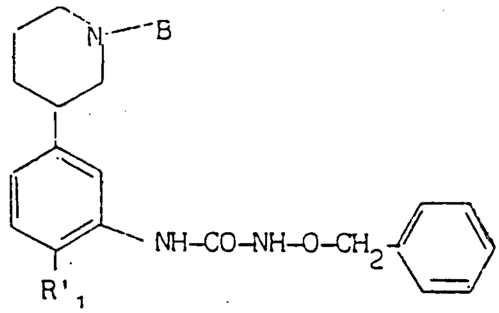

(IV')

zu erhalten, worin B und $R'_1$ die angegebene Bedeutung besitzen, das man cyclisiert, um zu einem Derivat der Formel (VII)

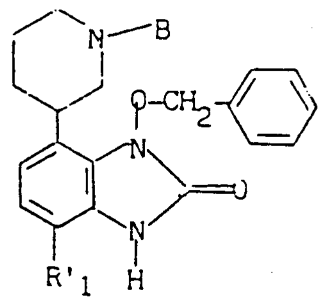

(VII)

zu gelangen, worin B und $R'_1$ die angegebene Bedeutung besitzen, entweder dessen Aminfunktionen deblockiert, um ein Derivat der Formel ($I'_A$)

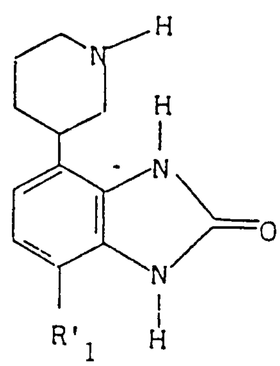

($I'_A$)

zu erhalten, worin $R'_1$ die angegebene Bedeutung besitzt, welches man entweder isoliert und gewünschtenfalls in ein Salz überführt, oder selektiv das Amin des Piperidinrings deblockiert, um ein Derivat der Formel (VIII)

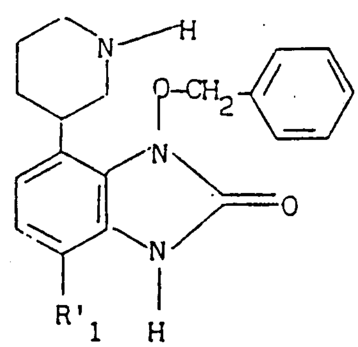

(VIII)

zu erhalten, worin $R'_1$ die angegebene Bedeutung besitzt, welches man der Einwirkung eines Mittels unterzieht, das in der Lage ist, einen Rest R' einzuführen, wobei R' die angegebene Bedeutung besitzt, um ein Derivat der Formel (IX)

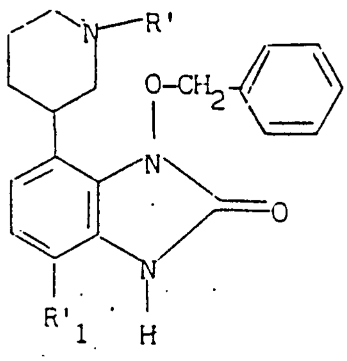

(IX)

zu erhalten, worin R' und $R'_1$ die angegebene Bedeutung besitzen, hiernach die Derivate der Formeln (VIII) und (IX) einer katalytischen Hydrierung unterzieht, um zu einem Derivat der Formel ($I'_B$)

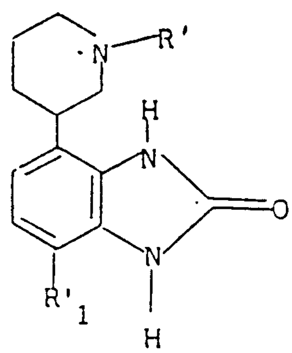

($I'_B$)

zu gelangen, worin R' und $R'_1$ die angegebene Bedeutung besitzen, welches man entweder isoliert und gewünschtenfalls in ein Salz überführt oder die Derivate der Formel ($I'_A$) und ($I'_B$) der Einwirkung eines Mittels zur Deblokkierung der Hydroxylgruppe unterzieht, um ein Derivat der Formel ($I'_C$)

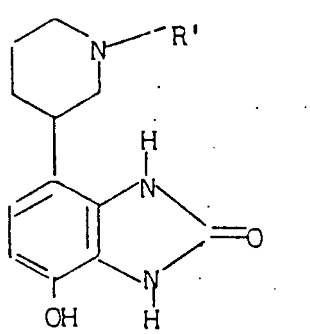

($I'_C$)

zu erhalten, worin R' die angegebene Bedeutung besitzt, das man isoliert und gewünschtenfalls in ein Salz überführt, oder selektiv bei dem Derivat der Formel (VII) das Amin des Imidazolrings, danach das Amin des Piperidins und schließlich das Hydroxyl, wenn erwünscht, deblockiert.

**2.** Verfahren gemäß Anspruch 1 zur Herstellung der Benzimidazol-2-on-Derivate der Formel (I) gemäß Anspruch 1, worin $R_1$ ein Wasserstoffatom bedeutet, sowie von deren Salzen, dadurch gekennzeichnet, daß man ein Derivat der Formel (II)

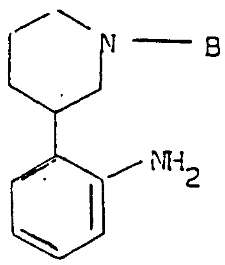

(II)

worin B ein reversibles Blockierungsmittel für ein sekundäres Amin bedeutet, mit einem Alkylhalogenformiat der Formel (III)

Hal-COO-Alk (III)

worin Hal ein Chlor-, Brom- oder Jodatom bedeutet und Alk für einen Alkylrest mit 1 bis 5 Kohlenstoffatomen steht, umsetzt, um zu einem Derivat der Formel (IV)

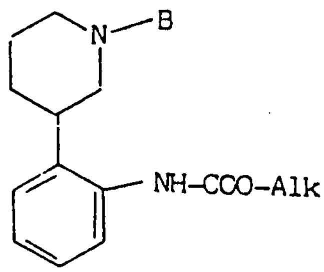

(IV)

zu gelangen, worin B und Alk die vorstehend angegebene Bedeutung besitzen, welches man einer Nitrierung unterzieht, um zu einem Derivat der Formel (V)

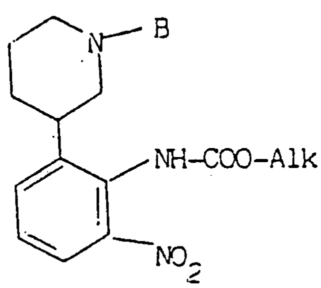

(V)

zu gelangen, worin B und Alk die vorstehend angegebene Bedeutung besitzen, man entweder dessen Nitrogruppe reduziert, um zu einem Derivat der Formel (VI)

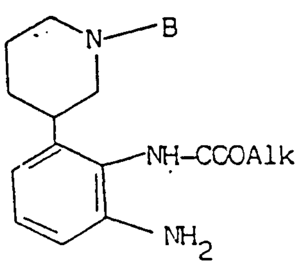

(VI)

zu gelangen, worin B und Alk die vorstehend angegebene Bedeutung besitzen, oder man das Derivat der Formel (V) oder das Derivat der Formel (VI) der Einwirkung eines Mittels für die Deblockierung des Amins des Piperidinrings unterzieht, um zu einem Derivat der Formel (V') bzw. (VI')

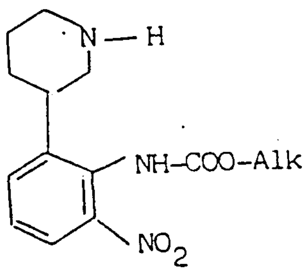
(V')

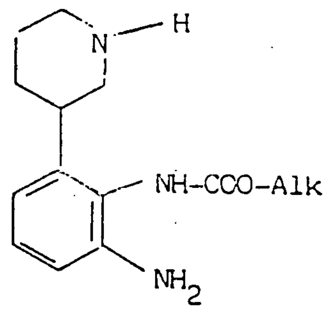
(VI')

zu gelangen, worin Alk die angegebene Bedeutung besitzt, welches man der Einwirkung eines Mittels unterzieht, das in der Lage ist, einen Rest R' einzuführen, wobei R' die Bedeutung von R mit Ausnahme des Wasserstoffatoms besitzt, um zu den Derivaten der Formeln (V") bzw. (VI")

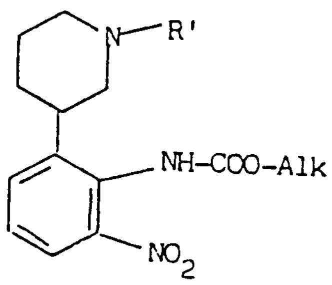
(V")

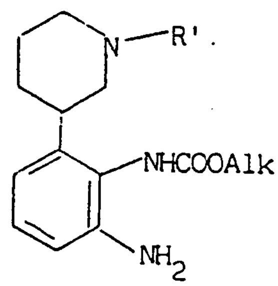
(VI")

zu gelangen, worin Alk die angegebene Bedeutung besitzt, danach die Nitrogruppe der Derivate der Formel (V') und (V") reduziert, um zu den Derivaten der Formeln (VI') bzw. (VI") zu gelangen, welche man durch Hydrolyse cyclisiert, um ein Derivat der Formel ($I_A$)

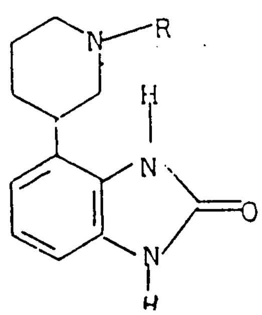
($I_A$)

worin R die angegebene Bedeutung besitzt, zu erhalten, das man isoliert und gewünschtenfalls in ein Salz überführt, oder das Derivat der Formel (VI) wie die Derivate der Formeln (VI') und (VI") cyclisiert, danach das sekundäre Amin des Piperidinrings deblockiert, um ein Derivat der Formel ($I_A$) zu erhalten, worin R für ein Wasserstoffatom steht, welches man gegebenenfalls substituieren kann, um zu den anderen Bedeutungen von R zu gelangen.

**3.** Verfahren gemäß Anspruch 2, dadurch gekennzeichnet, daß B für einen Trifluoracetylrest steht; die Nitrierung des Derivats der Formel (IV) mit Hilfe von Nitroniumtetrafluorborat erfolgt.

**4.** Verfahren gemäß Anspruch 1 zur Herstellung der Derivate der Formel (I) gemäß Anspruch 1, worin $R_1$ von einem Wasserstoffatom verschieden ist, sowie von deren Salzen, dadurch gekennzeichnet, daß man ein Derivat der Formel (II')

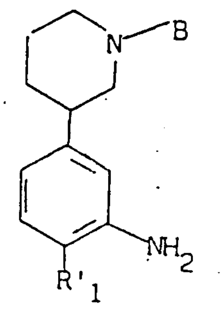

(II')

worin B die angegebene Bedeutung besitzt und R'$_1$ die angegebene Bedeutung von $R_1$ mit Ausnahme des Wasserstoffatoms und des Hydroxylrestes besitzt, mit Phosgen oder einem Vorläufer des Phosgens umsetzt, um ein Derivat der Formel (III')

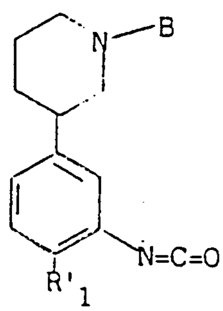

(III')

zu erhalten, worin B und R'$_1$ die angegebene Bedeutung besitzen, welches man mit O-Benzylhydroxylamin umsetzt, um ein Derivat der Formel (IV')

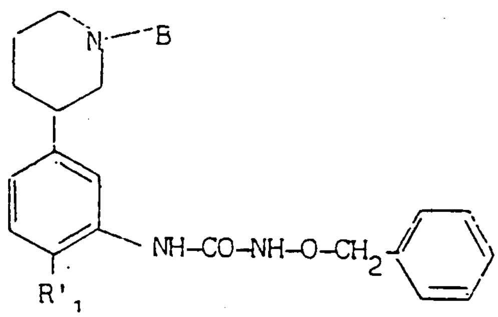

(IV')

zu erhalten, worin B und R'$_1$ die angegebene Bedeutung besitzen, das man cyclisiert, um zu einem Derivat der Formel (VII)

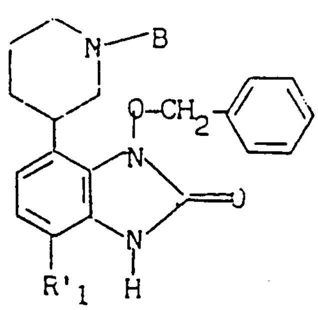

(VII)

zu gelangen, worin B und R'$_1$ die angegebene Bedeutung besitzen, entweder dessen Aminfunktionen deblockiert, um ein Derivat der Formel (I'$_A$)

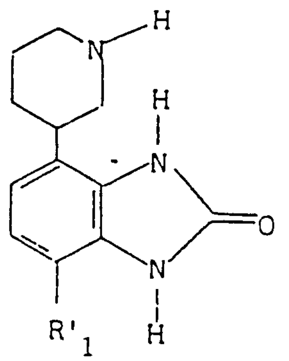

(I'A)

zu erhalten, worin R'₁ die angegebene Bedeutung besitzt, welches man entweder isoliert und gewünschtenfalls in ein Salz überführt, oder selektiv das Amin des Piperidinrings deblockiert, um ein Derivat der Formel (VIII)

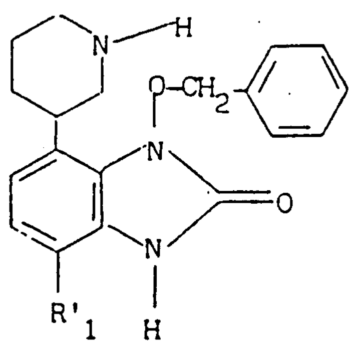

(VIII)

zu erhalten, worin $R'_1$ die angegebene Bedeutung besitzt, welches man der Einwirkung eines Mittels unterzieht, das in der Lage ist, einen Rest R' einzuführen, wobei R' die angegebene Bedeutung besitzt, um ein Derivat der Formel (IX)

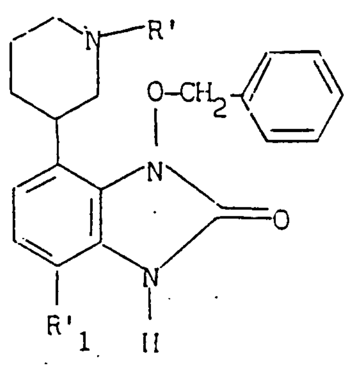

(IX)

zu erhalten, worin R' und $R'_1$ die angegebene Bedeutung besitzen, hiernach die Derivate der Formeln (VIII) und (IX) einer katalytischen Hydrierung unterzieht, um zu einem Derivat der Formel ($I'_B$)

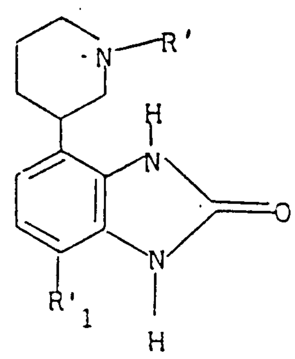

(I'$_B$)

zu gelangen, worin R' und R'$_1$ die angegebene Bedeutung besitzen, welches man entweder isoliert und gewünschtenfalls in ein Salz überführt oder die Derivate der Formel (I'$_A$) und (I'$_B$) der Einwirkung eines Mittels zur Deblokkierung der Hydroxylgruppe unterzieht, um ein Derivat der Formel (I'$_C$)

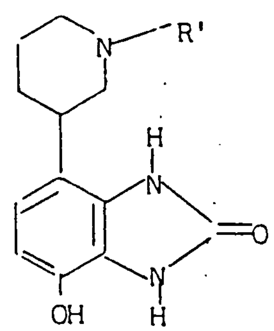

(I'$_C$)

zu erhalten, worin R' die angegebene Bedeutung besitzt, das man isoliert und gewünschtenfalls in ein Salz überführt, oder selektiv bei dem Derivat der Formel (VII) das Amin des Imidazolrings, danach das Amin des Piperidins und schließlich das Hydroxyl, wenn erwünscht, deblockiert.

5. Verfahren gemäß Anspruch 1, dadurch gekennzeichnet, daß man die Derivate der Formel (I), worin $R_1$ für ein Wasserstoffatom steht, sowie deren Salze herstellt.

6. Verfahren gemäß Anspruch 1 oder 4, dadurch gekennzeichnet, daß man die Derivate der Formel (I), worin R ein Wasserstoffatom oder einen Alkylrest mit 1 bis 5 Kohlenstoffatomen bedeutet, sowie deren Salze herstellt.

7. Verfahren gemäß Anspruch 1, dadurch gekennzeichnet, daß man herstellt:
   1,3-Dihydro-4-(1-propyl-3-piperidinyl)-2H-benzimidazol-2-on,
   1,3-Dihydro-4-hydroxy-7-(1-propyl-3-piperidinyl)-2H-benzimidazol-2-on,
   sowie deren Additionssalze mit Mineral- oder organischen Säuren.

**Patentansprüche für folgenden Vertragsstaat : GR**

1. Verfahren zur Herstellung von Benzimidazol-2-on-Derivaten sowie deren Additionssalzen mit Mineral- oder organischen Säuren der allgemeinen Formel (I)

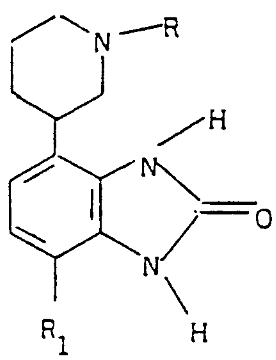

(I)

worin R für ein Wasserstoffatom, einen Alkylrest mit 1 bis 5 Kohlenstoffatomen, einen Cycloalkylalkylrest mit 4 bis 7 Kohlenstoffatomen, einen Aralkylrest mit 7 bis 12 Kohlenstoffatomen, der gegebenenfalls durch einen oder mehrere Reste substituiert ist, ausgewählt unter den Alkylresten mit 1 bis 5 Kohlenstoffatomen, den Alkoxyresten mit 1 bis 5 Kohlenstoffatomen und den Halogenatomen, steht oder R einen Alkenyl- oder einen Alkinylrest mit 3 bis 5 Kohlenstoffatomen bedeutet, wobei sich die Mehrfachbindung nicht zwischen den Kohlenstoffatomen in $\alpha$- und $\beta$-Stellung zum Stickstoffatom befinden kann, und $R_1$ ein Wasserstoffatom, eine Hydroxygruppe, eine Alkoxygruppe mit 1 bis 5 Kohlenstoffatomen, einen Phenylalkoxyrest mit 7 bis 9 Kohlenstoffatomen oder einen Phenoxyrest wiedergibt,

dadurch gekennzeichnet, daß man

(a) zur Herstellung der Derivate der Formel (I), worin $R_1$ für ein Wasserstoffatom steht, sowie von deren Salzen ein Derivat der Formel (II)

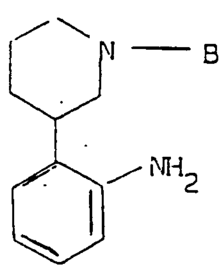

(II)

worin B ein reversibles Blockierungsmittel für ein sekundäres Amin bedeutet, mit einem Alkylhalogenformiat der Formel (III)

Hal-COO-Alk (III)

worin Hal ein Chlor-, Brom- oder Jodatom bedeutet und Alk für einen Alkylrest mit 1 bis 5 Kohlenstoffatomen steht, umsetzt, um zu einem Derivat der Formel (IV)

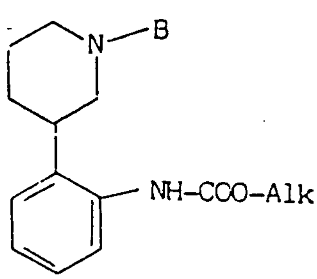

(IV)

zu gelangen, worin B und Alk die vorstehend angegebene Bedeutung besitzen, welches man einer Nitrierung unterzieht, um zu einem Derivat der Formel (V)

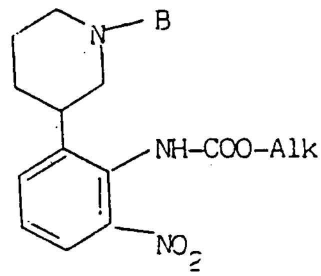

(V)

zu gelangen, worin B und Alk die vorstehend angegebene Bedeutung besitzen, man entweder dessen Nitrogruppe reduziert, um zu einem Derivat der Formel (VI)

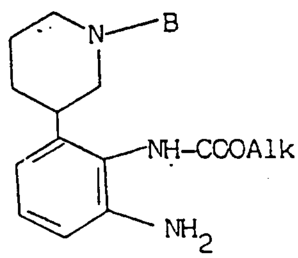

(VI)

zu gelangen, worin B und Alk die vorstehend angegebene Bedeutung besitzen, oder man das Derivat der Formel (V) oder das Derivat der Formel (VI) der Einwirkung eines Mittels für die Deblockierung des Amins des Piperidinrings unterzieht, um zu einem Derivat der Formel (V') bzw. (VI')

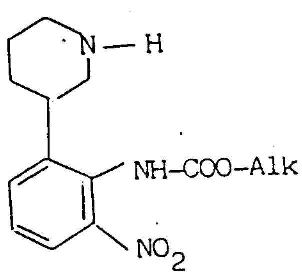

(V')

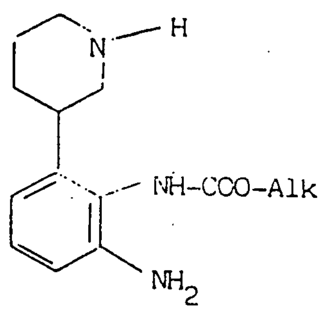

(VI')

zu gelangen, worin Alk die angegebene Bedeutung besitzt, welches man der Einwirkung eines Mittels unterzieht, das in der Lage ist, einen Rest R' einzuführen, wobei R' die Bedeutung von R mit Ausnahme des Wasserstoffatoms besitzt, um zu den Derivaten der Formeln (V") bzw. (VI")

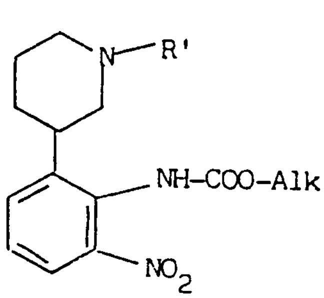

(V")

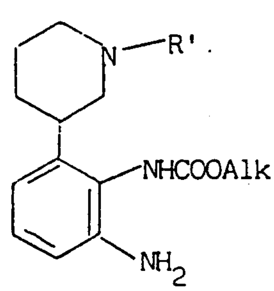

(VI")

zu gelangen, worin Alk die angegebene Bedeutung besitzt, danach die Nitrogruppe der Derivate der Formel (V') und (V") reduziert, um zu den Derivaten der Formeln (VI') bzw. (VI") zu gelangen, welche man durch Hydrolyse cyclisiert, um ein Derivat der Formel ($I_A$)

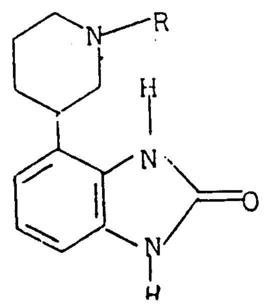

$(I_A)$

worin R die angegebene Bedeutung besitzt, zu erhalten, das man isoliert und gewünschtenfalls in ein Salz überführt, oder das Derivat der Formel (VI) wie die Derivate der Formeln (VI') und (VI") cyclisiert, danach das sekundäre Amin des Piperidinrings deblockiert, um ein Derivat der Formel ($I_A$) zu erhalten, worin R für ein Wasserstoffatom steht, welches man gegebenenfalls substituieren kann, um zu den anderen Bedeutungen von R zu gelangen;

(b) zur Herstellung der Derivate der Formel (I), worin $R_1$ von einem Wasserstoffatom verschieden ist, sowie von deren Salzen ein Derivat der Formel (II')

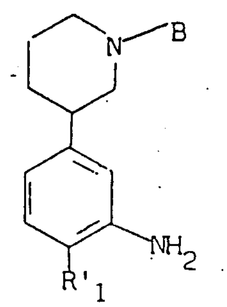

(II')

worin B die angegebene Bedeutung besitzt und $R'_1$ die angegebene Bedeutung von $R_1$ mit Ausnahme des Wasserstoffatoms und des Hydroxylrestes besitzt, mit Phosgen oder einem Vorläufer des Phosgens umsetzt, um ein Derivat der Formel (III')

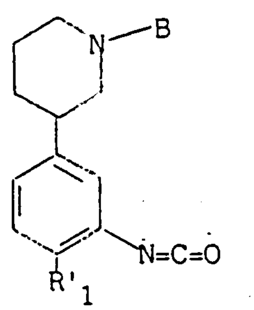

(III')

zu erhalten, worin B und $R'_1$ die angegebene Bedeutung besitzen, welches man mit O-Benzylhydroxylamin umsetzt, um ein Derivat der Formel (IV')

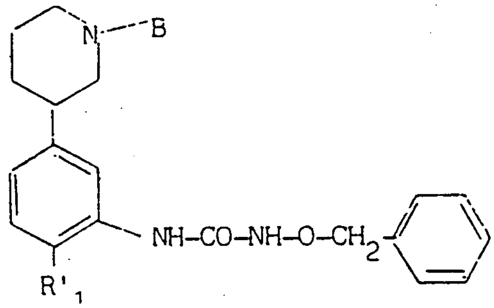

(IV')

zu erhalten, worin B und $R'_1$ die angegebene Bedeutung besitzen, das man cyclisiert, um zu einem Derivat der Formel (VII)

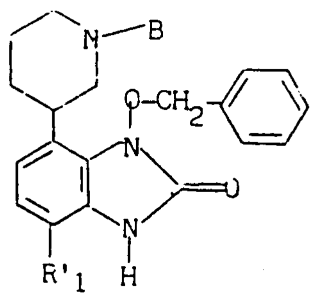

(VII)

zu gelangen, worin B und $R'_1$ die angegebene Bedeutung besitzen, entweder dessen Aminfunktionen deblockiert, um ein Derivat der Formel ($I'_A$)

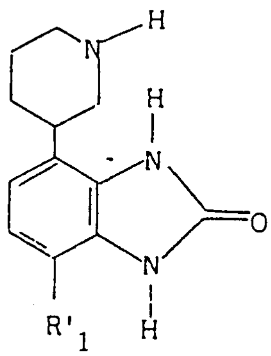

($I'_A$)

zu erhalten, worin $R'_1$ die angegebene Bedeutung besitzt, welches man entweder isoliert und gewünschtenfalls in ein Salz überführt,oder selektiv das Amin des Piperidinrings deblockiert, um ein Derivat der Formel (VIII)

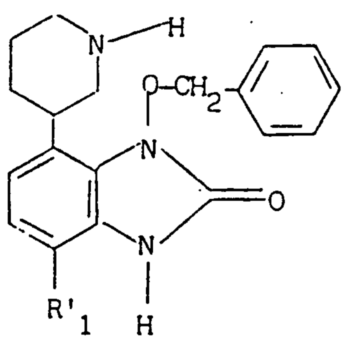

(VIII)

zu erhalten, worin $R'_1$ die angegebene Bedeutung besitzt, welches man der Einwirkung eines Mittels unterzieht, das in der Lage ist, einen Rest R' einzuführen, wobei R' die angegebene Bedeutung besitzt, um ein Derivat der Formel (IX)

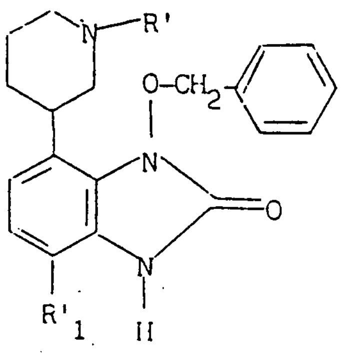

(IX)

zu erhalten, worin R' und $R'_1$ die angegebene Bedeutung besitzen, hiernach die Derivate der Formeln (VIII) und (IX) einer katalytischen Hydrierung unterzieht, um zu einem Derivat der Formel ($I'_B$)

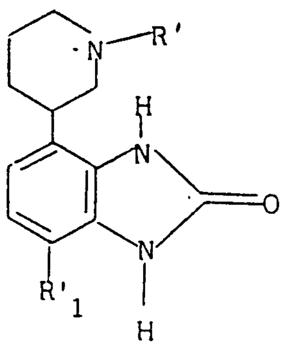

($I'_B$)

zu gelangen, worin R' und $R'_1$ die angegebene Bedeutung besitzen, welches man entweder isoliert und gewünschtenfalls in ein Salz überführt oder die Derivate der Formel ($I'_A$) und ($I'_B$) der Einwirkung eines Mittels zur Deblokkierung der Hydroxylgruppe unterzieht, um ein Derivat der Formel ($I'_C$)

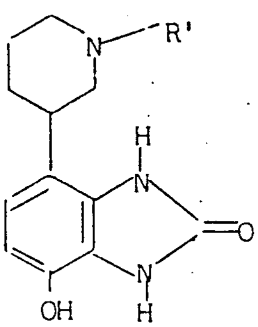

($I'_C$)

zu erhalten, worin R' die angegebene Bedeutung besitzt, das man isoliert und gewünschtenfalls in ein Salz überführt, oder selektiv bei dem Derivat der Formel (VII) das Amin des Imidazolrings, danach das Amin des Piperidins und schließlich das Hydroxyl, wenn erwünscht, deblockiert.

2. Verfahren gemäß Anspruch 1 zur Herstellung der Benzimidazol-2-on-Derivate der Formel (I) gemäß Anspruch 1, worin $R_1$ ein Wasserstoffatom bedeutet, sowie von deren Salzen, dadurch gekennzeichnet, daß man ein Derivat der Formel (II)

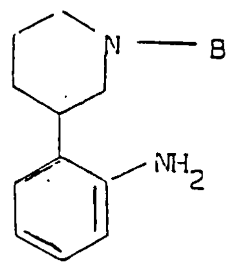

(II)

worin B ein reversibles Blockierungsmittel für ein sekundäres Amin bedeutet, mit einem Alkylhalogenformiat der Formel (III)

Hal-COO-Alk (III)

worin Hal ein Chlor-, Brom- oder Jodatom bedeutet und Alk für einen Alkylrest mit 1 bis 5 Kohlenstoffatomen steht, umsetzt, um zu einem Derivat der Formel (IV)

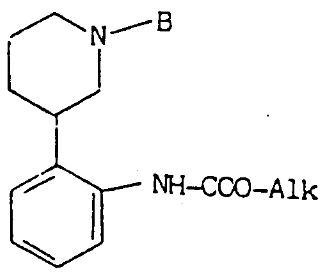

(IV)

zu gelangen, worin B und Alk die vorstehend angegebene Bedeutung besitzen, welches man einer Nitrierung unterzieht, um zu einem Derivat der Formel (V)

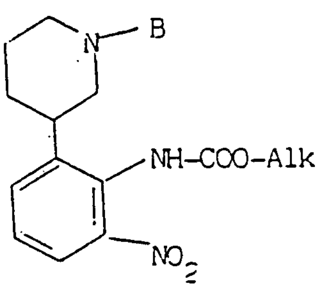

(V)

zu gelangen, worin B und Alk die vorstehend angegebene Bedeutung besitzen, man entweder dessen Nitrogruppe reduziert, um zu einem Derivat der Formel (VI)

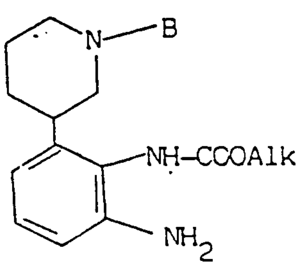

(VI)

zu gelangen, worin B und Alk die vorstehend angegebene Bedeutung besitzen, oder man das Derivat der Formel (V) oder das Derivat der Formel (VI) der Einwirkung eines Mittels für die Deblockierung des Amins des Piperidinrings unterzieht, um zu einem Derivat der Formel (V') bzw. (VI')

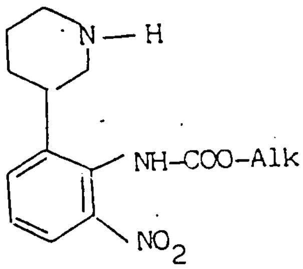

(V')

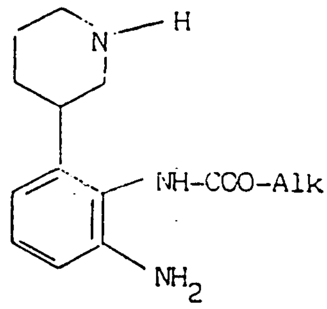

(VI')

zu gelangen, worin Alk die angegebene Bedeutung besitzt, welches man der Einwirkung eines Mittels unterzieht, das in der Lage ist, einen Rest R' einzuführen, wobei R' die Bedeutung von R mit Ausnahme des Wasserstoffatoms besitzt, um zu den Derivaten der Formeln (V") bzw. (VI")

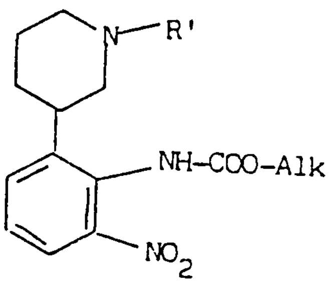

(V")

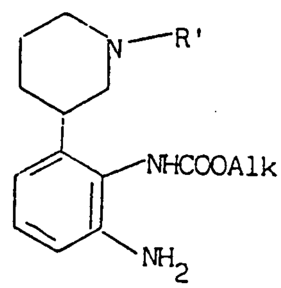

(VI")

zu gelangen, worin Alk die angegebene Bedeutung besitzt, danach die Nitrogruppe der Derivate der Formel (V') und (V") reduziert, um zu den Derivaten der Formeln (VI') bzw. (VI") zu gelangen, welche man durch Hydrolyse cyclisiert, um ein Derivat der Formel ($I_A$)

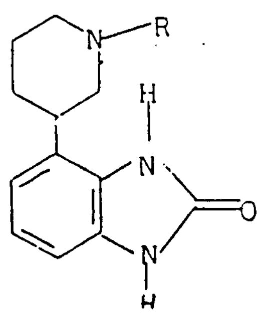

($I_A$)

worin R die angegebene Bedeutung besitzt, zu erhalten, das man isoliert und gewünschtenfalls in ein Salz überführt, oder das Derivat der Formel (VI) wie die Derivate der Formeln (VI') und (VI") cyclisiert, danach das sekundäre Amin des Piperidinrings deblockiert, um ein Derivat der Formel ($I_A$) zu erhalten, worin R für ein Wasserstoffatom steht, welches man gegebenenfalls substituieren kann, um zu den anderen Bedeutungen von R zu gelangen.

**3.** Verfahren gemäß Anspruch 2, dadurch gekennzeichnet, daß B für einen Trifluoracetylrest steht; die Nitrierung des Derivats der Formel (IV) mit Hilfe von Nitroniumtetrafluorborat erfolgt.

**4.** Verfahren gemäß Anspruch 1 zur Herstellung der Derivate der Formel (I) gemäß Anspruch 1, worin $R_1$ von einem Wasserstoffatom verschieden ist, sowie von deren Salzen, dadurch gekennzeichnet, daß man ein Derivat der Formel (II')

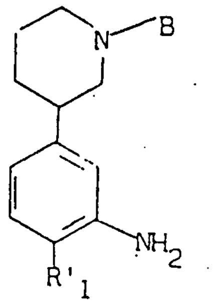

(II')

worin B die angegebene Bedeutung besitzt und $R'_1$ die angegebene Bedeutung von $R_1$ mit Ausnahme des Wasserstoffatoms und des Hydroxylrestes besitzt, mit Phosgen oder einem Vorläufer des Phosgens umsetzt, um ein Derivat der Formel (III')

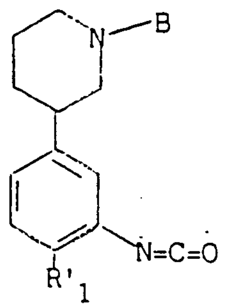

(III')

zu erhalten, worin B und $R'_1$ die angegebene Bedeutung besitzen, welches man mit O-Benzylhydroxylamin umsetzt, um ein Derivat der Formel (IV')

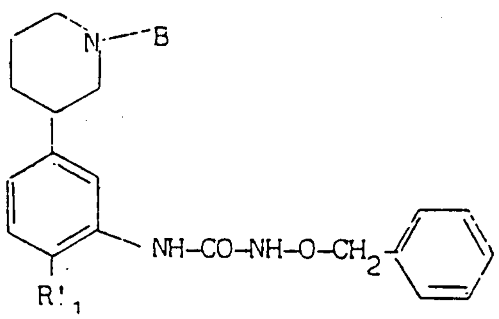

(IV')

zu erhalten, worin B und $R'_1$ die angegebene Bedeutung besitzen, das man cyclisiert, um zu einem Derivat der Formel (VII)

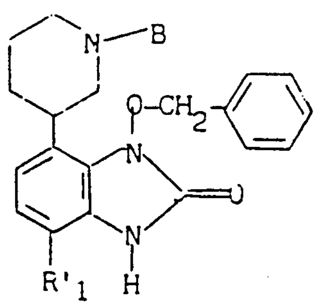

(VII)

zu gelangen, worin B und $R'_1$ die angegebene Bedeutung besitzen, entweder dessen Aminfunktionen deblockiert, um ein Derivat der Formel ($I'_A$)

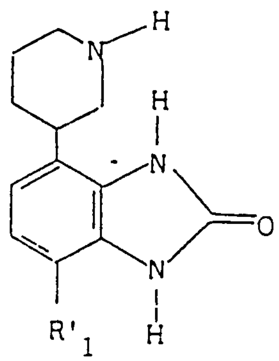

(I'$_A$)

zu erhalten, worin $R'_1$ die angegebene Bedeutung besitzt, welches man entweder isoliert und gewünschtenfalls in ein Salz überführt, oder selektiv das Amin des Piperidinrings deblockiert, um ein Derivat der Formel (VIII)

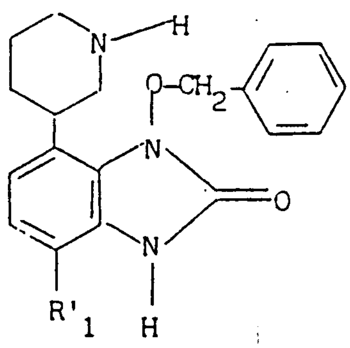

(VIII)

zu erhalten, worin $R'_1$ die angegebene Bedeutung besitzt, welches man der Einwirkung eines Mittels unterzieht, das in der Lage ist, einen Rest R' einzuführen, wobei R' die angegebene Bedeutung besitzt, um ein Derivat der Formel (IX)

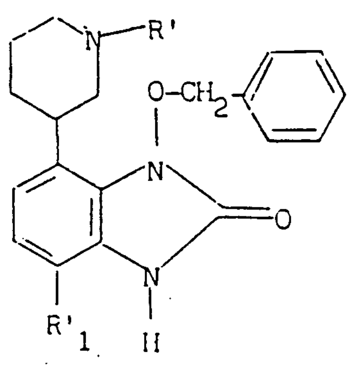

(IX)

zu erhalten, worin R' und $R'_1$ die angegebene Bedeutung besitzen, hiernach die Derivate der Formeln (VIII) und (IX) einer katalytischen Hydrierung unterzieht, um zu einem Derivat der Formel ($I'_B$)

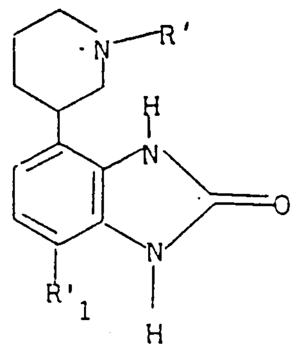

(I'$_B$)

zu gelangen, worin R' und R'$_1$ die angegebene Bedeutung besitzen, welches man entweder isoliert und gewünschtenfalls in ein Salz überführt oder die Derivate der Formel (I'$_A$) und (I'$_B$) der Einwirkung eines Mittels zur Deblokkierung der Hydroxylgruppe unterzieht, um ein Derivat der Formel (I'$_C$)

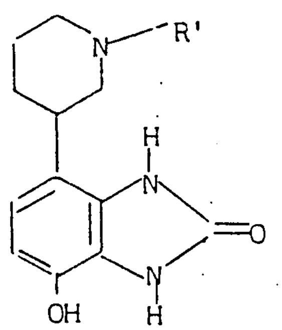

(I'$_C$)

zu erhalten, worin R' die angegebene Bedeutung besitzt, das man isoliert und gewünschtenfalls in ein Salz überführt, oder selektiv bei dem Derivat der Formel (VII) das Amin des Imidazolrings, danach das Amin des Piperidins und schließlich das Hydroxyl, wenn erwünscht, deblockiert.

5. Verfahren gemäß Anspruch 1, dadurch gekennzeichnet, daß man die Derivate der Formel (I), worin $R_1$ für ein Wasserstoffatom steht, sowie deren Salze herstellt.

6. Verfahren gemäß Anspruch 1 oder 5, dadurch gekennzeichnet, daß man die Derivate der Formel (I), worin R ein Wasserstoffatom oder einen Alkylrest mit 1 bis 5 Kohlenstoffatomen bedeutet, sowie deren Salze herstellt.

7. Verfahren gemäß Anspruch 1, dadurch gekennzeichnet, daß man herstellt:
   1,3-Dihydro-4-(1-propyl-3-piperidinyl)-2H-benzimidazol-2-on,
   1,3-Dihydro-4-hydroxy-7-(1-propyl -3-piperidinyl)-2H-benzimidazol-2-on,
   sowie deren Additionssalze mit Mineral- oder organischen Säuren.

8. Verfahren zur Herstellung von pharmazeutischen Zusammensetzungen, dadurch gekennzeichnet, daß man als Wirkstoff zumindest eines der Derivate der Formel (I) gemäß Anspruch 1 oder zumindest eines ihrer pharmazeutisch verträglichen Additionssalze mit Mineral- oder organischen Säuren in eine für diese Verwendung geeignete Form überführt.

9. Verfahren gemäß Anspruch 8, dadurch gekennzeichnet, daß man als Wirkstoff zumindest eines der Derivate der Formel (I), wie in Anspruch 7 definiert, oder zumindest eines ihrer Additionssalze mit pharmazeutisch verträglichen Mineral- oder organischen Säuren verwendet.

10. Derivate der Formeln (VI") und (VIII)

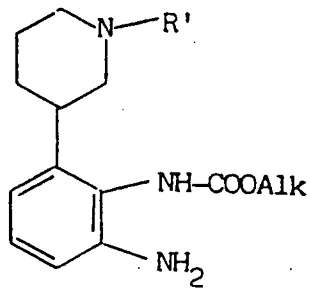 (VI")

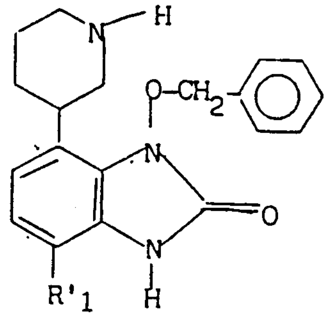 (VIII)

worin R', $R'_1$ und Alk die in Anspruch 1 angegebene Bedeutung besitzen.